(19)

Europäisches
Patentamt
European
Patent Office
Office européen
des brevets

(11)  **EP 2 426 216 A1**

(12)  **EUROPEAN PATENT APPLICATION**

(43) Date of publication:
**07.03.2012   Bulletin 2012/10**

(51) Int Cl.:
***C12Q 1/68*** *(2006.01)*

(21) Application number: **10305937.4**

(22) Date of filing: **01.09.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS**<br><br>(71) Applicant: **Institut Gustave Roussy (IGR)**<br>**94805 Villejuif Cedex (FR)**<br><br>(72) Inventors:<br>• **Galluzzi, Lorenzo**<br>  **94800 Villefjuif (FR)** | • **Harel-Bellan, Annick**<br>  **75005 Paris (FR)**<br>• **Kroemer, Guido**<br>  **75006 Paris (FR)**<br>• **Olaussen, Ken**<br>  **75014 Paris (FR)**<br>• **Soria, Jean-Charles**<br>  **91430 Igny (FR)**<br><br>(74) Representative: **Starck-Loudes, Anne-Caroline et al**<br>  **Cabinet Becker & Associés**<br>  **25, rue Louis Le Grand**<br>  **75002 Paris (FR)** |

(54)    **Prognostic and/or predictive biomarkers and biological applications thereof**

(57)    The present invention relates to the fields of genetics, immunology and medicine. The present invention more specifically relates to the identification of human genes and expression products thereof which can be used to assess the prognosis of a cancer in a subject, to assess the sensitivity of a subject to a treatment of cancer, or monitor, in particular determine the efficacy, of such a treatment of cancer after a given period of time. Said human genes and expression products can further be used (i) in the prevention or treatment of cancer, in particular to determine or select the appropriate cancer treatment for a given subject and/or for a particular tumor, as well as (ii) for the screening of therapeutically active drugs. Particular compounds capable to compensate, in a subject, an abnormal expression of one of the herein described products, in particular when the subject is exposed to a therapeutic treatment of cancer, thereby allowing or improving its efficiency in the subject, are also herein disclosed. Inventors in addition provide kits and DNA chips usable in the context of the present invention.

**EP 2 426 216 A1**

SEQUENCE LISTING

<110> Institut Gustave Roussy

<120> PROGNOSTIC AND/OR PREDICTIVE BIOMARKERS AND BIOLOGICAL
APPLICATIONS THEREOF

<130> B1064

<160> 186

<170> PatentIn version 3.3

<210> 1
<211> 1614
<212> DNA
<213> Homo sapiens

<220>
<221> CDS
<222> (58)..(1557)

<400> 1
ggtctctttg gcttcagaaa ttaccaagaa agcctggacc ccgggtgaaa cggagaa
    57

atg gac aca agt ccc ctg tgt ttc tcc att ctg ttg gtt tta tgc atc
    105
Met Asp Thr Ser Pro Leu Cys Phe Ser Ile Leu Leu Val Leu Cys Ile

1               5                   10                  15


ttt atc caa tca agt gcc ctt gga caa agc ctg aaa cca gag cca ttt
    153
Phe Ile Gln Ser Ser Ala Leu Gly Gln Ser Leu Lys Pro Glu Pro Phe

            20                  25                  30


gga aga aga gct caa gct gtt gaa aca aac aaa acg ctg cat gag atg
    201
Gly Arg Arg Ala Gln Ala Val Glu Thr Asn Lys Thr Leu His Glu Met

            35                  40                  45


aag acc aga ttc ctg ctc ttt gga gaa acc aat cag ggc tgt cag att
    249
Lys Thr Arg Phe Leu Leu Phe Gly Glu Thr Asn Gln Gly Cys Gln Ile

        50                  55                  60


cga atc aat cat ccg gac acg tta cag gag tgc ggc ttc aac tcc tcc
    297
Arg Ile Asn His Pro Asp Thr Leu Gln Glu Cys Gly Phe Asn Ser Ser

65                  70                  75                  80

```
ctg cct ctg gtg atg ata atc cac ggg tgg tcg gtg gac ggc gtg cta
    345
Leu Pro Leu Val Met Ile Ile His Gly Trp Ser Val Asp Gly Val Leu
                85                  90                  95


gaa aac tgg atc tgg cag atg gtg gcc gcg ctg aag tct cag ccg gcc
    393
Glu Asn Trp Ile Trp Gln Met Val Ala Ala Leu Lys Ser Gln Pro Ala
           100                 105                 110


cag cca gtg aac gtg ggg ctg gtg gac tgg atc acc ctg gcc cac gac
    441
Gln Pro Val Asn Val Gly Leu Val Asp Trp Ile Thr Leu Ala His Asp
           115                 120                 125


cac tac acc atc gcc gtc cgc aac acc cgc ctt gtg ggc aag gag gtc
    489
His Tyr Thr Ile Ala Val Arg Asn Thr Arg Leu Val Gly Lys Glu Val
        130                 135                 140


gcg gct ctt ctc cgg tgg ctg gag gaa tct gtg caa ctc tct cga agc
    537
Ala Ala Leu Leu Arg Trp Leu Glu Glu Ser Val Gln Leu Ser Arg Ser
145                 150                 155                 160


cat gtt cac cta att ggg tac agc ctg ggt gca cac gtg tca gga ttt
    585
His Val His Leu Ile Gly Tyr Ser Leu Gly Ala His Val Ser Gly Phe
                165                 170                 175


gcc ggc agt tcc atc ggt gga acg cac aag att ggg aga atc aca ggg
    633
Ala Gly Ser Ser Ile Gly Gly Thr His Lys Ile Gly Arg Ile Thr Gly
           180                 185                 190


ctg gat gcc gcg gga cct ttg ttt gag gga agt gcc ccc agc aat cgt
    681
Leu Asp Ala Ala Gly Pro Leu Phe Glu Gly Ser Ala Pro Ser Asn Arg
           195                 200                 205


ctt tct cca gat gat gcc aat ttt gtg gat gcc att cat acc ttt acc
    729
Leu Ser Pro Asp Asp Ala Asn Phe Val Asp Ala Ile His Thr Phe Thr
        210                 215                 220
```

cgg gag cac atg ggc ctg agc gtg ggc atc aaa cag ccc ata gga cac
777
Arg Glu His Met Gly Leu Ser Val Gly Ile Lys Gln Pro Ile Gly His
225             230             235             240

tat gac ttc tat ccc aac ggg ggc tcc ttc cag cct ggc tgc cac ttc
825
Tyr Asp Phe Tyr Pro Asn Gly Gly Ser Phe Gln Pro Gly Cys His Phe
          245             250             255

cta gag ctc tac aga cat att gcc cag cac ggc ttc aat gcc atc acc
873
Leu Glu Leu Tyr Arg His Ile Ala Gln His Gly Phe Asn Ala Ile Thr
          260             265             270

cag acc ata aaa tgc tcc cac gag cga tcg gtg cac ctt ttc atc gac
921
Gln Thr Ile Lys Cys Ser His Glu Arg Ser Val His Leu Phe Ile Asp
          275             280             285

tcc ttg ctg cac gcc ggc acg cag agc atg gcc tac ccg tgt ggt gac
969
Ser Leu Leu His Ala Gly Thr Gln Ser Met Ala Tyr Pro Cys Gly Asp
    290             295             300

atg aac agc ttc agc cag ggc ctg tgc ctg agc tgc aag aag ggc cgc
1017
Met Asn Ser Phe Ser Gln Gly Leu Cys Leu Ser Cys Lys Lys Gly Arg
305             310             315             320

tgc aac acg ctg ggc tac cac gtc cgc cag gag ccg cgg agc aag agc
1065
Cys Asn Thr Leu Gly Tyr His Val Arg Gln Glu Pro Arg Ser Lys Ser
          325             330             335

aag agg ctc ttc ctc gta acg cga gcc cag tcc ccc ttc aaa gtt tat
1113
Lys Arg Leu Phe Leu Val Thr Arg Ala Gln Ser Pro Phe Lys Val Tyr
          340             345             350

cat tac cag ttc aag atc cag ttc atc aac caa act gag aca cca ata
1161
His Tyr Gln Phe Lys Ile Gln Phe Ile Asn Gln Thr Glu Thr Pro Ile
          355             360             365

```
caa aca act ttt acc atg tca cta ctc gga aca aaa gag aaa atg cag
  1209
Gln Thr Thr Phe Thr Met Ser Leu Leu Gly Thr Lys Glu Lys Met Gln
    370                 375                 380

aaa att ccc atc act ctg ggc aaa gga att gct agt aat aaa acg tat
  1257
Lys Ile Pro Ile Thr Leu Gly Lys Gly Ile Ala Ser Asn Lys Thr Tyr
385                 390                 395                 400

tcc ttt ctt atc acg ctg gat gtg gat atc ggc gag ctg atc atg atc
  1305
Ser Phe Leu Ile Thr Leu Asp Val Asp Ile Gly Glu Leu Ile Met Ile
                405                 410                 415

aag ttc aag tgg gaa aac agt gca gtg tgg gcc aat gtc tgg gac acg
  1353
Lys Phe Lys Trp Glu Asn Ser Ala Val Trp Ala Asn Val Trp Asp Thr
                420                 425                 430

gtc cag acc atc atc cca tgg agc aca ggg ccg cgc cac tca ggc ctc
  1401
Val Gln Thr Ile Ile Pro Trp Ser Thr Gly Pro Arg His Ser Gly Leu
                435                 440                 445

gtt ctg aag acg atc aga gtc aaa gca gga gaa acc cag caa aga atg
  1449
Val Leu Lys Thr Ile Arg Val Lys Ala Gly Glu Thr Gln Gln Arg Met
    450                 455                 460

aca ttt tgt tca gaa aac aca gat gac cta cta ctt cgc cca acc cag
  1497
Thr Phe Cys Ser Glu Asn Thr Asp Asp Leu Leu Leu Arg Pro Thr Gln
465                 470                 475                 480

gaa aaa atc ttc gtg aaa tgt gaa ata aag tct aaa aca tca aag cga
  1545
Glu Lys Ile Phe Val Lys Cys Glu Ile Lys Ser Lys Thr Ser Lys Arg
                485                 490                 495

aag atc aga tga gatttaatga agacccagtg taaagaataa atgaatctta
  1597
Lys Ile Arg
```

```
ctccttaaaa aaaaaaa
  1614


<210>   2
<211>   499
<212>   PRT
<213>   Homo sapiens

<400>   2

Met Asp Thr Ser Pro Leu Cys Phe Ser Ile Leu Leu Val Leu Cys Ile
1               5                   10                  15


Phe Ile Gln Ser Ser Ala Leu Gly Gln Ser Leu Lys Pro Glu Pro Phe
            20                  25                  30


Gly Arg Arg Ala Gln Ala Val Glu Thr Asn Lys Thr Leu His Glu Met
        35                  40                  45


Lys Thr Arg Phe Leu Leu Phe Gly Glu Thr Asn Gln Gly Cys Gln Ile
    50                  55                  60


Arg Ile Asn His Pro Asp Thr Leu Gln Glu Cys Gly Phe Asn Ser Ser
65                  70                  75                  80


Leu Pro Leu Val Met Ile Ile His Gly Trp Ser Val Asp Gly Val Leu
                85                  90                  95


Glu Asn Trp Ile Trp Gln Met Val Ala Ala Leu Lys Ser Gln Pro Ala
            100                 105                 110


Gln Pro Val Asn Val Gly Leu Val Asp Trp Ile Thr Leu Ala His Asp
        115                 120                 125


His Tyr Thr Ile Ala Val Arg Asn Thr Arg Leu Val Gly Lys Glu Val
    130                 135                 140


Ala Ala Leu Leu Arg Trp Leu Glu Glu Ser Val Gln Leu Ser Arg Ser
145                 150                 155                 160


His Val His Leu Ile Gly Tyr Ser Leu Gly Ala His Val Ser Gly Phe
                165                 170                 175


Ala Gly Ser Ser Ile Gly Gly Thr His Lys Ile Gly Arg Ile Thr Gly
            180                 185                 190
```

Leu Asp Ala Ala Gly Pro Leu Phe Glu Gly Ser Ala Pro Ser Asn Arg
        195              200              205

Leu Ser Pro Asp Asp Ala Asn Phe Val Asp Ala Ile His Thr Phe Thr
        210              215              220

Arg Glu His Met Gly Leu Ser Val Gly Ile Lys Gln Pro Ile Gly His
225              230              235              240

Tyr Asp Phe Tyr Pro Asn Gly Gly Ser Phe Gln Pro Gly Cys His Phe
                245              250              255

Leu Glu Leu Tyr Arg His Ile Ala Gln His Gly Phe Asn Ala Ile Thr
            260              265              270

Gln Thr Ile Lys Cys Ser His Glu Arg Ser Val His Leu Phe Ile Asp
        275              280              285

Ser Leu Leu His Ala Gly Thr Gln Ser Met Ala Tyr Pro Cys Gly Asp
        290              295              300

Met Asn Ser Phe Ser Gln Gly Leu Cys Leu Ser Cys Lys Lys Gly Arg
305              310              315              320

Cys Asn Thr Leu Gly Tyr His Val Arg Gln Glu Pro Arg Ser Lys Ser
            325              330              335

Lys Arg Leu Phe Leu Val Thr Arg Ala Gln Ser Pro Phe Lys Val Tyr
            340              345              350

His Tyr Gln Phe Lys Ile Gln Phe Ile Asn Gln Thr Glu Thr Pro Ile
        355              360              365

Gln Thr Thr Phe Thr Met Ser Leu Leu Gly Thr Lys Glu Lys Met Gln
        370              375              380

Lys Ile Pro Ile Thr Leu Gly Lys Gly Ile Ala Ser Asn Lys Thr Tyr
385              390              395              400

Ser Phe Leu Ile Thr Leu Asp Val Asp Ile Gly Glu Leu Ile Met Ile
                405              410              415

Lys Phe Lys Trp Glu Asn Ser Ala Val Trp Ala Asn Val Trp Asp Thr
            420              425              430

Val Gln Thr Ile Ile Pro Trp Ser Thr Gly Pro Arg His Ser Gly Leu
        435              440              445

Val Leu Lys Thr Ile Arg Val Lys Ala Gly Glu Thr Gln Gln Arg Met
    450                 455                 460

Thr Phe Cys Ser Glu Asn Thr Asp Asp Leu Leu Leu Arg Pro Thr Gln
465                 470                 475                 480

Glu Lys Ile Phe Val Lys Cys Glu Ile Lys Ser Lys Thr Ser Lys Arg
                485                 490                 495

Lys Ile Arg


<210>   3
<211>   7390
<212>   DNA
<213>   Homo sapiens


<220>
<221>   CDS
<222>   (199)..(1137)

<400>   3
cggaactcgc gggttcggag ccgcccgctg aggtcagaag gaggcgtctg cgctgatcgg
    60

gtccgccgcg cgccagagcc agagtcgcag ccgaggggag ccggggccgg agcccgagcc
    120

cgagccgagc cggagcccga gcgagcggcg gagaccgtgc ccccgcctcg gccccgcgcc
    180

gccgcggcca ggcccggc atg gag gag gag tgc cgg gtg ctc tcc ata cag
    231                 Met Glu Glu Glu Cys Arg Val Leu Ser Ile Gln
                        1               5                   10

agc cac gtc atc cgc ggc tac gtg ggc aac cgg gcg gcc acg ttc ccg
    279
Ser His Val Ile Arg Gly Tyr Val Gly Asn Arg Ala Ala Thr Phe Pro
                15                  20                  25

ctg cag gtt ttg gga ttt gag att gac gcg gtg aac tct gtc cag ttt
    327
Leu Gln Val Leu Gly Phe Glu Ile Asp Ala Val Asn Ser Val Gln Phe
            30                  35                  40

tca aac cac aca ggc tat gcc cac tgg aag ggc caa gtg ctg aat tca
    375
Ser Asn His Thr Gly Tyr Ala His Trp Lys Gly Gln Val Leu Asn Ser

45                          50                          55

gat gag ctc cag gag ttg tac gaa ggc ctg agg ctg aac aac atg aat
423
Asp Glu Leu Gln Glu Leu Tyr Glu Gly Leu Arg Leu Asn Asn Met Asn

60              65                      70                      75

aaa tat gac tac gtg ctc aca ggt tat acg agg gac aag tcg ttc ctg
471
Lys Tyr Asp Tyr Val Leu Thr Gly Tyr Thr Arg Asp Lys Ser Phe Leu
                80                      85                      90

gcc atg gtg gtg gac att gtg cag gag ctg aag cag cag aac ccc agg
519
Ala Met Val Val Asp Ile Val Gln Glu Leu Lys Gln Gln Asn Pro Arg
                    95                      100                     105

ctg gtg tac gtg tgt gat cca gtc ttg ggt gac aag tgg gac ggc gaa
567
Leu Val Tyr Val Cys Asp Pro Val Leu Gly Asp Lys Trp Asp Gly Glu
            110                     115                     120

ggc tcg atg tac gtc ccg gag gac ctc ctt ccc gtc tac aaa gaa aaa
615
Gly Ser Met Tyr Val Pro Glu Asp Leu Leu Pro Val Tyr Lys Glu Lys
            125                     130                     135

gtg gtg ccg ctt gca gac att atc acg ccc aac cag ttt gag gcc gag
663
Val Val Pro Leu Ala Asp Ile Ile Thr Pro Asn Gln Phe Glu Ala Glu
140                     145                     150                     155

tta ctg agt ggc cgg aag atc cac agc cag gag gaa gcc ttg cgg gtg
711
Leu Leu Ser Gly Arg Lys Ile His Ser Gln Glu Glu Ala Leu Arg Val
                160                     165                     170

atg gac atg ctg cac tct atg ggc ccc gac acc gtg gtc atc acc agc
759
Met Asp Met Leu His Ser Met Gly Pro Asp Thr Val Val Ile Thr Ser
                175                     180                     185

tcc gac ctg ccc tcc ccg cag ggc agc aac tac ctg att gtg ctg ggg
807

```
Ser Asp Leu Pro Ser Pro Gln Gly Ser Asn Tyr Leu Ile Val Leu Gly
        190                 195                 200


agt cag agg agg agg aat ccc gct ggc tcc gtg gtg atg gaa cgc atc
    855
Ser Gln Arg Arg Arg Asn Pro Ala Gly Ser Val Val Met Glu Arg Ile
    205                 210                 215


cgg atg gac att cgc aaa gtg gac gcc gtc ttt gtg ggc act ggg gac
    903
Arg Met Asp Ile Arg Lys Val Asp Ala Val Phe Val Gly Thr Gly Asp
220                 225                 230                 235


ctg ttt gct gcc atg ctc ctg gcg tgg aca cac aag cac ccc aat aac
    951
Leu Phe Ala Ala Met Leu Leu Ala Trp Thr His Lys His Pro Asn Asn
                240                 245                 250


ctc aag gtg gcc tgt gag aag acc gtg tct acc ttg cac cac gtt ctg
    999
Leu Lys Val Ala Cys Glu Lys Thr Val Ser Thr Leu His His Val Leu
                255                 260                 265


cag agg acc atc cag tgt gca aaa gcc cag gcc ggg gaa gga gtg agg
    1047
Gln Arg Thr Ile Gln Cys Ala Lys Ala Gln Ala Gly Glu Gly Val Arg
                270                 275                 280


ccc agc ccc atg cag ctg gag ctg cgg atg gtg cag agc aaa agg gac
    1095
Pro Ser Pro Met Gln Leu Glu Leu Arg Met Val Gln Ser Lys Arg Asp
        285                 290                 295


atc gag gac cca gag atc gtc gtc cag gcc acg gtg ctg tga
    1137
Ile Glu Asp Pro Glu Ile Val Val Gln Ala Thr Val Leu
300                 305                 310


gggccccgcc gcttgcccgt gacacgcagc gcgttggtgt ctccgtgttt gtccctgtga
    1197

aaacatgtaa cgtctgcctt agagccatga ccgaaacttg atattttttt ctttcatgag
    1257

tgtccggcat ctgctggtct tcattgtgaa acgtgccagt cgtgctttgt gaaaaataac
    1317
```

```
aaagtggtca cagaaatttg tgatctgaaa acccggctcc cttccccaca aggctcctgg
   1377

gcctccggga agacgggccc ctgtttgcca tctcgggggt gttccctgtg ggagggtgag
   1437

tgggtgaggc cgagcctgct gcgtgtggag cctcgagtgg gccctggctg ccactaccgt
   1497

acagaggccg tgtcgcgctg ggctgggcct gggtggcctc tgtctttgca tctctgagaa
   1557

ggagtcgggt ggtaacggtt ggggtcagga agaattctgc caagtatctt tactgtcatt
   1617

ctgaccatag cctctttgtt cccgcattcg aacttttggt tcttactttg ctgctcgttt
   1677

agtccctggg gatttcagat cttaggctgt tgtttcaccg tatgggaggg ttgatgtgag
   1737

cttgcttgga gacacacggt gcagcatcag ggaccttccc aggccccagc aaattcaagt
   1797

cggtctgcag acctctcagc tacccgcggg acctcttgta acccatcggc atcttccagg
   1857

aatccgccga gtgacttgag gaagatgcta acgcagtaag gtctgtgctg ggccaagagc
   1917

agctttgaag ctccagagaa ccaccccgtc aggttccttg tggaagctcc cctcatccgt
   1977

ggtgcagcag gctgagcact gcgcgtttgc cacgtgctgc ccgtgacagc acattgagcc
   2037

acagcatttg tagacaggac agaggggtgc ctgcccctg ccctgctgg cacatttaac
   2097

ccttgtcccc tgacctcagt tctgtgcccc accaaatgcc caggggcaag aggccaccct
   2157

ggaagctgcc aatcttccaa ggtgggtgtg gggcacggtg ggggcgggca gctcccaggc
   2217

ccttgggcag gctggggtga cggcagaggc cacagcacca gctctgacaa gtcctatcat
   2277

cctctgctca gcagtgacct ccctggcccc actttgccca gagtttgggg tccccccagg
   2337

tatagctata ggcggcagtg cctgtccctg gcctgccttg atttcagcca cacccctgca
   2397

gccctgcatc ccagctctgg ggtgtgcaga ggtttgtgtc tccagggaac ccacggctgg
   2457

agagaaatag ggagatgcag gaagtggggg cccatggggc ccccaagaag cggactctcc
   2517

aaggggtacc cccaccccgc taccttcccc acggacgggc ccctcctgga gcccataccc
```

2577

tcctgtgagg ccattccagt gtcttctaga aagactcgct tgccaggagt gcgttctttg
2637

ttgaaaaatg ccctgaagcg aaaagatgca ggtttatatg gaacccccac cccctcccccc
2697

actctcccac tctgttcgtt ctgaatgtct tcacgagcgt gcatcagggc gcctggctcc
2757

cccacctcag ccagtgagtc agacacgggt ttcgcagcca tgtttcctgg ctccgaggac
2817

acgggtggca ggcccgttgc agcccagagc cactggtccc tacagggcgc cgccacacca
2877

gcaggaagga ggatggctgt gtccggagcc tggcggggag gcggcctccc cagtatgtga
2937

gtgcagggat ctgccagaac cacctggccc tctgtagggc gtttaactgg aaataccctc
2997

actgccaagt ggagactggg gcgtgtgcca cattgccagc caccaggaaa gctttctttt
3057

ttcttttttt tttttttta aacaccaaga gcacgtatag catgggggaa agaacctaaa
3117

tgtctctctg tcctgtgagc tggtgaaaaa cccagcatga gaacgcagtg tcaggtgtgg
3177

gactccttct gcccctgcag tgggtgttac gggcggtgtg ccctggcgag caagctttga
3237

ttcttggttc tttgagctcg tttcagaggc tgagtcccca catcagcttt agttcttgga
3297

cttccctgta ttaagcaaga attaggagaa tggctgtccc tgcaggcgcc tcccgtaaat
3357

cctgagctct ctggcgcaat ctgaaacttc tcttctgttt tctttggctg tatcagccga
3417

accaggagag gcctgggctg cgactaagga gaaagaaatc gggggtttct gagagcagat
3477

ggtgcctttg tgggtgcagg gcttttgtgg aaattgtcag cctctacggg cagagtccgg
3537

catcccctcc ccagactgcc tgctgtcaaa ccacggagca gctggagcct gccctgtcca
3597

cggcccgttt ccacccgggc atgttcgtct ctcatgactt cggcagaggc ccctggtggc
3657

cttcagtttc agtttctcat ccaggaaggt aaccttgggc attggcagtg ggtttcccta
3717

tggcttggat ccagattaga attgatcttt gttttcactt tccatagtta ataacatgca
3777

```
aaataatgag aagaatttat tttaaggtga cagctatact ggtccaacat cgcctgctta
   3837

ttgtcagggt acagaagttt aatactttct taatccagtt tttcaaactt ctccctgtag
   3897

accgtaagga tgaattccac aataggatcc tttttaaaat cgattttaaa ttgttgccta
   3957

gtcctgccaa ggttattatg tgcatctgtt attttttccaa tacatgtaaa cagttgcagc
   4017

atgatgcttt gtttaatgtc ctgttcttaa gctcgttaga gccagttttg aaacgtttgg
   4077

tcttaccgtg aacggaggct ggcttggctt agccacgctg atgagtaagt gagggatgtc
   4137

tccatcttga gatcaccagg caagagagtt gcctgcacca ggtaagaggc caaagcccct
   4197

ggggtaacag tccccaccgc tacccgaggt aaaacaataa aagctatgtg gttgagctca
   4257

ggcctctcgt gcctggtgtc agagaaggca gagcccacag taggtgcacg gtgcaaggcc
   4317

ctgggagggc actggccagg gaaggtggta tagatggccc tcagattgcg gggccccgag
   4377

cagctcccca ctctgcccgt ccaccttccc tggctccagc ctcattctct ctttagttta
   4437

actatgcaaa gagaggaggt tgagagtgtt ctggcagctg gagctctttt ccttgtcctt
   4497

cctgccctcc gatggggcca cctgtgtcgg ggcagcagtg tccatgttta tggagatcag
   4557

aggtgtcccc actgtgtggc tggactgtac tctgctgccc gggtagccag gagtctctcc
   4617

ctctctcccc tgccgcctgc ctggtctcat gggcctcctt cacacacccc tccctgtgga
   4677

tcgcctgcct gggcccagag caggggaact ggagtttgtg agtgagcaga gcaggttatg
   4737

tgcagacagg gaaacgagaa ctttggacct ggctttctga gtccaggtga gagctgtgtg
   4797

gccccccgat gccactctgc ccgccggagg gatgtgcctg ctgagccttt ccttccacg
   4857

ccgcctctca ctgccaggcc agcggcttcc gctgagactc gctggagagg cggctcccgt
   4917

gtccgtccac cgagcactca gatggatgct gatcaccagg gccgaggggg ctcccagaag
   4977

gaccccaggc cctggggagg gtggctgtgg gaggccaagt ccactgcccg gaagtcttgt
   5037
```

cagccctaag ccagggaagc ctggagcgtg gcctggcggg tctgggtgga caccgtcccc

actccggact cccagcacag gggaggatac ctgagcctgt atggccctgt agccctgggc

agagctgggc ctgtcgtgtg ttcctgcctg gcaggtgcag gtgctggcca tctgcaggtg

gaaggaggtg ggaatcttgg attttttgtt tttttttgtt tttttttttt tgagatgaag

tctcgctctg acacccaggc tggcgtgcag tggtgtgatc tcggctcact gcaaactccg

cttcctgggt tcaagtggtt ctcctgcccc agcctcccaa gtagctggga ttacaggcat

gcgccaccac gctcagctga tttttgtatt tttagtagag atggggtttc accatgttgg

ccaagctggt ctcaaactcc tgacctcaag tgatctgccc gcctcggcct cccagagtgc

tgggattaca ggcatgagcc agtgcacccg gcggaatctt ggaattttta tagacagcac

ctcagtttct gactccagcc gcacacctcc tgcctctgcc agcaggggtt gccgccagac

cagagccagg gccaggtccc tgcgtccatc ccccccggta ggatggacgt gagccatcct

tctaggggac ttttttcagt gtgcgactcg tctctgttag gtggtaggag ccagtttgtg

tggcctgtgc cacgctccac agtgcgtggc tgggctctgt gtgtggcctg tgtcccctgt

ccctgcagga cccagcaggc atcgtggcgt gacagctgtg tccaagccac tgcccgggca

tcccatcacc caccagggtg cacggtctct cctgctgggg gctttctgtc gcatgtgtgt

ctcctgtcga ctctgcagtt tgttctcaga gcagaatgtt tcctgttctc agtgcacaaa

gacactggtt ttcaatcggc gtctaaaacc acgttcctgc ctttcattgc aacacggtgt

gttcatttgt ttaaaacagt ttaatgagta agtttagatg actggtcaat atcttaaaaa

tgtatattag taagaagttc ttcctggaat ttttctttcg attctggcag aataaacagg

tgtttttagt tttcccactg tctgagccaa gcaggaccct gtcccagagc aagagatgtc

cccttccatc tctgaccctt gcctgggaca agctttgatg gggggcccca gcttcaaggc

6297

tgtggtggga acagcacccc caaatgccag cctctccttt cttcccatcc accagtatac
6357

tgcggggcca tttctggtct ttgtccaaca ggaaacccat ttctggtggg atatgccttc
6417

cagtgccaca gggccactca ccccatgcat ctctgtcctg cccgtcagtg ctgggacgga
6477

cagcaagggc aagcccagtg tctggcggat aggtgggtgg aacagagag gggagaatgc
6537

cgtcctaagc ttctgcttgg ggatccccca cacgacctgg gtactgcctg ggaaacctgt
6597

cctaagtaaa actatggacc tcgcctcgcc caccggcctg cgaagccagc atctccgtga
6657

aggtggatgg aagcgccttt gtcctcattt tgagctgcaa gctgggtcag cggctctgaa
6717

gccctcgagt gactttctaa cccaagaccc agcccctggc aggaggaggg tgggtgcagg
6777

gctggtggga caaaaagagg cctcagcagg cctggaagac ccttccagta catcccacag
6837

cgtgtcgagc agctgggaga acctgtgtca agctcgagcc gtcataggtc cccatgaggt
6897

gtctgaagcc ccttcttggt gatgggaggc agaggtgctg acgttctgga gcatggacgt
6957

gagtcctcag ctggctccgc gtgggccctt ggagggtgcc aggtgtgtgg tgaccttctg
7017

gatgccttta acttcatggc tgcgtcattc ctgatttaga actttaaccg gagcttcatc
7077

tagtgattgc aaaactggac caatgggagg acggcggcgc agcccgctcc ctccgtggaa
7137

tggagctcag ctcttcggag gcatcaaagc acctgtcgcc tccgtggtcc ccctgctgag
7197

ggagtgcggc ctctgcaagg ttcgggggtg gcttcgtttg cctggagtgg ccggccctgc
7257

ttgtgccatg tggatgtttg tgagcctcgg tcctacagca ctgtgtaggc tgcatctgtt
7317

tcgtgctggt cctgttgact tgtatgatat ccacaaataa atattttcat ggcggtcgtg
7377

ttgaaaaaaa aaa
7390


<210> 4
<211> 312

```
<212>   PRT
<213>   Homo sapiens

<400>   4

Met Glu Glu Glu Cys Arg Val Leu Ser Ile Gln Ser His Val Ile Arg
1               5                   10                  15


Gly Tyr Val Gly Asn Arg Ala Ala Thr Phe Pro Leu Gln Val Leu Gly
                20                  25                  30


Phe Glu Ile Asp Ala Val Asn Ser Val Gln Phe Ser Asn His Thr Gly
            35                  40                  45


Tyr Ala His Trp Lys Gly Gln Val Leu Asn Ser Asp Glu Leu Gln Glu
        50                  55                  60


Leu Tyr Glu Gly Leu Arg Leu Asn Asn Met Asn Lys Tyr Asp Tyr Val
65                  70                  75                  80


Leu Thr Gly Tyr Thr Arg Asp Lys Ser Phe Leu Ala Met Val Val Asp
                85                  90                  95


Ile Val Gln Glu Leu Lys Gln Gln Asn Pro Arg Leu Val Tyr Val Cys
                100                 105                 110


Asp Pro Val Leu Gly Asp Lys Trp Asp Gly Glu Gly Ser Met Tyr Val
            115                 120                 125


Pro Glu Asp Leu Leu Pro Val Tyr Lys Glu Lys Val Val Pro Leu Ala
            130                 135                 140


Asp Ile Ile Thr Pro Asn Gln Phe Glu Ala Glu Leu Leu Ser Gly Arg
145                 150                 155                 160


Lys Ile His Ser Gln Glu Glu Ala Leu Arg Val Met Asp Met Leu His
                165                 170                 175


Ser Met Gly Pro Asp Thr Val Val Ile Thr Ser Ser Asp Leu Pro Ser
                180                 185                 190


Pro Gln Gly Ser Asn Tyr Leu Ile Val Leu Gly Ser Gln Arg Arg Arg
            195                 200                 205


Asn Pro Ala Gly Ser Val Val Met Glu Arg Ile Arg Met Asp Ile Arg
        210                 215                 220


Lys Val Asp Ala Val Phe Val Gly Thr Gly Asp Leu Phe Ala Ala Met
```

```
                225                 230                 235                 240

        Leu Leu Ala Trp Thr His Lys His Pro Asn Asn Leu Lys Val Ala Cys
                        245                 250                 255

        Glu Lys Thr Val Ser Thr Leu His His Val Leu Gln Arg Thr Ile Gln
                        260                 265                 270

        Cys Ala Lys Ala Gln Ala Gly Glu Gly Val Arg Pro Ser Pro Met Gln
                    275                 280                 285

        Leu Glu Leu Arg Met Val Gln Ser Lys Arg Asp Ile Glu Asp Pro Glu
                290                 295                 300

        Ile Val Val Gln Ala Thr Val Leu
        305                 310
```

```
<210>    5
<211>    21
<212>    DNA
<213>    artificial sequence

<220>
<223>    siACHE_1

<400>    5
cgccctctgg ctgcaaataa a
      21


<210>    6
<211>    21
<212>    DNA
<213>    artificial sequence

<220>
<223>    siACHE_2

<400>    6
ccagagtgtc tgctaccaat a
      21


<210>    7
<211>    21
<212>    DNA
<213>    artificial sequence

<220>
<223>    siADAR_1

<400>    7
tccctcgata acagtcagct a
      21
```

<210> 8
<211> 21
<212> DNA
<213> artificial sequence

<220>
<223> siADAR_2

<400> 8
ttccgttacc gcagggatct a
21

<210> 9
<211> 21
<212> DNA
<213> artificial sequence

<220>
<223> siALDH7A1_1

<400> 9
tccgattctc tatgtcttta a
21

<210> 10
<211> 21
<212> DNA
<213> artificial sequence

<220>
<223> siALDH7A1_2

<400> 10
aaggatgatt ggaggaccta t
21

<210> 11
<211> 21
<212> DNA
<213> artificial sequence

<220>
<223> siALK_1

<400> 11
cacctacgta tttaagatga a
21

<210> 12
<211> 21
<212> DNA
<213> artificial sequence

<220>
<223> siALK_2

<400> 12
ctcgaccatc atgaccgact a

21

```
<210>   13
<211>   21
<212>   DNA
<213>   artificial sequence

<220>
<223>   siAPAF1_1

<400>   13
cagtgaaggt atggaatatt a                21


<210>   14
<211>   21
<212>   DNA
<213>   artificial sequence

<220>
<223>   siAPAF1_2

<400>   14
taggcagagt ataaagtatt a                21


<210>   15
<211>   21
<212>   DNA
<213>   artificial sequence

<220>
<223>   siASTL_1

<400>   15
caggctttga aatcaacttc a                21


<210>   16
<211>   21
<212>   DNA
<213>   artificial sequence

<220>
<223>   siASTL_2

<400>   16
tcccatcttc agaagcagga a                21


<210>   17
<211>   21
<212>   DNA
<213>   artificial sequence

<220>
<223>   siBAIAP3_1
```

<400> 17
gtcgaccttg ctggacatta a
21


<210> 18
<211> 21
<212> DNA
<213> artificial sequence

<220>
<223> siBAIAP3_2

<400> 18
ctcgcctgac tccatccaga a
21


<210> 19
<211> 21
<212> RNA
<213> artificial sequence

<220>
<223> siBAK1_1


<220>
<221> misc_feature
<222> (20)..(21)
<223> n is dT

<400> 19
gcgaagucuu ugccuucucn n
21


<210> 20
<211> 21
<212> RNA
<213> artificial sequence

<220>
<223> siBAX_1


<220>
<221> misc_feature
<222> (20)..(21)
<223> n is dT

<400> 20
ggugccggaa cugaucagan n
21


<210> 21
<211> 21
<212> RNA
<213> artificial sequence

```
<220>
<223>  siBCL2_1


<220>
<221>  misc_feature
<222>  (20)..(21)
<223>  n is dT

<400>  21
gcugcaccug acgcccuucn n
    21


<210>  22
<211>  21
<212>  DNA
<213>  artificial sequence

<220>
<223>  siBCL2L1_1

<400>  22
aaagtgcagt tcagtaataa a
    21


<210>  23
<211>  21
<212>  DNA
<213>  artificial sequence

<220>
<223>  siBCL2L1_2

<400>  23
caagctttcc cagaaaggat a
    21


<210>  24
<211>  21
<212>  DNA
<213>  artificial sequence

<220>
<223>  siBMP7_1

<400>  24
cccggaagtt cctgtaataa a
    21


<210>  25
<211>  21
<212>  DNA
<213>  artificial sequence

<220>
<223>  siBMP7_2

<400>  25
```

```
ctcgtggaac atgacaagga a
    21
```

<210>  26
<211>  21
<212>  DNA
<213>  artificial sequence

<220>
<223>  siBSN_1

<400>  26
```
aaaggacttg atataaacaa a
    21
```

<210>  27
<211>  21
<212>  DNA
<213>  artificial sequence

<220>
<223>  siBSN_2

<400>  27
```
cacgctgtgt cccatatgca a
    21
```

<210>  28
<211>  21
<212>  DNA
<213>  artificial sequence

<220>
<223>  siBTNL2_1

<400>  28
```
atggagggac atggaaggaa a
    21
```

<210>  29
<211>  21
<212>  DNA
<213>  artificial sequence

<220>
<223>  siBTNL2_2

<400>  29
```
cagagtggga gaagatatac a
    21
```

<210>  30
<211>  21
<212>  DNA
<213>  artificial sequence

<220>

<223>  siC1OORF92_1

<400>  30
cagggcctat tgtcacttca a
   21

<210>  31
<211>  21
<212>  DNA
<213>  artificial sequence

<220>
<223>  siC1OORF92_2

<400>  31
caggagaggc gaggacctga a
   21

<210>  32
<211>  21
<212>  DNA
<213>  artificial sequence

<220>
<223>  siC1ORF91_1

<400>  32
accgtctgtc ttcacaaagt a
   21

<210>  33
<211>  21
<212>  DNA
<213>  artificial sequence

<220>
<223>  siC1ORF91_2

<400>  33
ctggcgagta gtagctgtca a
   21

<210>  34
<211>  21
<212>  DNA
<213>  artificial sequence

<220>
<223>  siC21ORF2_1

<400>  34
aagcgctttg ttggaatggt a
   21

<210>  35
<211>  21
<212>  DNA

```
<213>   artificial sequence

<220>
<223>   siC21ORF2_2

<400>   35
ccgcatgaag ctgacgcgga a
    21


<210>   36
<211>   21
<212>   RNA
<213>   artificial sequence

<220>
<223>   siCASP2_1


<220>
<221>   misc_feature
<222>   (20)..(21)
<223>   n is dT

<400>   36
acagcuguug uugagcgaan n
    21


<210>   37
<211>   21
<212>   RNA
<213>   artificial sequence

<220>
<223>   siCCBL1_1


<220>
<221>   misc_feature
<222>   (20)..(21)
<223>   n is dT

<400>   37
ccaguacacc aagacauuun n
    21


<210>   38
<211>   21
<212>   RNA
<213>   artificial sequence

<220>
<223>   siCCBL1_2


<220>
<221>   misc_feature
<222>   (20)..(21)
<223>   n is dT
```

```
<400>   38
gguccagauc acaucaugan n
      21


<210>   39
<211>   21
<212>   DNA
<213>   artificial sequence

<220>
<223>   siCD151_1

<400>   39
caccctgtgc catcaccata a
      21


<210>   40
<211>   21
<212>   DNA
<213>   artificial sequence

<220>
<223>   siCD151_2

<400>   40
ctgcctgtac aggagtctca a
      21


<210>   41
<211>   21
<212>   DNA
<213>   artificial sequence

<220>
<223>   siCD34_1

<400>   41
cacgtggtgg ctgataccga a
      21


<210>   42
<211>   21
<212>   DNA
<213>   artificial sequence

<220>
<223>   siCD34_2

<400>   42
aacggccatt cagcaagaca a
      21


<210>   43
<211>   21
<212>   DNA
<213>   artificial sequence
```

<220>
<223>  siCLIC1_1

<400>  43
aaggtgtctc tcagaggaag t
21


<210>  44
<211>  21
<212>  DNA
<213>  artificial sequence

<220>
<223>  siCLIC1_2

<400>  44
ttcctgctgt atggcactga a
21


<210>  45
<211>  21
<212>  DNA
<213>  artificial sequence

<220>
<223>  siCOMMD9_1

<400>  45
aagctcctga gtgcaactta a
21


<210>  46
<211>  21
<212>  DNA
<213>  artificial sequence

<220>
<223>  siCOMMD9_2

<400>  46
cccgctgttc tcactcatct t
21


<210>  47
<211>  21
<212>  DNA
<213>  artificial sequence

<220>
<223>  siCOX5B_1

<400>  47
cacctgcact aaattactca a
21


<210>  48
<211>  21

```
<212>   DNA
<213>   artificial sequence

<220>
<223>   siCOX5B_2

<400>   48
aggcaccagg gaagaccccta a
     21


<210>   49
<211>   21
<212>   DNA
<213>   artificial sequence

<220>
<223>   siCOX7C_1

<400>   49
ctagatatgt ttgtcaataa a
     21


<210>   50
<211>   21
<212>   DNA
<213>   artificial sequence

<220>
<223>   siCOX7C_2

<400>   50
caagtggtcg ttactagcta a
     21


<210>   51
<211>   21
<212>   DNA
<213>   artificial sequence

<220>
<223>   siCYP11B2_1

<400>   51
cactagatca tgggatccta a
     21


<210>   52
<211>   21
<212>   DNA
<213>   artificial sequence

<220>
<223>   siCYP11B2_2

<400>   52
aaggcggaac tgtcactaga a
     21
```

```
<210>   53
<211>   21
<212>   DNA
<213>   artificial sequence

<220>
<223>   siDEFB4_1

<400>   53
gccctagaag gtataaacaa a                    21


<210>   54
<211>   21
<212>   DNA
<213>   artificial sequence

<220>
<223>   siDEFB4_2

<400>   54
tgccttaaga gtggagccat a                    21


<210>   55
<211>   21
<212>   DNA
<213>   artificial sequence

<220>
<223>   siDHRSX_1

<400>   55
ccgcgttaat acaactgagt a                    21


<210>   56
<211>   21
<212>   DNA
<213>   artificial sequence

<220>
<223>   siDHRSX_2

<400>   56
tgccctgata agcaacttaa a                    21


<210>   57
<211>   21
<212>   DNA
<213>   artificial sequence

<220>
<223>   siEBP_1

<400>   57
```

```
actggacaac tttgtaccta a
    21
```

```
<210>   58
<211>   21
<212>   DNA
<213>   artificial sequence

<220>
<223>   siEBP_2

<400>   58
cacagtgtgc atggaaacca t
    21
```

```
<210>   59
<211>   21
<212>   DNA
<213>   artificial sequence

<220>
<223>   siEEF2_1

<400>   59
ccgcgccatc atggacaaga a
    21
```

```
<210>   60
<211>   21
<212>   DNA
<213>   artificial sequence

<220>
<223>   siEEF2_2

<400>   60
tgccgtttct ttcaatattt a
    21
```

```
<210>   61
<211>   21
<212>   DNA
<213>   artificial sequence

<220>
<223>   siFBXO42_1

<400>   61
ctgggatcaa atctccatta a
    21
```

```
<210>   62
<211>   21
<212>   DNA
<213>   artificial sequence

<220>
```

```
<223>   siFBXO42_2

<400>   62
ctcctgtgtg atagatgata a
   21


<210>   63
<211>   21
<212>   DNA
<213>   artificial sequence

<220>
<223>   siFLOT2_1

<400>   63
ctgcctgtcc cttctggtaa a
   21


<210>   64
<211>   21
<212>   DNA
<213>   artificial sequence

<220>
<223>   siFLOT2_2

<400>   64
caccaagatt gctgactcta a
   21


<210>   65
<211>   21
<212>   DNA
<213>   artificial sequence

<220>
<223>   siFN3KRP_1

<400>   65
ccggactagc ttaagaccaa t
   21


<210>   66
<211>   21
<212>   DNA
<213>   artificial sequence

<220>
<223>   siFN3KRP_2

<400>   66
acgagtgttc gtgaaagtga a
   21


<210>   67
<211>   21
<212>   DNA
```

<213> artificial sequence

<220>
<223> siGRLF1_1

<400> 67
caggatgttc tgggagagga a
21

<210> 68
<211> 21
<212> DNA
<213> artificial sequence

<220>
<223> siGRLF1_2

<400> 68
caccaccgaa gaggtgttta a
21

<210> 69
<211> 21
<212> DNA
<213> artificial sequence

<220>
<223> siGUCA1A_1

<400> 69
caccgataca gtgttctcca a
21

<210> 70
<211> 21
<212> DNA
<213> artificial sequence

<220>
<223> siGUCA1A_2

<400> 70
gccggtcgtt gtggactcta a
21

<210> 71
<211> 21
<212> DNA
<213> artificial sequence

<220>
<223> siHDLBP_1

<400> 71
aaggatctaa tcattgagca a
21

```
<210>   72
<211>   21
<212>   DNA
<213>   artificial sequence

<220>
<223>   siHDLBP_2

<400>   72
atccgtgaaa ttcgtgacaa a                     21


<210>   73
<211>   21
<212>   DNA
<213>   artificial sequence

<220>
<223>   siHGD_1

<400>   73
accctacaag tacaacctga a                     21


<210>   74
<211>   21
<212>   DNA
<213>   artificial sequence

<220>
<223>   siHGD_2

<400>   74
cgggaattat acaccctaca a                     21


<210>   75
<211>   21
<212>   DNA
<213>   artificial sequence

<220>
<223>   siHIRIP3_1

<400>   75
taggaagaaa cctgtggtaa a                     21


<210>   76
<211>   21
<212>   DNA
<213>   artificial sequence

<220>
<223>   siHIRIP3_2

<400>   76
cagccaaaga ggagaatcca a
```

21

<210> 77
<211> 21
<212> DNA
<213> artificial sequence

<220>
<223> siIAPP_1

<400> 77
ttagaggaca atgtaactct a
21

<210> 78
<211> 21
<212> DNA
<213> artificial sequence

<220>
<223> siIAPP_2

<400> 78
atgcagggta ttgcgaaaca a
21

<210> 79
<211> 21
<212> DNA
<213> artificial sequence

<220>
<223> siIL6R_1

<400> 79
acccagttag ctctcaagtt a
21

<210> 80
<211> 21
<212> DNA
<213> artificial sequence

<220>
<223> siIL6R_2

<400> 80
ctgcgggacc atggagtggt a
21

<210> 81
<211> 21
<212> DNA
<213> artificial sequence

<220>
<223> siITGB6_1

<400> 81
ctggaattac ttgtcagccc a
21


<210> 82
<211> 21
<212> DNA
<213> artificial sequence

<220>
<223> siITGB6_2

<400> 82
aacccttgca gtagtattcc a
21


<210> 83
<211> 21
<212> DNA
<213> artificial sequence

<220>
<223> siKHSRP_1

<400> 83
caagaggaga ttgaaactga a
21


<210> 84
<211> 21
<212> DNA
<213> artificial sequence

<220>
<223> siKHSRP_2

<400> 84
caggattcag gctgcaaagt a
21


<210> 85
<211> 21
<212> DNA
<213> artificial sequence

<220>
<223> siKIA1161_1

<400> 85
caggccagac ttcgtgcctt a
21


<210> 86
<211> 21
<212> DNA
<213> artificial sequence

```
<220>
<223>  siKIA1161_2

<400>  86
cgcggccgcc atcaaagtca a          21


<210>  87
<211>  21
<212>  DNA
<213>  artificial sequence

<220>
<223>  siLIPC_1

<400>  87
caggagaaac ccagcaaaga a          21


<210>  88
<211>  21
<212>  DNA
<213>  artificial sequence

<220>
<223>  siLIPC_2

<400>  88
ctgaagacga tcagagtcaa a          21


<210>  89
<211>  21
<212>  DNA
<213>  artificial sequence

<220>
<223>  siLOC388882_1

<400>  89
caccttggtg agaatcagga a          21


<210>  90
<211>  21
<212>  DNA
<213>  artificial sequence

<220>
<223>  siLOC388882_2

<400>  90
ctgctgtaca tgacatctct a          21


<210>  91
```

```
<211>  21
<212>  DNA
<213>  artificial sequence

<220>
<223>  siLOC389727_1

<400>  91
ccacgcagag atgacagcca a            21


<210>  92
<211>  21
<212>  DNA
<213>  artificial sequence

<220>
<223>  siLOC389727_2

<400>  92
ctggcggtga atgactttga a            21


<210>  93
<211>  21
<212>  DNA
<213>  artificial sequence

<220>
<223>  siLOC390533_1

<400>  93
cgggtggtat ggccaaagca t            21


<210>  94
<211>  21
<212>  DNA
<213>  artificial sequence

<220>
<223>  siLOC390533_2

<400>  94
atacaacagc ttcttaggta a            21


<210>  95
<211>  21
<212>  DNA
<213>  artificial sequence

<220>
<223>  siLOC440056_1

<400>  95
cccgaggctt cgctccgcaa a            21
```

<210> 96
<211> 21
<212> DNA
<213> artificial sequence

<220>
<223> siLOC440056_2

<400> 96
ctccggcgcg agcctcccaa a                21


<210> 97
<211> 21
<212> DNA
<213> artificial sequence

<220>
<223> siLOC440611_1

<400> 97
caccgaaaga gtgtttccaa a                21


<210> 98
<211> 21
<212> DNA
<213> artificial sequence

<220>
<223> siLOC440611_2

<400> 98
aaagagtgtt tcaaacgtga a                21


<210> 99
<211> 21
<212> DNA
<213> artificial sequence

<220>
<223> siLOC441115_1

<400> 99
cagagctacc tcaaacagga a                21


<210> 100
<211> 21
<212> DNA
<213> artificial sequence

<220>
<223> siLOC441115_2

<400> 100
agggaggtct ttgaaagttc a 21

<210> 101
<211> 21
<212> DNA
<213> artificial sequence

<220>
<223> siLOC442126_1

<400> 101
cagggcgggc gcagtcctgg a 21

<210> 102
<211> 21
<212> DNA
<213> artificial sequence

<220>
<223> siLOC442126_2

<400> 102
tcgcacgagc gggatactcc a 21

<210> 103
<211> 21
<212> DNA
<213> artificial sequence

<220>
<223> siLRMP_1

<400> 103
ctgggaaagt atagcatgaa a 21

<210> 104
<211> 21
<212> DNA
<213> artificial sequence

<220>
<223> siLRMP_2

<400> 104
cacaaactat atggtggtca a 21

<210> 105
<211> 21
<212> DNA
<213> artificial sequence

<220>
<223> siMAT2A_1

<400> 105
cagcaggatc ctgatgccaa a
21

<210> 106
<211> 21
<212> DNA
<213> artificial sequence

<220>
<223> siMAT2A_2

<400> 106
cagggagtgt tccctatcca a
21

<210> 107
<211> 21
<212> DNA
<213> artificial sequence

<220>
<223> siMCL1_1

<400> 107
ctggtttggc atatctaata a
21

<210> 108
<211> 21
<212> DNA
<213> artificial sequence

<220>
<223> siMCL1_2

<400> 108
cgggactggc tagttaaaca a
21

<210> 109
<211> 21
<212> DNA
<213> artificial sequence

<220>
<223> siMPP6_1

<400> 109
tggaaggtgt actaatatat a
21

<210> 110
<211> 21

```
<212>   DNA
<213>   artificial sequence

<220>
<223>   siMPP6_2

<400>   110
ctgatgattc agtaaggtta a
    21


<210>   111
<211>   21
<212>   DNA
<213>   artificial sequence

<220>
<223>   siNANOS1_1

<400>   111
caggcagtgc tcaaactaga a
    21


<210>   112
<211>   21
<212>   DNA
<213>   artificial sequence

<220>
<223>   siNANOS1_2

<400>   112
caccagttta cccataagca a
    21


<210>   113
<211>   21
<212>   DNA
<213>   artificial sequence

<220>
<223>   siNCKAP1_1

<400>   113
acgcatgaac tatgtccaga a
    21


<210>   114
<211>   21
<212>   DNA
<213>   artificial sequence

<220>
<223>   siNCKAP1_2

<400>   114
caggcatata ctagtgtctc a
    21
```

<210> 115
<211> 21
<212> DNA
<213> artificial sequence

<220>
<223> siNCOA3_1

<400> 115
caggaggaga ttataatact t          21

<210> 116
<211> 21
<212> DNA
<213> artificial sequence

<220>
<223> siNCOA3_2

<400> 116
ccgacaggca cttgaattga a          21

<210> 117
<211> 21
<212> DNA
<213> artificial sequence

<220>
<223> siNLRP1_1

<400> 117
cagcttctgc tcgccaataa a          21

<210> 118
<211> 21
<212> DNA
<213> artificial sequence

<220>
<223> siNLRP1_2

<400> 118
ctggtttgcc ttccagcact a          21

<210> 119
<211> 21
<212> DNA
<213> artificial sequence

<220>
<223> siOSTCL_1

<400> 119

```
aagcattggc tctatgactg a
    21
```

```
<210>  120
<211>  21
<212>  DNA
<213>  artificial sequence

<220>
<223>  siOSTCL_2

<400>  120
aaaggctgtc caatcctcta a
    21
```

```
<210>  121
<211>  21
<212>  DNA
<213>  artificial sequence

<220>
<223>  siOVCH1_1

<400>  121
tacgaggtgc atttggtata a
    21
```

```
<210>  122
<211>  21
<212>  DNA
<213>  artificial sequence

<220>
<223>  siOVCH1_2

<400>  122
agcgtgtaat actgtgctca a
    21
```

```
<210>  123
<211>  21
<212>  DNA
<213>  artificial sequence

<220>
<223>  siPDXK_1

<400>  123
tcgagtgact ttctaaccca a
    21
```

```
<210>  124
<211>  21
<212>  DNA
<213>  artificial sequence

<220>
```

```
<223>  siPDXK_2

<400>  124
ccctgtatta agcaagaatt a
    21


<210>  125
<211>  21
<212>  RNA
<213>  artificial sequence

<220>
<223>  siPDXP_1


<220>
<221>  misc_feature
<222>  (20)..(21)
<223>  n is dT

<400>  125
gguuugaggg cccuugcaan n
    21


<210>  126
<211>  21
<212>  RNA
<213>  artificial sequence

<220>
<223>  siPDXP_2


<220>
<221>  misc_feature
<222>  (20)..(21)
<223>  n is dT

<400>  126
gugauaguga cucaucaaun n
    21


<210>  127
<211>  21
<212>  DNA
<213>  artificial sequence

<220>
<223>  siPLD3_1

<400>  127
ttgagggaag atgaagccta a
    21


<210>  128
<211>  21
<212>  DNA
<213>  artificial sequence
```

<220>
<223> siPLD3_2

<400> 128
ccgcatggtg gacatgcaga a     21


<210> 129
<211> 21
<212> DNA
<213> artificial sequence

<220>
<223> siPLK1S1_1

<400> 129
aaggaaatat gtgaatctga a     21


<210> 130
<211> 21
<212> DNA
<213> artificial sequence

<220>
<223> siPLK1S1_2

<400> 130
cagcggtgca ttcgagacaa a     21


<210> 131
<211> 21
<212> DNA
<213> artificial sequence

<220>
<223> siPPM1B_1

<400> 131
caaccaagtg tttagaatga a     21


<210> 132
<211> 21
<212> DNA
<213> artificial sequence

<220>
<223> siPPM1B_2

<400> 132
tagcctaact acacacatca a     21


<210> 133

```
<211>   21
<212>   DNA
<213>   artificial sequence

<220>
<223>   siPPM1J_1

<400>   133
aacgagatgg ataaaggtga a
        21


<210>   134
<211>   21
<212>   DNA
<213>   artificial sequence

<220>
<223>   siPPM1J_2

<400>   134
agggatgtcc gctatatcca a
        21


<210>   135
<211>   21
<212>   DNA
<213>   artificial sequence

<220>
<223>   siPRSS21_1

<400>   135
ccactttgag tggatccaga a
        21


<210>   136
<211>   21
<212>   DNA
<213>   artificial sequence

<220>
<223>   siPRSS21_2

<400>   136
acccttggcc tgtaacaaga a
        21


<210>   137
<211>   21
<212>   DNA
<213>   artificial sequence

<220>
<223>   siPSMC5_1

<400>   137
ttggtcaagg tacatcctga a
        21
```

<210> 138
<211> 21
<212> DNA
<213> artificial sequence

<220>
<223> siPSMC5_2

<400> 138
ccgggtggct ctaaggaatg a
21

<210> 139
<211> 21
<212> DNA
<213> artificial sequence

<220>
<223> siPSMD7_1

<400> 139
ttccgtattg gtcatcattg a
21

<210> 140
<211> 21
<212> DNA
<213> artificial sequence

<220>
<223> siPSMD7_2

<400> 140
caggccctaa actacacaag a
21

<210> 141
<211> 21
<212> DNA
<213> artificial sequence

<220>
<223> siRAB3C_1

<400> 141
gtggcaaata tgtgatctta a
21

<210> 142
<211> 21
<212> DNA
<213> artificial sequence

<220>
<223> siRAB3C_2

```
<400>  142
aaggacattt gccaacaatg a
    21


<210>  143
<211>  21
<212>  DNA
<213>  artificial sequence

<220>
<223>  siRAB40A_1

<400>  143
atgggaggaa gaaatagtac a
    21


<210>  144
<211>  21
<212>  DNA
<213>  artificial sequence

<220>
<223>  siRAB40A_2

<400>  144
taggctgaat aatctcctgt a
    21


<210>  145
<211>  21
<212>  DNA
<213>  artificial sequence

<220>
<223>  siRAC2_1

<400>  145
aaggagattg actcggtgaa a
    21


<210>  146
<211>  21
<212>  DNA
<213>  artificial sequence

<220>
<223>  siRAC2_2

<400>  146
caatgtgatg gtggacagca a
    21


<210>  147
<211>  21
<212>  DNA
<213>  artificial sequence
```

```
<220>
<223>  siRNF26_1

<400>  147
cactagggtc caaatacaga a                                    21


<210>  148
<211>  21
<212>  DNA
<213>  artificial sequence

<220>
<223>  siRNF26_2

<400>  148
gaccttggtg ttggacctca a                                    21


<210>  149
<211>  21
<212>  DNA
<213>  artificial sequence

<220>
<223>  siRPSAP32_1

<400>  149
cactactgaa atcctcacta a                                    21


<210>  150
<211>  21
<212>  DNA
<213>  artificial sequence

<220>
<223>  siRPSAP32_2

<400>  150
ctgactatca ttactctatg a                                    21


<210>  151
<211>  21
<212>  DNA
<213>  artificial sequence

<220>
<223>  siRRAD_1

<400>  151
cgcggtggtc tttgactgca a                                    21


<210>  152
<211>  21
```

```
<212>   DNA
<213>   artificial sequence

<220>
<223>   siRRAD_2

<400>   152
cccggacgcg atgaccctga a
       21


<210>   153
<211>   21
<212>   DNA
<213>   artificial sequence

<220>
<223>   siSEMA3C_1

<400>   153
cagaatatga ttcactattt a
       21


<210>   154
<211>   21
<212>   DNA
<213>   artificial sequence

<220>
<223>   siSEMA3C_2

<400>   154
tccctgaata ttaacaatat a
       21


<210>   155
<211>   21
<212>   DNA
<213>   artificial sequence

<220>
<223>   siSEMG2_1

<400>   155
caggtaacaa ttcatagtca a
       21


<210>   156
<211>   21
<212>   DNA
<213>   artificial sequence

<220>
<223>   siSEMG2_2

<400>   156
aaggcagtat ttcgatccaa a
       21
```

<210> 157
<211> 21
<212> DNA
<213> artificial sequence

<220>
<223> siSLC22A18AS_1

<400> 157
ctgcagcaac atagagtaca a
21

<210> 158
<211> 21
<212> DNA
<213> artificial sequence

<220>
<223> siSLC22A18AS_2

<400> 158
ctcagccagt cttaaaggca a
21

<210> 159
<211> 21
<212> DNA
<213> artificial sequence

<220>
<223> siSLC39A5_1

<400> 159
ctcctgggac ctcgtctact a
21

<210> 160
<211> 21
<212> DNA
<213> artificial sequence

<220>
<223> siSLC39A5_2

<400> 160
caggcttctg ttgctggacc a
21

<210> 161
<211> 21
<212> DNA
<213> artificial sequence

<220>
<223> siSOCS6_1

<400> 161

```
ttgatctaat tgagcattca a
    21
```

<210> 162
<211> 21
<212> DNA
<213> artificial sequence

<220>
<223> siSOCS6_2

<400> 162
```
tagaatcgtg aattgacata a
    21
```

<210> 163
<211> 21
<212> DNA
<213> artificial sequence

<220>
<223> siSTAT3_1

<400> 163
```
ctggtcttaa ctctgattgt a
    21
```

<210> 164
<211> 21
<212> DNA
<213> artificial sequence

<220>
<223> siTCTE3_1

<400> 164
```
tagaatggag ccattgaaga a
    21
```

<210> 165
<211> 21
<212> DNA
<213> artificial sequence

<220>
<223> siTCTE3_2

<400> 165
```
ctcaattgct gatataggta a
    21
```

<210> 166
<211> 21
<212> DNA
<213> artificial sequence

<220>

```
<223>   siTFF1_1

<400>   166
caccatggag aacaaggtga t
    21


<210>   167
<211>   21
<212>   DNA
<213>   artificial sequence

<220>
<223>   siTFF1_2

<400>   167
accatcgacg tccctccaga a
    21


<210>   168
<211>   21
<212>   DNA
<213>   artificial sequence

<220>
<223>   siTMED1_1

<400>   168
ccggccaaag tctaggcaga a
    21


<210>   169
<211>   21
<212>   DNA
<213>   artificial sequence

<220>
<223>   siTMED1_2

<400>   169
gaggagatgc tggatgttaa a
    21


<210>   170
<211>   21
<212>   RNA
<213>   artificial sequence

<220>
<223>   siTP53_1


<220>
<221>   misc_feature
<222>   (20)..(21)
<223>   n is dT

<400>   170
gacuccagug guaaucuacn n
```

21

```
<210>  171
<211>  21
<212>  DNA
<213>  artificial sequence

<220>
<223>  siTRIP4_1

<400>  171
cagaaatcag gcgaccatct a                                    21


<210>  172
<211>  21
<212>  DNA
<213>  artificial sequence

<220>
<223>  siTRIP4_2

<400>  172
cagctgcgaa tccaggatca a                                    21


<210>  173
<211>  21
<212>  DNA
<213>  artificial sequence

<220>
<223>  siUBE2L3_1

<400>  173
accaccgaag atcacattta a                                    21


<210>  174
<211>  21
<212>  DNA
<213>  artificial sequence

<220>
<223>  siUBE2L3_2

<400>  174
aaggagcagc accaaatcca a                                    21


<210>  175
<211>  21
<212>  DNA
<213>  artificial sequence

<220>
<223>  siUBE2T_1
```

<400> 175
tcgcaactgt gttgacctct a
21


<210> 176
<211> 21
<212> DNA
<213> artificial sequence

<220>
<223> siUBE2T_2

<400> 176
aagagagagc tgcacatgtt a
21


<210> 177
<211> 21
<212> RNA
<213> artificial sequence

<220>
<223> siUNR


<220>
<221> misc_feature
<222> (20)..(21)
<223> n is dT

<400> 177
gccgguaugc cgguuaagun n
21


<210> 178
<211> 21
<212> DNA
<213> artificial sequence

<220>
<223> siUSP8_1

<400> 178
caggttcagg caagccattt a
21


<210> 179
<211> 21
<212> DNA
<213> artificial sequence

<220>
<223> siUSP8_2

<400> 179
aaggctcgta ttcatgcaga a
21

```
<210>  180
<211>  21
<212>  RNA
<213>  artificial sequence

<220>
<223>  siVDAC1_1


<220>
<221>  misc_feature
<222>  (20)..(21)
<223>  n is dT

<400>  180
guacggccug acguuuacan n                                      21


<210>  181
<211>  21
<212>  DNA
<213>  artificial sequence

<220>
<223>  siWSB2_1

<400>  181
cacggcttct tacgatacca a                                      21


<210>  182
<211>  21
<212>  DNA
<213>  artificial sequence

<220>
<223>  siWSB2_2

<400>  182
cacgttaatt cggaagctag a                                      21


<210>  183
<211>  21
<212>  DNA
<213>  artificial sequence

<220>
<223>  siZDHHC9_1

<400>  183
aaccagattg tgaaactgaa a                                      21


<210>  184
<211>  21
```

```
<212>   DNA
<213>   artificial sequence

<220>
<223>   siZDHHC9_2

<400>   184
agcaggaatg gcagtaataa a        21


<210>   185
<211>   21
<212>   DNA
<213>   artificial sequence

<220>
<223>   siZNF878_1

<400>   185
aagcattatc ttatcttgta a        21


<210>   186
<211>   21
<212>   DNA
<213>   artificial sequence

<220>
<223>   siZNF878_2

<400>   186
tagagttgcc tcacaactta a        21
```

## Description

The present disclosure generally relates to the fields of genetics, immunology and medicine. The inventors more particularly disclose the identification of human genes and expression products thereof which can be used to assess the prognosis of a cancer in a subject, to assess the sensitivity of a subject to a treatment of cancer, or monitor, in particular determine the efficacy, of such a treatment of cancer after a given period of time. Said human genes and expression products can further be used (i) in the prevention or treatment of cancer, in particular to determine or select the appropriate cancer treatment for a given subject and/or for a particular tumor, as well as (ii) for the screening of therapeutically active drugs. The present disclosure further describes particular compounds capable to compensate, in a subject, an abnormal expression of one of the herein described products, in particular when the subject is exposed to a therapeutic treatment of cancer thereby allowing or improving its efficiency in the subject. Inventors in addition provide kits and DNA chips usable in the context of the present invention.

## BACKGROUND ART

Cancer is the major cause of mortality in most industrialized countries. Non-small cell lung cancer (NSCLC), in particular, is one of the leading causes of cancer-related death among males, both in industrialized and developing countries {Beers, 2008; Jemal, 2008}. In spite of encouraging progress in developing personalized treatments, only a small fraction of NSCLC patients bear tumors whose specific characteristics allow them to benefit from therapies targeting specific growth receptors (such as the epidermal growth factor receptor, EGFR) {Kancha, 2009} or signal transducers (such as the anaplastic lymphoma kinase, ALK) {Soda, 2007}. Most NSCLC patients receive cytotoxic chemotherapeutics including platinum-based agents such as cisplatin (cis-diammineplatinum(II) dichloride, CDDP) and carboplatin {Cosaert, 2002; Seve, 2005} yielding highly heterogeneous therapeutic responses.

CDDP is used to treat various types of carcinomas (e.g., lung and ovarian cancer), sarcomas, lymphomas, and germ cell tumors. CDDP is a DNA damaging agent that induces 1,2-intrastrand d(GpG), 1,2-intrastrand d(ApG) and 1,3-intrastrand d(GpXpG) adducts. This latter type of adduct is readily excised by the nucleotide excision repair (NER) {Martin, 2008}, and the NER rate likewise determines the efficacy of CDDP-based chemotherapy *in vivo*. Thus, the expression level of ERCC1, one of the principal enzymes involved in NER, is predictive of the response to CDDP in NSCLC. Thus, patients with completely resected ERCC1-negative NSCLC benefit from adjuvant CDDP-based chemotherapy, whereas patients with ERCC1-positive tumors do not {Olaussen, 2006}.

Nonetheless, the response to CDDP is determined by multiple factors beyond NER. For instance, the ABC transporter breast cancer resistance protein (BCRP) can mediate CDDP resistance by increasing the efflux of CDDP from cells {Ota, 2009}. Downstream of the DNA damage response, the expression level of cell cycle blockers (such as the cyclin-dependent kinase inhibitor $p^{27KIP1}$) {Osoegawa, 2004}, and that of anti-apoptotic proteins (such as survivin and SMAC) may influence the clinical response to "adjuvant chemotherapy" (this expression being herein understood as a therapy applied to a subject having a tumor after surgical resection of at least part of said tumor) {Dai, 2010}. Moreover, CDDP can trigger apoptotic changes in enucleated cells, indicating the existence of cytoplasmic structures that are targeted by CDDP {Mandic, 2003}. Other factors such as the presence of stem cell-like CD133[+] NSCLC cells may have a negative effect on the CDDP response rate {Bertolini, 2009}.

Biomarkers that provide prognostic information and predict the response of patients to chemotherapy are important in guiding therapeutic decisions. As an example, ERCCl-positivity of NSCLC tumors does not only predict the absence of any therapeutic benefit of CDDP-based chemotherapy, but also actually correlates with a detrimental outcome of chemotherapy. Treated individuals die earlier than untreated ones {Olaussen, 2006}. However, the clinical impact of ERCC1 measurements is marginal and controversial {Breen, 2008}, implying that more accurate biomarkers are urgently awaited.

There is an increasing need to identify or distinguish those patients who will respond to an existing treatment of cancer from those who will not.

Solutions to detect dysfunctions responsible for an absent or reduced response to existing treatments of cancer, in particular chemotherapeutic treatments, as well as compounds usable to overcome said dysfunctions and/or modify the response to conventional treatments of cancer further appear critical for the patient and are herein advantageously provided by inventors.

## SUMMARY

The present invention is based on the discovery by inventors of novel anti-cancer agent response modifiers (also herein identified as modifiers of the chemotherapeutic response) based on a genome-wide siRNA screen.

Inventors herein provide an extensive phenotypic, mechanistic and epistatic analysis of these factors and disclose advantageous biological applications thereof, in particular in the context of cancer prevention or treatment.

Personalized cancer therapy in particular relies on biomarkers capable of predicting the evolution of an individual tumor as well as its response to a conventional treatment of cancer. Advantageous tools usable in the "personalized" follow-up, treatment and cure of a subject having a cancer are herein disclosed.

Inventors herein notably demonstrate that the expression of proteins that are involved in intermediate metabolism, a vitamin B6-activating enzyme and a triglyceride lipase in particular, influence responses to chemotherapeutic agents, such as in particular cisplatin (CDDP), *in vitro* and *in vivo,* in suitable experimental systems. Such proteins are herein identified as cisplatin response modifiers (CRM). The level of expression of these proteins can further be used to predict the fate of patients suffering from a cancer or, in other words, to assess the prognosis of a cancer in patients, in particular a cancer selected from non-small cell lung cancer (NSCLC), small cell lung cancer (SCLC), head and neck cancer, cervical carcinoma, ovarian cancer, osteosarcoma, melanoma and colorectal cancer, in particular non-small cell lung cancer (NSCLC).

An *in vitro* or *ex vivo* method of diagnosing, assessing or monitoring the sensitivity of a subject having a tumor to a treatment of cancer (chemotherapy for example) is herein described. Such a method may advantageously be used to avoid or stop a useless treatment. Such a method of the invention preferably comprises a step of determining, in a biological sample of said subject, the absence, presence or expression level of the expression product of at least one gene selected from the genes herein identified in Table I (see below), or a step of detecting an alteration, such as a single nucleotide polymorphism (SNP), in at least one of said genes, thereby assessing or monitoring whether the subject having a tumor is responsive or resistant to the treatment of cancer. In a particular embodiment, the treatment of cancer is a conventional therapeutic treatment of cancer, preferably a chemotherapeutic treatment of cancer, even more preferably a chemotherapy using a drug selected from an alkylating agent such as camptothecin, cyclophosphamide, mechlorethamine, uramustine, melphalan, chlorambucil, ifosfamide, carmustine, lomostine, streoptozocin, busulfan, thiotepa, dacarbazine, mitozolomide, temozolomide, procarbazine, altretamine, dacarbazine, or an alkylating-like agent, in particular a platinum-based agent selected from cis-platinum (cisplatin or CDDP), carboplatin, nedaplatin, satraplatin and oxali-platinum (oxaliplatin or OXP), most preferably a platinum-based agent, in particular CDDP.

If the subject is identified, using a method according to the present invention, as resistant to a particular treatment of cancer, the method advantageously further comprises a step of selecting a "compensatory molecule", to be used, alone or in combination with the particular treatment of cancer (typically a conventional treatment of cancer as herein defined) as the appropriate therapeutic treatment of cancer for the subject.

A method of selecting an appropriate, preferably optimal, therapeutic treatment of cancer for a subject having a tumor, is therefore in addition herein described, as well as compensatory molecules for use in such a treatment of cancer, preferably in combination with a conventional treatment of cancer, in particular a chemotherapeutic treatment of cancer, in a subject identified, using a method as herein described, as resistant to the conventional treatment of cancer.

In a particular embodiment, the method of selecting an appropriate treatment of cancer for a subject having a tumor, comprises a step of determining presence, absence or expression level of the hepatic lipase (LIPC) in a biological sample of said subject, the absence of LIPC in the biological sample being the indication that a chemotherapy will be efficient in the subject, the presence of LIPC in the biological sample, or an over expression thereof when compared to a reference expression level, being, on the contrary, the indication that a chemotherapy, in particular a chemotherapy using cis-platinum (cisplatin or CDDP), more particularly as sole treatment, will be detrimental to the subject.

In another particular embodiment, the method comprises a step of determining the presence or absence of LIPC in a biological sample of said subject, the presence of LIPC in the biological sample, or an over expression thereof when compared to a reference expression level, being the indication that another molecule, in particular orlistat®, should preferably be administered to the patient together with a conventional treatment of cancer such as a chemotherapy, in particular a chemotherapy using cis-platinum.

In a further particular embodiment, the method comprises a step of determining the expression level of a compound selected from PDXK, PDXP and aldehyde dehydrogenase 7 family, member A1 (ALDH7A1) in a biological sample of said subject, the non expression thereof or a reduced expression thereof, when compared to a reference expression level, being the indication that a compound selected from vitamin B6, pyridoxal (PL), pyridoxal-5-phosphate (PLP), pyridoxamine (PM), pyridoxamine-5'-phosphate, pyridoxine (PN), pyridoxine-5'-phosphate, L-2-aminoadipate and L-2-aminoadipate 6-semialdehyde, should preferably be administered to the patient together with a conventional treatment of cancer such as a chemotherapy, in particular a chemotherapy using cis-platinum.

Herein disclosed is also a method for screening or identifying a compound suitable for improving the treatment of a cancer (also herein identified as "compensatory molecule") in a subject having a tumor, said method comprising determining the ability of a test compound to modify the expression in particular of at least one gene selected from the genes identified in Table I, or compensate an abnormal or altered expression thereof.

Further herein disclosed is the use of a compensatory molecule as herein described for treating a cancer or to prepare a pharmaceutical composition for treating a cancer in a subject, identified, by a method as herein described, as resistant to a treatment of cancer, typically to a conventional treatment of cancer. Preferably, the pharmaceutical composition further comprises, as a combined preparation, a drug used in a conventional treatment of cancer, for simultaneous, separate or sequential use in the treatment of said cancer.

Also herein disclosed is a method of assessing the prognosis of a cancer in a subject, the method comprising a step of determining, in a biological sample of said subject, the presence, absence or expression level of a compound selected from vitamin B6, pyridoxal (PL), pyridoxal-5-phosphate (PLP), pyridoxamine (PM), pyridoxamine-5'-phosphate, pyridoxine (PN), pyridoxine-5'-phosphate, L-2-aminoadipate and L-2-aminoadipate 6-semialdehyde, and/or of the expression product of at least one gene selected from the genes identified in Table I, or a derivative product thereof, thereby assessing the prognosis of the cancer in the subject.

Herein described is, in addition, a method of treating cancer comprising the administration to a subject in need thereof, as previously explained, of a compensatory molecule, preferably together with a drug used in a conventional treatment of cancer (as a combined preparation), the molecules being administrable together or separately in a common or unique protocol.

Further herein described is a DNA chip comprising a solid support which carries nucleic acids that are specific to at least two genes, for example three genes, selected from the genes identified in Table I.

Also herein described is a kit for assessing or monitoring the sensitivity of a subject having a tumor to a treatment of cancer, in particular a chemotherapy, or the prognosis of a cancer in a subject, wherein the kit comprises (i) detection means selected from the group consisting of a pair of primers, a probe, at least one antibody specific to the expression product of a gene selected, in particular from the genes identified in Table I, and/or specific to a derivative product thereof, and a DNA chip as herein described, and, optionally, (ii) a leaflet providing the wild-type sequence of the gene and/or the control quantitative expression value corresponding to the expression product of the gene in a control population and/or corresponding to a derivative product thereof.

## LEGENDS TO THE FIGURES

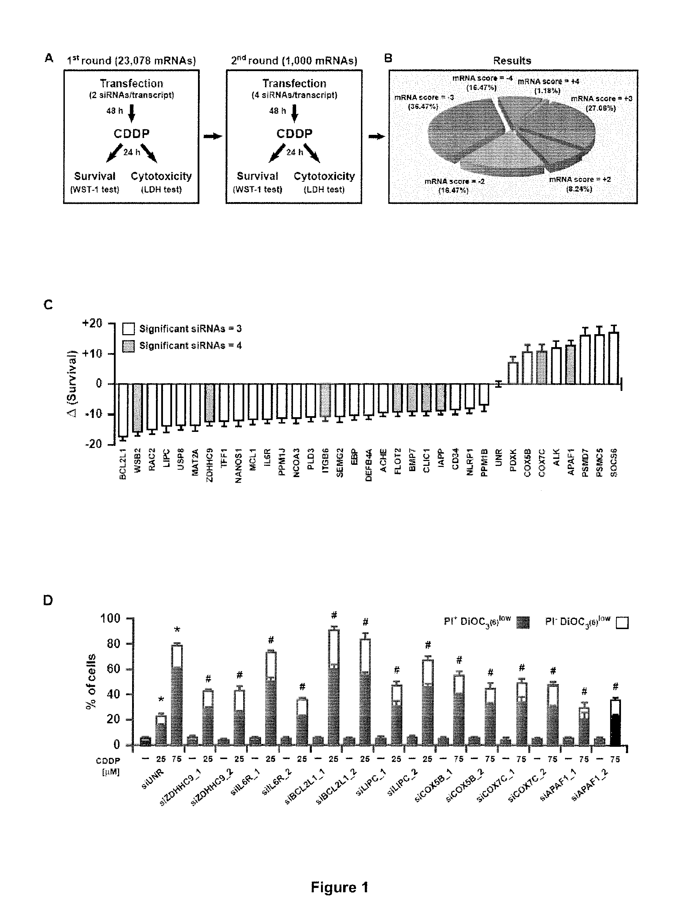

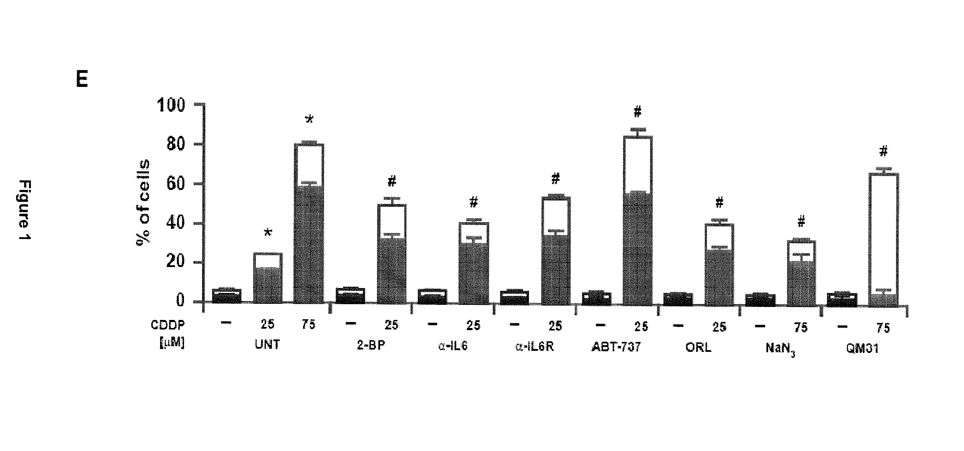

### Figure 1. Genome-wide siRNA screen for the identification of novel CRMs.

(A) siRNA screening setup. CDDP, cisplatin.

(B) Results of the 2nd round of siRNA screen. Each tested siRNA (4 x 1,000) has been given a score of -1 = statistically significant chemosensitizer, 0 = cytotoxic or antiproliferative or non-affecting CDDP-induced cell death, +1 = statistically significant cytoprotector. mRNA scores were calculated by summing the scores of the corresponding siRNAs. 85 transcripts with a score of -4, - 3, +3 or +4 were retained for further analysis. See also Table 1.

(C) Effects of the depletion of druggable hits from the 2nd round of siRNA screen on the response of A549 cells to CDDP. $\Delta$ represents the difference between the residual proliferation (upon the administration of CDDP) in siRNA-transfected cells and the residual proliferation in cells transfected with a control, non-targeting siRNA (siUNR). See also Supplementary Materials and Methods. Mean $\pm$ SEM (n = 3). All results are statistically significant (Student's $t$ test, $p < 0.05$), as compared to control conditions (UNR).

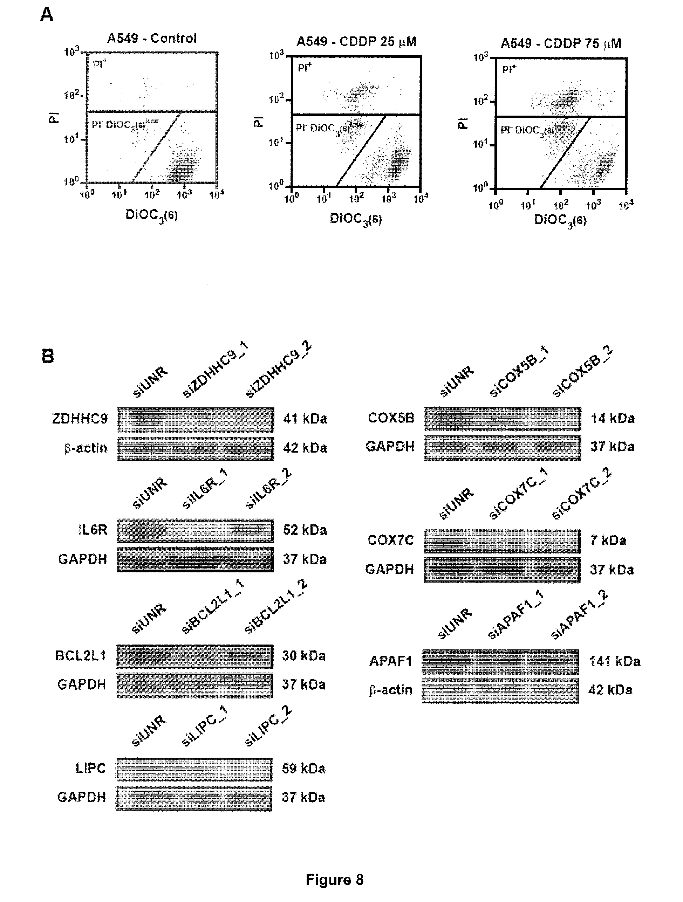

(D,E) Cytofluorometric assessment of cell death induced by 25 or 75 $\mu$M CDDP in A549 cells transfected with siRNAs targeting the indicated proteins (D) or co-treated with the indicated molecules. PI$^+$ DiOC$_3$(6)$^{low}$ = dead cells; PI$^-$ DiOC$_3$(6)$^{low}$ = dying cells. Mean $\pm$ SEM (n = 3). * = $p < 0.05$ (Student's $t$ test), as compared to untreated siUNR-transfected (D) or untransfected cells (E). # = $p < 0.05$ (Student's $t$ test), as compared to siUNR-transfected (D) or untransfected (E) cells treated with the same concentration of CDDP. See also Fig. 8A,B and Table 2.

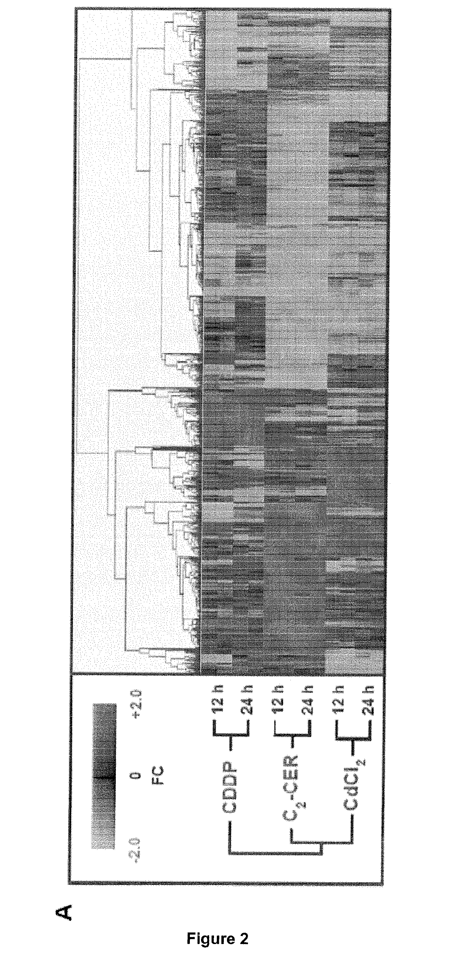

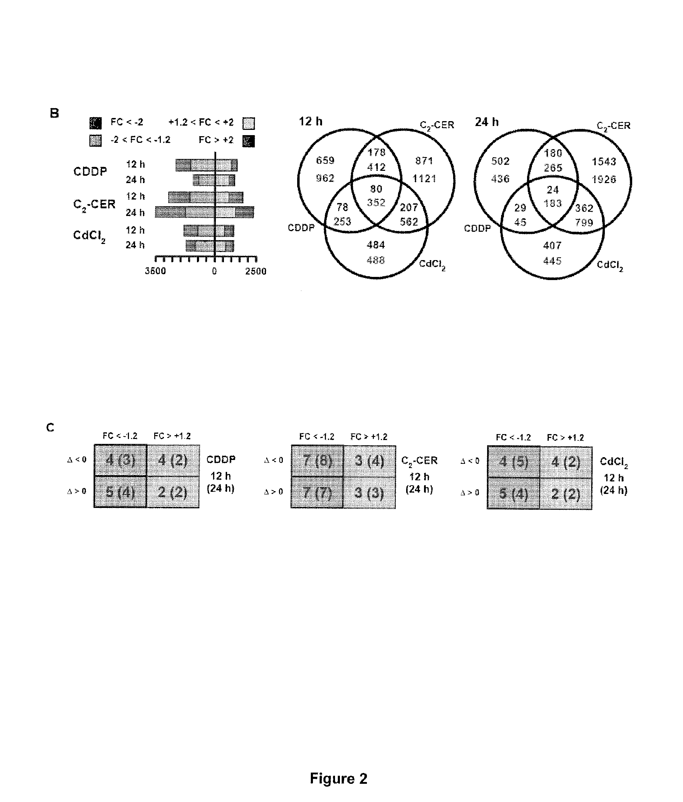

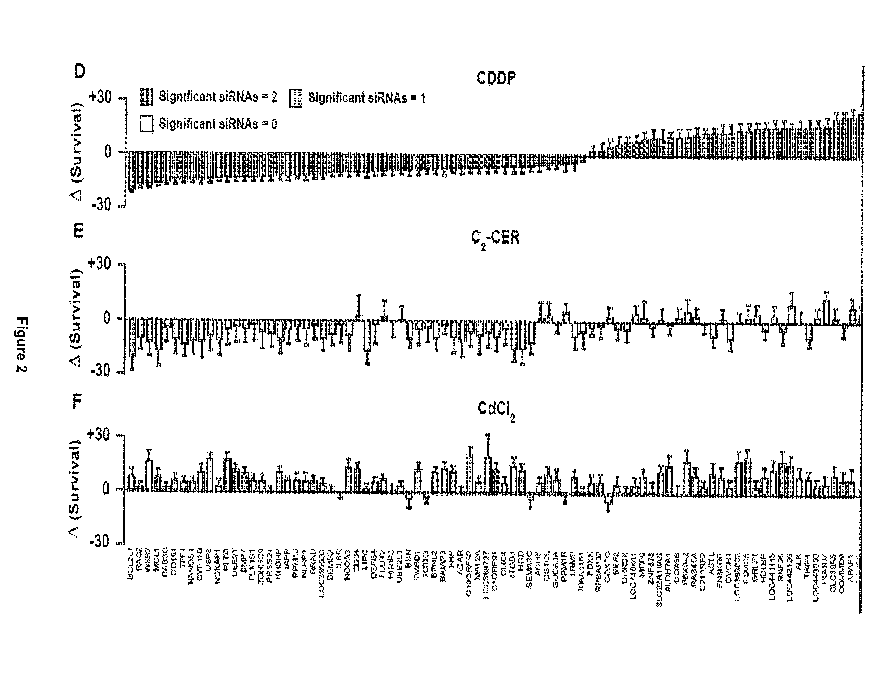

### Figure 2. Genetic and functional signatures of A549 cells responding to CDDP, C$_2$-CER and CdCl$_2$.

(A) Unsupervised hierarchical clustering of the transcriptional modifications induced in A549 cells by the administration of cisplatin (CDDP), C$_2$-ceramide (C$_2$-CER) and cadmium chloride (CdCl$_2$) for 12 or 24 h. FC = fold change, as compared to control conditions (untreated cells).

(B) Graphical representation of the absolute number of transcripts significantly up- (FC > +1.2) or downregulated (FC < -1.2) in the experimental conditions described in (A). Venn diagrams depict the overlap among these transcriptional signatures.

(C) Overlap between the 85 hits from the 2nd round of siRNA screen and the transcriptional signatures induced by CDDP, C$_2$-CER and CdCl$_2$. $\Delta$ is defined as in Fig. 1C (See also Materials and Methods of example 1). See also Table 3.

(D-F) Effects of the depletion of the 85 hits from the 2nd round of siRNA screen on the response of A549 cells to CDDP (D), C$_2$-CER (E) and CdCl$_2$ (F). $\Delta$ is defined as in Fig. 1C (See also Materials and Methods of example 1). Mean $\pm$ SEM (n = 3). Significance (Student's $t$ test, $p < 0.05$, as compared to siUNR-transfected cells) is color-coded: dark grey = 2 significant siRNAs; light grey = 1 significant siRNA; white = non significant.

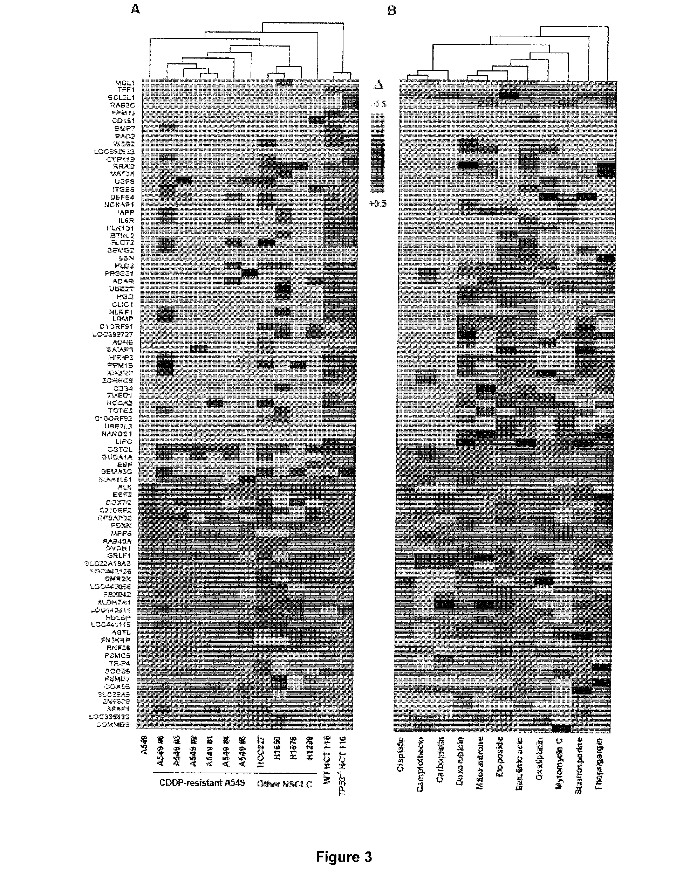

**Figure 3. Functional characterization of CRMs in multiple cancer cell lines and in A549 cells challenged with CDDP-related and -unrelated cell death inducers.**

(A) Unsupervised hierarchical clustering of the effects of the 85 hits from the 2nd round of siRNA screen on the response to cisplatin (CDDP) of wild type (WT) human non-small cell lung cancer (NSCLC) A549 cells (control condition), of six distinct CDDP-resistant A549 clones (A549 #1-6), of four NSCLC cell lines other than A549 (*i.e.,* HCC827, H1299, H1650 and H1975 cells) as well as of WT and *TP5T3*$^{-/-}$ colon carcinoma HCT 116 cells. $\Delta$ is defined as in Fig. 1C (See also Materials and Methods of example 1). See also Table 4.
(B) Unsupervised hierarchical clustering of the effects of the 85 hits from the 2nd round of siRNA screen on the response of human NSCLC A549 cells to CDDP (control condition) and to betulinic acid, carboplatin, camptothecin, doxorubicine, etoposide, mitoxantrone, mytomycin C, oxaliplatin, staurosporine, thapsigargin. $\Delta$ is defined as in Fig. 1C (See also Materials and Methods of example 1). See also Table 4.

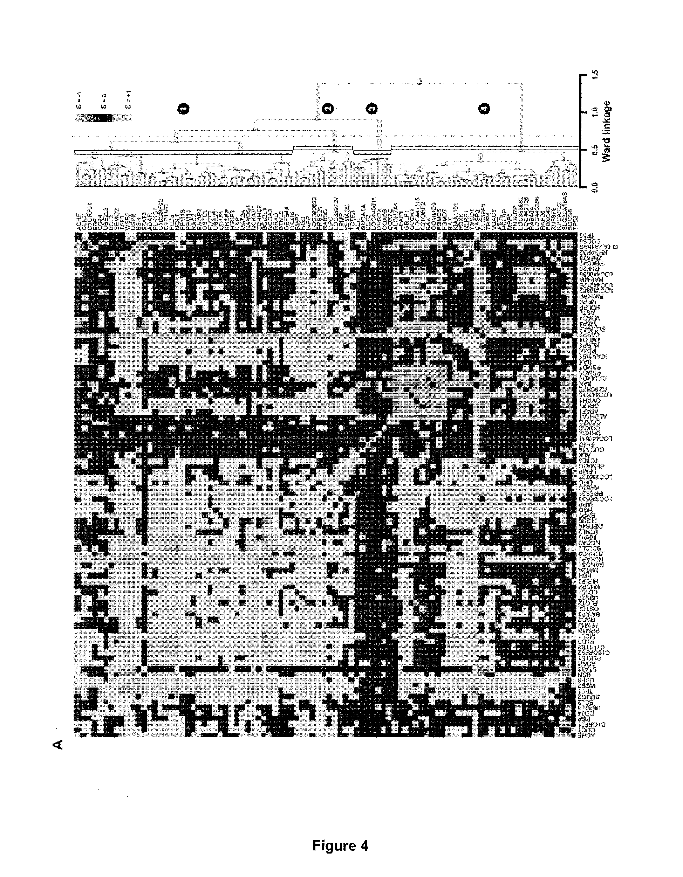

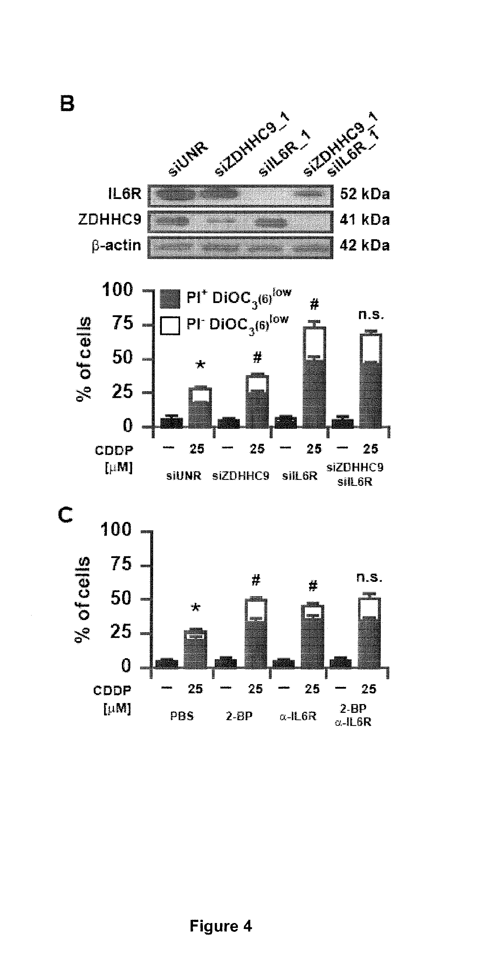

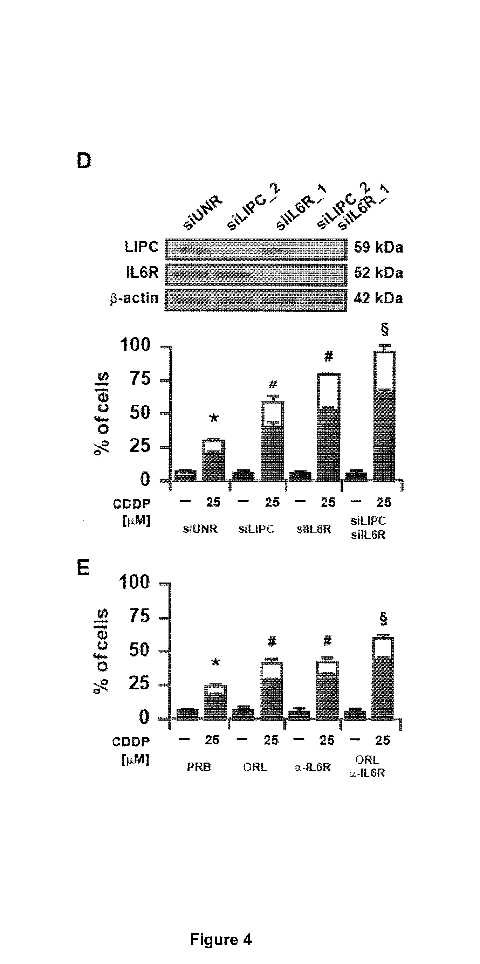

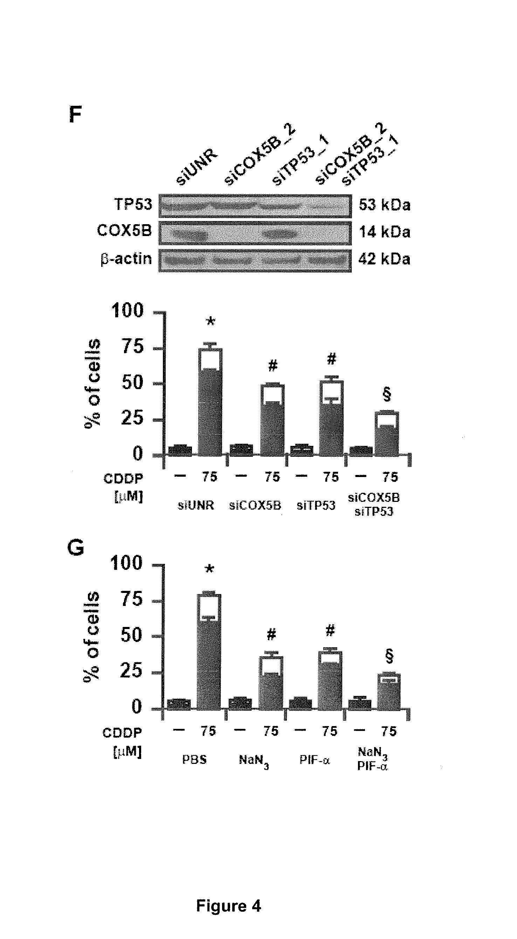

**Figure 4. Epistatic clustering and validation of the interactions between CRMs.**

(A) Epistatic clustering of the effects of the co-transfection of each of the 85 hits from the 2nd round with each other on the response of A549 cells to cisplatin (CDDP). $\varepsilon$ (varying from -1 to + 1) indicates the degree of epistatic interaction (See also Materials and Methods of example 1). This analysis allows for the identification of 4 clusters of CDDP response modifiers. See also Table 5.
(B-G) Cytofluorometric validation of the epistatic clustering depicted in A upon transfection of A549 cells with the indicated siRNAs (alone or in couples) (B,D,F) or treatment with the indicated agents (alone or in combination) (C,E,G) followed by CDDP administration. PI$^+$ DiOC$_3$(6)$^{low}$ = dead cells; PI$^-$ DiOC$_3$(6)$^{low}$ = dying cells. Mean $\pm$ SEM (n = 3). * = $p < 0.05$ (Student's $t$ test), as compared to untreated siUNR-transfected (B,D,F) or untransfected cells (C,E,G). # = $p < 0.05$ (Student's $t$ test), as compared to siUNR-transfected (B,D,F) or untransfected (C,E, G) cells treated with the same concentration of CDDP. § = $p < 0.05$ (Student's $t$ test), as compared to cells transfected with either siRNA alone (D,F) or cells treated with either pharmacological modulator alone (E,G) and the same concentration of CDDP. n.s. = non significant (Student's $t$ test), as compared to cells transfected with either siRNA alone (B) or cells treated with either pharmacological modulator alone (C) and the same concentration of CDDP. Immunoblots depict the efficiency of siRNA-mediated target downregulation. ($\beta$-actin or glyceraldehyde-3-phosphate dehydrogenase (GAPDH) levels were assessed to monitor equal loading of lanes.

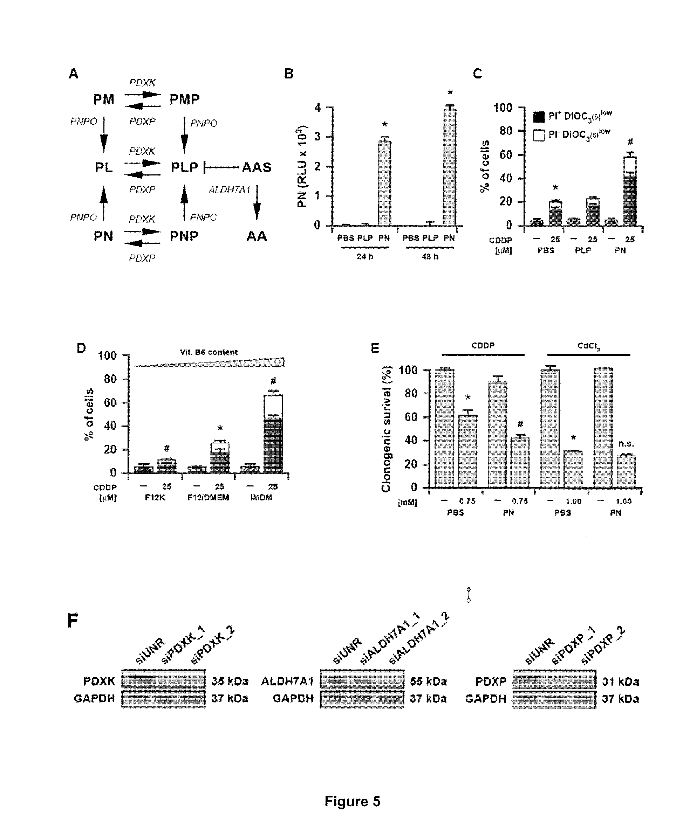

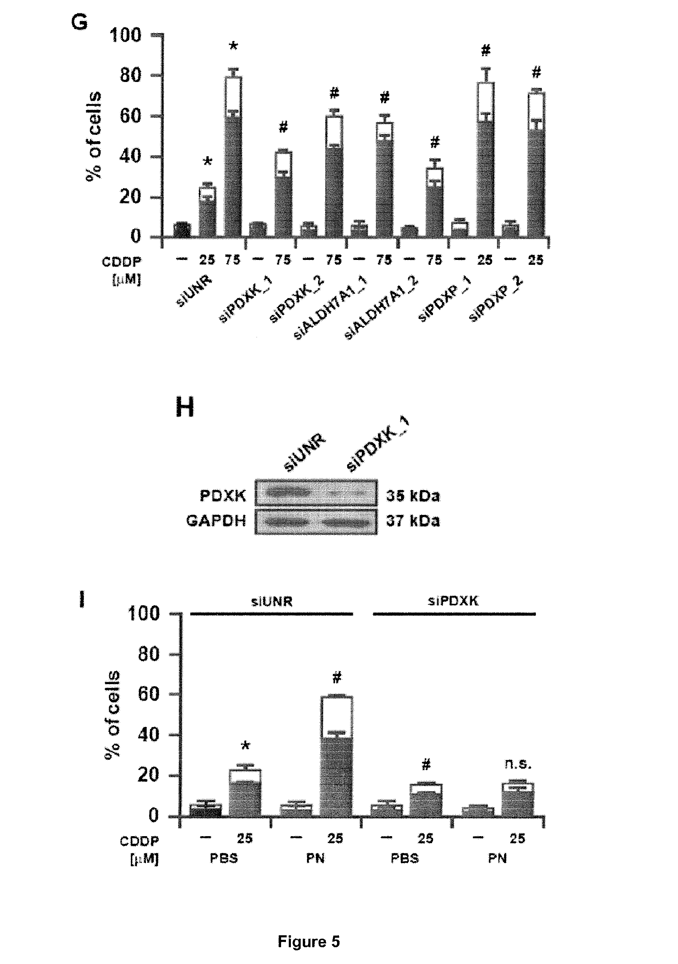

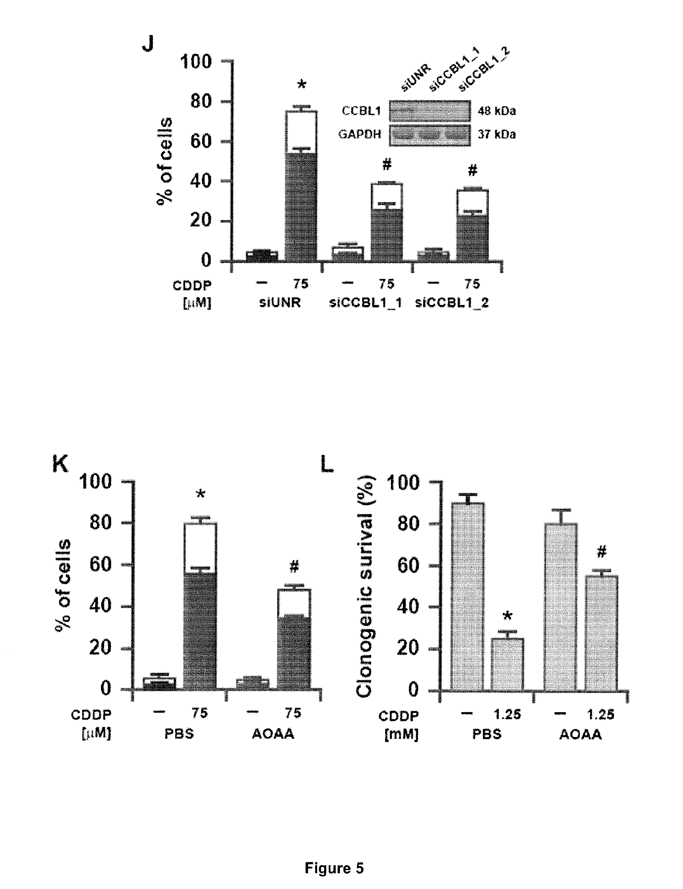

**Figure 5*. *Influence of the vitamin B6 metabolism in the response to CDDP of NSCLC cells and *Saccharomyces cerevisiae.***

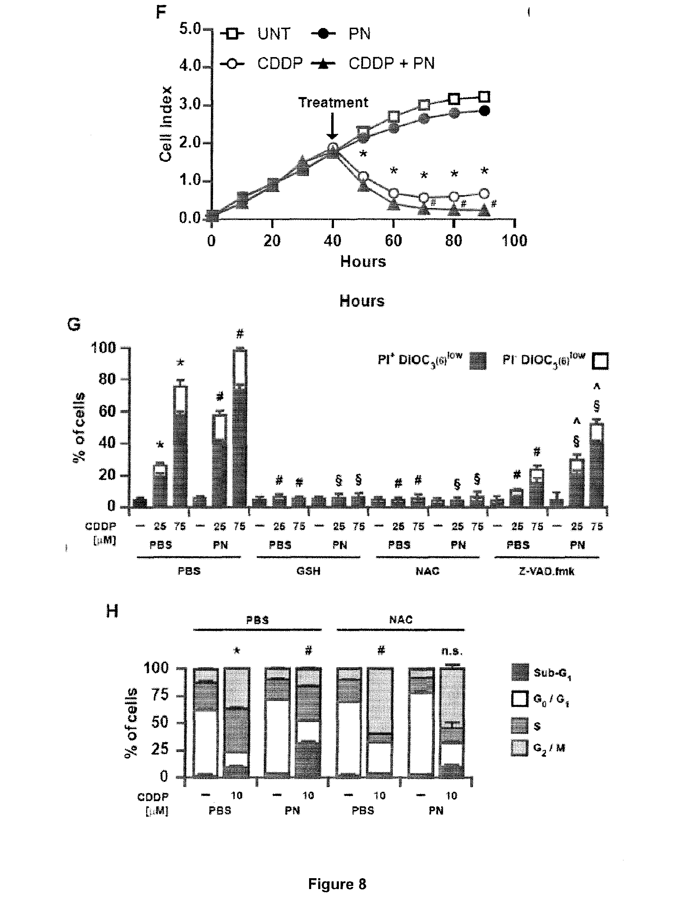

(A) Vitamin B6 metabolism in humans. AA, L-2-aminoadipate; AAS, L-2-aminoadipate 6-semialdehyde; ALDH7A1, aldehyde dehydrogenase 7 family, member A1; PDXK, pyridoxal kinase; PDXP, pyridozxal phosphatase; PNPO, pyridoxamine 5'-phosphate oxidase; PL, pyridoxal; PLP, pyridoxal-5-phosphate; PM, pyridoxamine; PMP, pyridoxamine-5'-phosphate; PN, pyridoxine; PNP, pyridoxine-5'-phosphate.
(B) Quantification of intracellular PN upon acute administration of PLP and PN to A549 cells for 24 or 48 h. * = $p < 0.001$ (Student's $t$ test), as compared to PBS-treated cells.
(C, D) Cytofluorometric assessment of cell death induced by 25 $\mu$M cisplatin (CDDP) in A549 cells co-treated with the indicated vitamin B6 vitamers for 48 h (C) or previously cultured for 1 month in media containing different amounts of vitamin B6 vitamers (D). PI$^+$ DiOC$_3$(6)$^{low}$ = dead cells; PI$^-$ DiOC$_3$(6)$^{low}$ = dying cells. Mean $\pm$ SEM (n = 3). * = $p < 0.05$ (Student's $t$ test), as compared to untreated cells cultured in standard conditions (DMEM/F12 medium). # = $p < 0.05$ (Student's $t$ test), as compared to cells maintained in standard conditions (DMEM/F12 medium) and treated with the same concentration of CDDP. See also Figure 8F.
(E) Clonogenic survival of *Saccharomyces cerevisiae* cells treated with the indicated concentration of CDDP or cadmium dichloride (CdCl$_2$) in the absence or in the presence of PN. Mean $\pm$ SEM, normalized to control conditions (n = 3). * = $p < 0.05$ (Student's $t$ test), as compared to untreated cells. # = $p < 0.05$ (Student's $t$ test), as compared to cells incubated with the same concentration of CDDP. n.s. = non significant (Student's $t$ test), as compared to cells incubated with the same concentration of CdCl$_2$.
(F, G) Cytofluorometric assessment of cell death induced by 25 or 75 $\mu$M CDDP in A549 cells transfected with siRNAs targeting the indicated vitamin B6-relevant enzymes. Immunoblots depict the efficiency of siRNA-mediated target downregulation. Glyceraldehyde-3-phosphate dehydrogenase (GAPDH) levels were monitored to ensure equal loading of lanes (F). PI$^+$ DiOC$_3$(6)$^{low}$ = dead cells; PI$^-$ DiOC$_3$(6)$^{low}$ = dying cells. Mean $\pm$ SEM

(n = 3). * = $p < 0.05$ (Student's *t* test), as compared to untreated siUNR-transfected cells. # = $p < 0.05$ (Student's *t* test), as compared to siUNR-transfected cells treated with the same concentration of CDDP (G).

(H, I) Combined effects of PDXK depletion and PN administration on CDDP-induced cell death. Immunoblots depict the efficiency of siRNA-mediated target downregulation. GAPDH levels were assessed to monitor equal lane loading (H). PI$^+$ DiOC$_3$(6)$^{low}$ = dead cells; PI$^-$ DiOC$_3$(6)$^{low}$ = dying cells. Mean $\pm$ SEM (n = 3). * = $p < 0.05$ (Student's *t* test), as compared to untreated siUNR-transfected cells. # = $p < 0.05$ (Student's *t* test), as compared to siUNR-transfected cells treated with the same concentration of CDDP. n.s. = non significant (Student's *t* test), as compared to siPDXK-transfected cells treated with the same concentration of CDDP (I).

(J, K) Cytofluorometric quantification of cell death induced by 75 $\mu$M CDDP in A549 cells transfected with siRNAs targeting cysteine conjugate-$\beta$ lyase (CCBE1) (J) or co-treated with the CCBE1 inhibitor aminooxyacetic acid (AOAA) (K). The insert in (J) shows the efficiency of siRNA-mediated CCBL1 depletion. GAPDH abundance was monitored to ensure equal loading of lanes. PI$^+$ DiOC$_3$(6)$^{low}$ = dead cells; PI$^-$ DiOC$_3$(6)$^{low}$ = dying cells. Mean $\pm$ SEM (n = 3). * = $p < 0.05$ (Student's *t* test), as compared to untreated siUNR-transfected (J) or untransfected (K) cells. # = $p < 0.05$ (Student's *t* test), as compared to siUNR-transfected (J) or untransfected (K) cells treated with the same concentration of CDDP.

(L) Clonogenic survival of yeast cells treated with the indicated concentration of CDDP alone or in the presence of AOAA. Mean $\pm$ SEM (n = 3). * = $p < 0.05$ (Student's *t* test), as compared to untreated cells. # = $p < 0.05$ (Student's *t* test), as compared to cells incubated with the same concentration of CDDP.

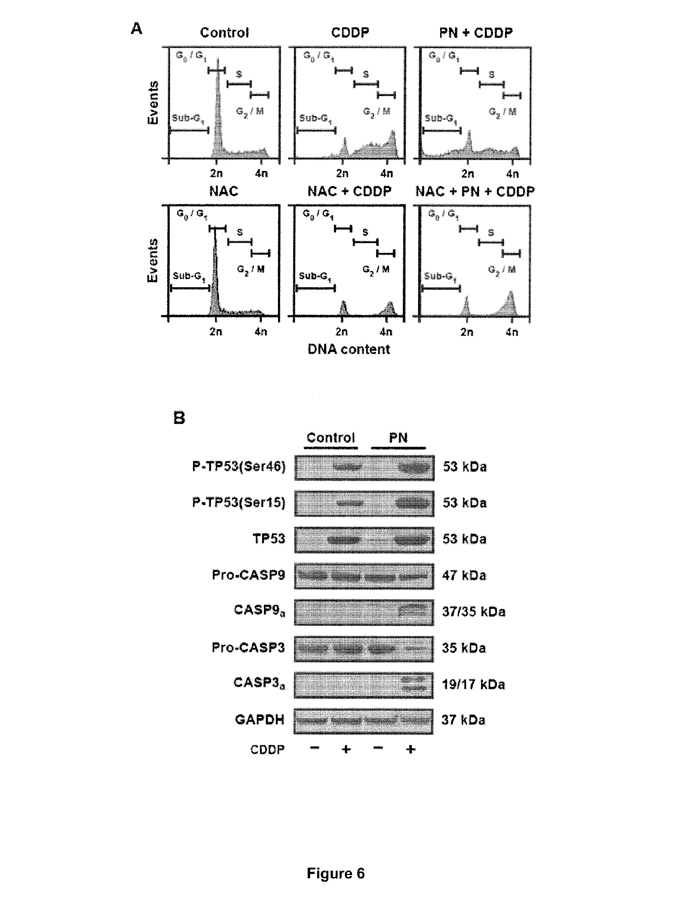

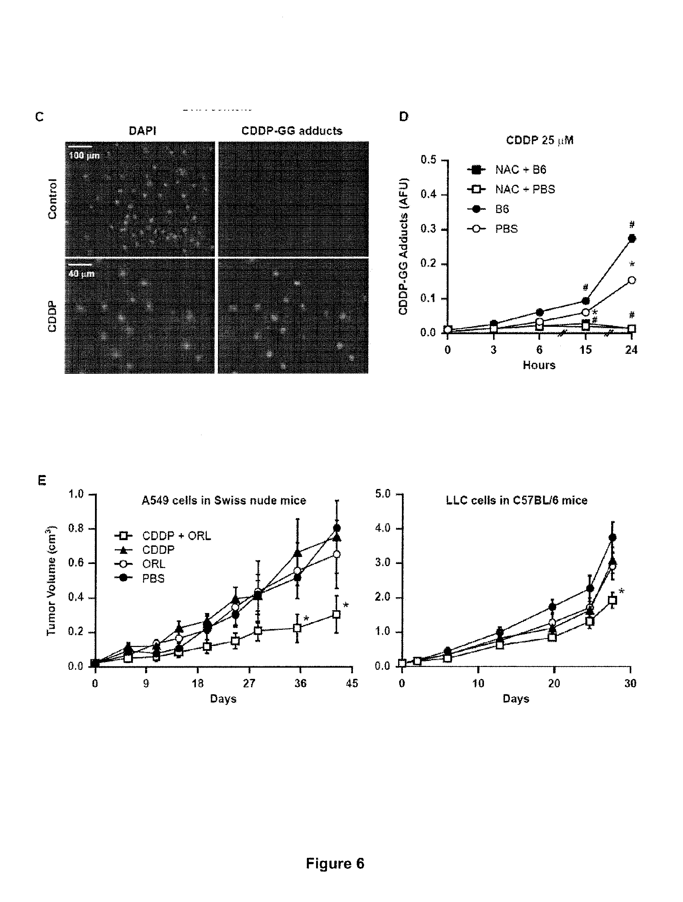

**Figure 6. Functional characterization of chemosensitization mediated by PN and LIPC inhibition *in vitro* and *in vivo*.**

(A) Cell cycle distributions of A549 cells treated with 10 $\mu$M cisplatin (CDDP) alone or in combination with pyridoxine (PN) and/or N-acetylcysteine (NAC). See also Fig. 8G, H.

(B) Immunoblotting-based assessment of the activation of the intrinsic apoptosis pathway in A549 cells treated with 25 $\mu$M CDDP alone or in combination with PN. CASP, caspase. Glyceraldehyde-3-phosphate dehydrogenase (GAPDH) levels were monitored to ensure balanced lane loading. (C, D) Immunofluorescence microscopic evaluation of CDDP-DNA adducts in A549 cells responding to 25 $\mu$M CDDP alone or combined with PN. Immunofluorescence microphotographs of untreated *versus* CDDP-treated cells. White bars indicate picture scale (C). Quantitative kinetic data as obtained upon automatic image analysis. Mean $\pm$ SEM (n = 3). * = $p < 0.05$ (Student's *t* test), as compared to untreated cells. # = $p < 0.05$ (Student's *t* test), as compared to cells treated with CDDP only (D).

(E) *In vivo* growth of human A549 cells xenografted into immunodeficient Swiss nude mice (left panel) and murine Lewis lung carcinoma (LLC) cells xenografted into immunocompetent C57BL/6 mice (right panel) upon treatment with intraperitoneal CDDP alone or in combination with orlistat. Mean $\pm$ SEM (n = 4-6 mice/group). * = $p < 0.05$ (two-way ANOVA), as compared to PBS-treated mice. See also Materials and Methods of example 1.

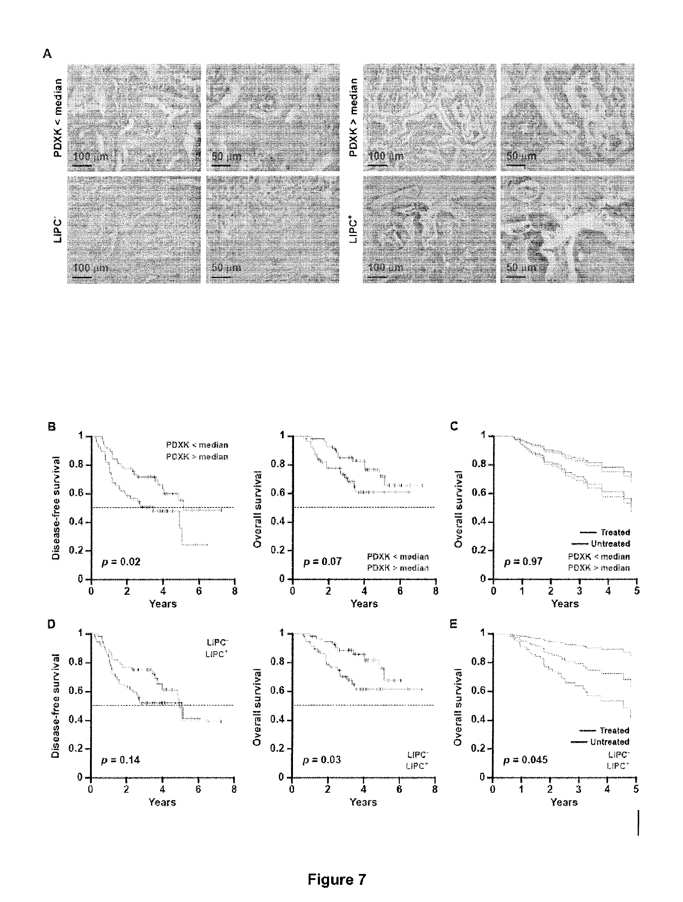

**Figure 7. Prognostic and predictive value of PDXK and LIPC status in lung cancer patients.**

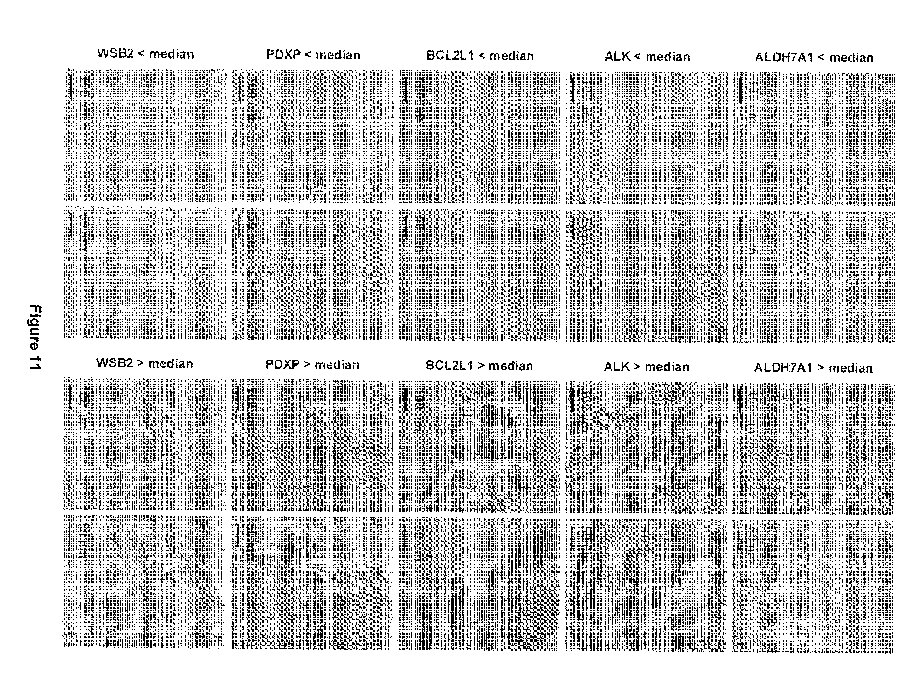

(A) Immunohistochemical detection of pyridoxal kinase (PDXK) and hepatic lipase (LIPC) levels in biopsies from lung cancer patients. Bars indicate picture scale. See also Fig. 11-13.

(B, D) Kaplan-Meier curves for disease-free survival and overall upon stratification of patients according to the median of expression of PDXK (B) or LIPC (D). *p* values (log-rank test) are depicted.

(C, E) Estimated survival curves for lung cancer patients stratified according to the median of PDXK (C) or LIPC (E) expression, based on a Cox proportional hazard model with chemotherapy marker interaction. The *p* value of the interaction is depicted.

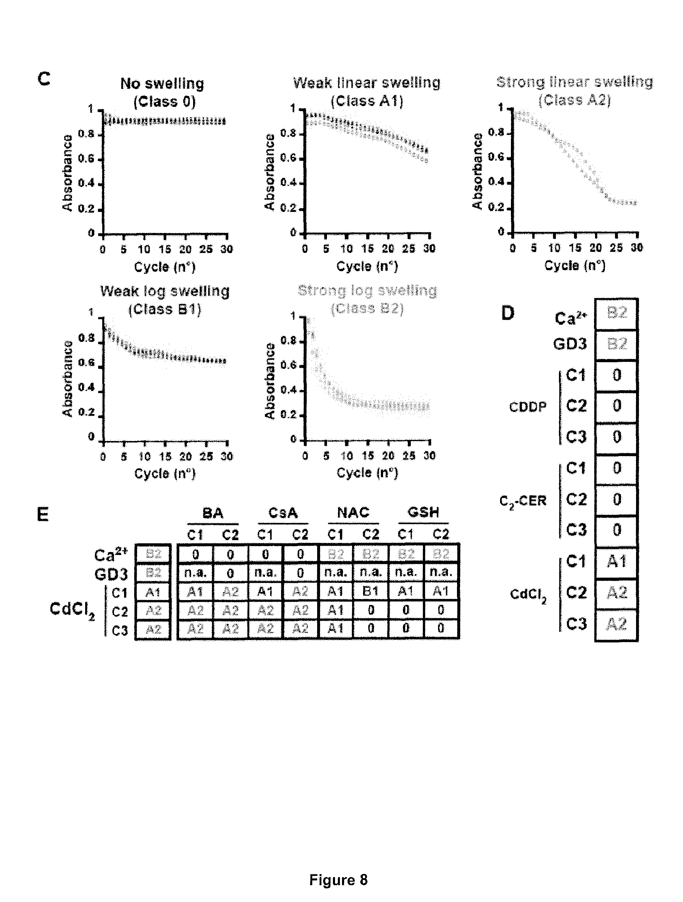

**Figure 8. Cytofluorometric quantification of cell death (A, G), immunoblotting-based assessment of the efficiency of siRNA (B), response of isolated mitochondria (C-E), detachment from the substrate of A549 cells treated with 50 $\mu$M CDDP (F), cell cycle distributions of A549 cells treated with 10 $\mu$M CDDP (H)**

(A) Cytofluorometric quantification of cell death induced by 25 or 75 $\mu$M cisplatin (CDDP) in A549 cells. PI$^+$ events = dead cells; PI$^-$ DiOC$_3$(6)$^{low}$ events = dying cells. Quantitative results are reported in Fig. 1D, E.

(B) Immunoblotting-based assessment of the efficiency of siRNA-mediated target downregulation. $\beta$-actin or glyceraldehyde-3-phosphate dehydrogenase (GAPDH) levels were assessed to monitor equal loading of lanes.

(C-E) Response of isolated mitochondria to calcium ions (Ca$^{2+}$), GD3 ganglioside (both employed as positive control conditions), CDDP, C2-ceramide (C$_2$-CER) and cadmium dichloride (CdCl$_2$). Large amplitude swelling categories based on intensity and kinetic of absorbance decrease (C). Direct mitochondrion-permeabilizing effects of the indicated molecules (D). Response of $Ca^{2+}$-, GD3- and $CdCl_2$-mediated swelling to known swelling inhibitors such as bongkrekic acid (BA), cyclosporine A (CsA), *N*-acetylcysteine (NAC) and non-oxidized glutathione (GSH). n.a. = not assessed (E).

(F) Detachment from the substrate of A549 cells treated with 50 $\mu$M CDDP alone or in combination with PN. Cell attachment was quantified as a function of the impedance of special cell culture plates integrated with microelectrodes (See also Materials and Methods of example 1). Mean $\pm$ SEM (n = 3). * = $p < 0.05$ (Student's *t* test), as compared to untreated siUNR-transfected cells. # = $p < 0.05$ (Student's *t* test), as compared to siUNR-transfected cells treated with the same concentration of CDDP.

(G) Cytofluorometric quantification of A549 cell death induced by 25 or 75 $\mu$M CDDP alone or in combination with GSH, NAC or the pan-caspase inhibitor Z-VAD-fmk. $PI^+ DiOC_3(6)^{low}$ = dead cells; $PI^- DiOC_3(6)^{low}$ = dying cells. Mean $\pm$ SEM (n = 3). * = $p < 0.05$ (Student's *t* test), as compared to untreated cells. # = $p < 0.05$ (Student's *t* test), as compared to cells treated with the same concentration of CDDP. § = $p < 0.05$ (Student's *t* test), as compared to cells treated with the same concentration of CDDP and PN. ^ = $p < 0.05$ (Student's *t* test), as compared to cells treated with the same concentration of CDDP and Z-VAD-fmk.

(H) Cell cycle distributions of A549 cells treated with 10 $\mu$M cisplatin (CDDP) alone or in combination with pyridoxine (PN) and/or N-acetylcysteine (NAC). Mean $\pm$ SEM (n = 3). * = $p < 0.05$ (Student's *t* test), as compared to untreated cells. # = $p < 0.05$ (Student's *t* test), as compared to cells treated with the same concentration of CDDP. n.s. = non significant (Student's *t* test), as compared to cells treated with the same concentration of CDDP and NAC.

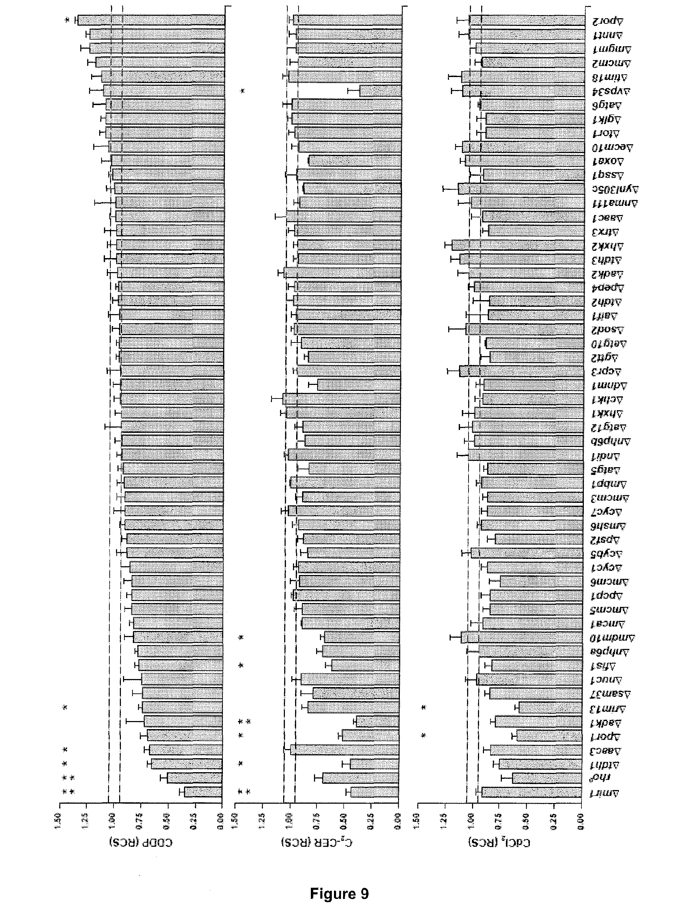

**Figure 9. Residual clonogenic survival of a panel of *Saccharomyces cerevisiae* strains**

Residual clonogenic survival (RCS, normalized to parental untreated cells) of a panel of *Saccharomyces cerevisiae* strains missing the indicated genes upon treatment with cisplatin (CDDP, upper panel), $C_2$-ceramide ($C_2$-CER, middle panel) or cadmium dichloride ($CdCl_2$, lower panel). Mean $\pm$ SEM (n = 3). * = $p < 0.05$ and ** = $p < 0.01$ (Student's *t* test), as compared to the response of parental cells. See also Materials and Methods of example 1.

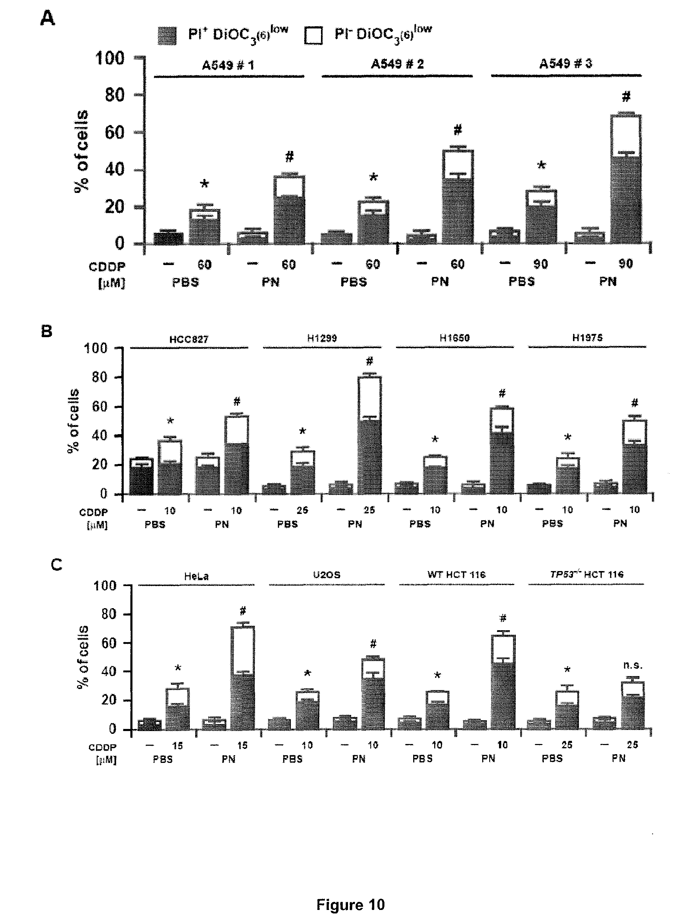

**Figure 10. Cell death induced by cisplatin (CDDP) in distinct CDDP-resistant cell lines, in 4 non-small cell lung cancer (NSCLC) cell lines, in cervical carcinoma, in osteosarcoma as well as in colon carcinoma.**

(A-C) Cytofluorometric assessment of cell death induced by the indicated sub-apoptotic dose of cisplatin (CDDP) alone or combined with PN in 3 distinct CDDP-resistant A549 clones (A549 #1-3, A), in 4 non-small cell lung cancer (NSCLC) cell lines other than A549 (HCC827, H1299, H1650 and H1975 cells, B), in cervical carcinoma HeLa cells, in osteosarcoma U20S cells as well as in wild type (WT) or $TP53^{-/-}$ colon carcinoma HCT 116 cells (C). Mean $\pm$ SEM (n = 3). * = $p < 0.05$ (Student's *t* test), as compared to untreated cells. # = $p < 0.05$ (Student's *t* test), as compared to cells treated with the same concentration of CDDP. n.s. = non significant (Student's *t* test), as compared to cells treated with the same concentration of CDDP.

**Figure 11. Immunohistochemical detection of ALDH7A1, ALK, BCL2L1, PDXP and WSB2** Immunohistochemical detection of aldehyde dehydrogenase, family 7, member A1 (ALDH7A1), anaplastic lymphoma kinase (ALK), BCL-XL (BCL2L1), pyridoxal phosphatase (PDXP) and WSB2 expression levels in bioptic specimens from lung cancer patients. Bars depict picture scale.

**Figure 12. Analysis of the distribution of the levels of expression of ALDH7A1, ALK, BCL2L1, PDXP and WSB2**

Analysis of the distribution of the levels of expression of aldehyde dehydrogenase, family 7, member A1 (ALDH7A1), anaplastic lymphoma kinase (ALK), BCL-X$_L$ (BCL2L1), hepatic lipase (LIPC), pyridoxal kinase (PDXK) and pyridoxal phosphatase (PDXP) and WSB2 in tissue samples from lung cancer patients. Histograms (diagonal) depict the distribution of the expression of each marker. Dot plots (left panels) illustrate the correlation between the expression levels of all possible couples of markers. Cutoff values for patient stratification are indicated by red dashed lines. Spearman coefficients of correlation are reported in the panels on the right of the diagonal.

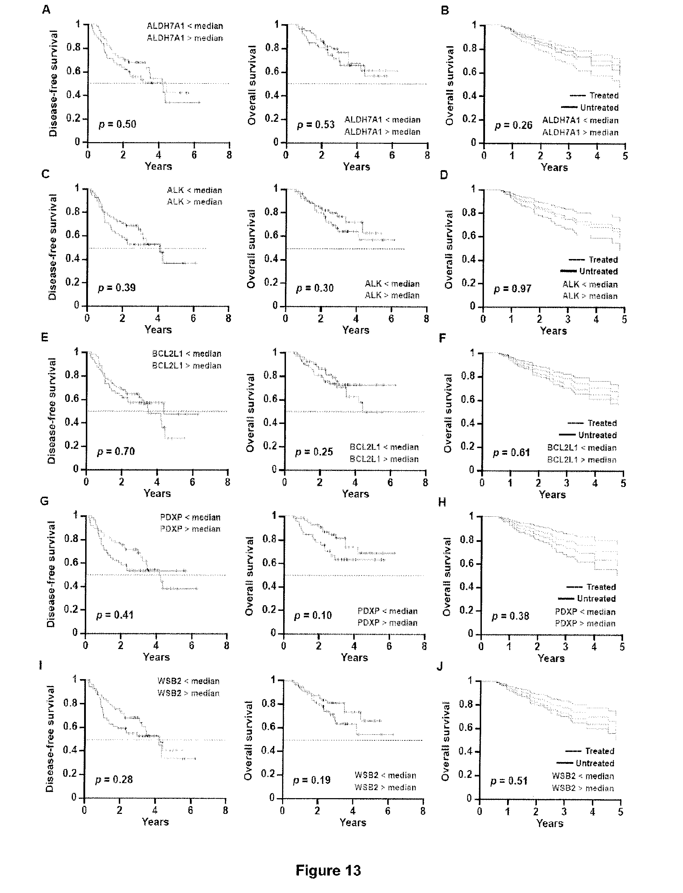

**Figure 13. No prognostic or predictive value of ALDH7A1, ALK, BCL2L1, PDXP and WSB2 status in lung cancer patients**

(A,C,E,G,I) Kaplan-Meier curves for disease-free survival and overall upon stratification of patients according to the median of expression of aldehyde dehydrogenase, family 7, member A1 (ALDH7A1, A), anaplastic lymphoma kinase (ALK, C), BCL-XL (BCL2L1, E), pyridoxal phosphatase (PDXP, G) and WSB2 (I). p values are depicted.

(B,D,F,H,J) Estimated survival curves for lung cancer patients stratified according to the median of ALDH7A1 (B), ALK (D), BCL2L1 (F), PDXP (H) or WSB2 (J) expression, based on a Cox proportional hazard model with chemotherapy marker interaction. The p value of the interaction is depicted.

## DETAILED DESCRIPTION OF THE INVENTION

Inventors herein below identify particular products (modifiers of a cancer treatment response) the detection or measure (of the expression level) of which, can be performed (i) to predictively determine if a subject having a tumor, will respond to a treatment of cancer, in particular to a conventional treatment of cancer, or will be resistant to said treatment, and/or (ii) to assess the prognosis of a cancer in a subject having a tumor, i.e., to assess whether the subject will survive to the cancer or die from the cancer.

Inventors in particular identify below methods which can be used to determine the presence, functional expression, or level of expression of the previously mentioned particular products in a biological sample of a subject having a tumor.

In the context of the present invention, the patient or **subject** is a mammal having a tumor. In a particular embodiment, the mammal is a human being, whatever its age or sex. Unless otherwise specified in the present disclosure, the tumor is a malignant or cancerous tumor.

In the context of the present invention, a **"conventional treatment of cancer"** may be selected from a chemotherapy, a radiotherapy, an hormonotherapy, an immunotherapy, a specific kinase inhibitor-based therapy, an antiangiogenic agent based-therapy, an antibody-based therapy, in particular a monoclonal antibody-based therapy, and surgery. The term "conventionally" means that the therapy is applied or, if not routinely applied, is appropriate and at least recommended by health authorities. The "conventional" treatment is selected by the cancerologist depending on the specific cancer to be prevented or treated.

The conventional treatment is typically a **chemotherapy.** In the context of a conventional chemotherapy, the treatment may use a cytotoxic agent or cell death inducer (chemotherapeutic agent), in particular a genotoxic agent. The chemotherapy may use a drug selected from an anthracyclin, a platin, a taxane and an antimitotic agent. In a preferred embodiment, the treatment of cancer is a chemotherapy using a drug selected from an alkylating agent such as camptothecin, cyclophosphamide, mechlorethamine, uramustine, melphalan, chlorambucil, ifosfamide, carmustine, lomostine, streoptozocin, busulfan, thiotepa, dacarbazine, mitozolomide, temozolomide, procarbazine, altretamine, dacarbazine, or an alkylating-like agent, in particular a platin (or platinum-based agent) selected from cis-platinum (cisplatin or CDDP), carboplatin, nedaplatin, satraplatin and oxali-platinum (oxaliplatin or OXP), most preferably a platinum-based agent, in particular CDDP.

The herein described methods of the invention may be **applied before and/or after** exposition of the subject to the conventional treatment of cancer. It is preferably applied after exposition of the subject to the conventional treatment of cancer. In a particular embodiment, the method of the invention is applied in a subject in the context of a "cancer adjuvant therapy" or "cancer conventional adjuvant therapy", this expression being herein understood, as explained previously, as a therapy applied to the subject having a tumor after surgical resection of at least part of said tumor.

In a particular and preferred embodiment of the present invention, the subject is typically a subject undergoing a treatment of cancer, in particular a conventional treatment of cancer (in particular a chemotherapy, for example a **"cancer adjuvant chemotherapy"**). This means that, typically, before assessing the prognosis of a cancer in a subject, or before assessing the sensitivity of the subject to a particular treatment of cancer, this subject has been exposed to said particular treatment of cancer. The subject may have been exposed to part of a complete conventional treatment protocol, for example to at least one cycle of the all treatment protocol, for example two, three or four cycles of the all treatment protocol.

In another particular embodiment of the present invention, the method of assessing the prognosis of a cancer in a subject or the method of assessing the sensitivity of a subject to a treatment of cancer is applied on a subject who has not been previously exposed to a conventional treatment of cancer.

In the present invention, the **cancer** may be any kind of cancer or neoplasia. The cancer is typically a cancer that is usually or conventionally treated with a chemotherapy and/or surgery. The cancer is preferably selected from non-small cell lung cancer (NSCLC), small cell lung cancer (SCLC), head and neck cancer, cervical carcinoma, ovarian cancer, osteosarcoma, melanoma and colorectal cancer, in particular non-small cell lung cancer (NSCLC).

The term **"biological sample"** means any biological sample derived from a patient, preferably a sample which contains nucleic acids or proteins. Examples of such samples include fluids, tissues, cell samples, organs, biopsies, etc. Most preferred samples are cancer or tumor tissue samples, in particular lung, skin, intestine, in particular colorectal, uterus, ovary, bone or head and neck tumor samples. Blood, plasma, serum, saliva, urine, seminal fluid, and bronchoalveolar lavage (BAL), etc, may also be used. Cancer cells from a metastase or cancer cells obtained from blood as circulating tumor cells may also be used.

The biological sample of the subject is preferably selected from a tumor sample, a blood sample, a serum sample and a bronchoalveolar lavage (BAL).

The biological sample may be treated prior to its use, *e.g.* in order to render nucleic acids or proteins available. Techniques of cell lysis, concentration or dilution of nucleic acids or proteins, are known by the skilled person.

An *in vitro* or *ex vivo* method of diagnosing, assessing or monitoring the sensitivity of a subject having a tumor to a treatment of cancer (in other words of predicting or determining the susceptibility of a patient having a tumor to respond to a treatment of cancer), in particular to a chemotherapy, is herein provided as a particular embodiment. This method comprises a step of determining, in a biological sample of said subject, the absence, presence or expression level of the expression product (mRNA or protein in particular) of at least one gene selected in particular from the genes herein below identified in Table I, or a step of detecting an alteration, such as a single nucleotide polymorphism (SNP), in at least one of said genes, thereby assessing or monitoring whether the subject having a tumor is responsive or resistant to the treatment of cancer.

Within the context of this invention, **"non-responder"** or **"resistant"** refers to the phenotype of a subject who does not respond to a treatment of cancer, in particular to a conventional treatment of cancer as herein defined, *i.e.* the volume of the tumor does not substantially decrease, or the symptoms of the cancer in the subject are not alleviated, or the cancer progresses, for example the volume of the tumor increases and/or the tumor generates local or distant metastasis. The terms "non-responder" or "resistant" also refer to the phenotype of a subject who will die from the cancer [the detected or measured parameter, for example the expression product of a gene as herein disclosed has a negative impact on the "overall survival" (OS)].

Within the context of this invention, **"responder"**, **"responsive"** or **"sensitive"** refers to the phenotype of a patient who responds to a treatment of cancer, in particular to a conventional treatment of cancer as previously defined, *i.e.* the volume of the tumor is decreased, at least one of his symptoms is alleviated, or the development of the cancer is stopped, or slowed down. Typically, a subject who responds to a cancer treatment is a subject who will be completely treated (cured), i.e., a subject who will survive to the cancer [the detected or measured parameter (for example the expression product of gene as herein disclosed) has a beneficial impact on the "overall survival" (OS)].

A subject who responds to a cancer treatment is also, in the sense of the present invention, a subject who typically has a much longer disease free survival (DFS) chance than a patient who has been identified, with a method as herein described, as resistant to a treatment of cancer.

The sensitivity or susceptibility of a subject to a treatment of cancer indicates whether the subject is "responder" or "non-responder", in other words whether the subject will or will not, be at least partially treated (tumor growth retardation or regression), preferably be completely treated (cured), by said cancer treatment.

The expression "predicting" herein refers to the likelihood that a subject will respond or not to a conventional treatment of cancer and also the extent of the response. Predictive methods of the invention can be used clinically to make treatment decisions by choosing the most appropriate treatment modalities for any particular subject. Therefore, the present invention also concerns a method for selecting a subject suffering of a cancer for a treatment with a particular conventional treatment of cancer, comprising determining the expression level of at least one gene selected from the genes listed in Table 1, in a biological sample of said subject, and selecting the subject predicted to be responsive to said treatment.

### Table 1: siRNA screening hits

| Transcripts whose depletion with at least three out of four non-overlapping siRNAs yielded significant and consistent chemosensitization or cytoprotection against cisplatin (CDDP)-induced cell death in A549 cells. Gene ID, accession number, score and $\Delta$ are reported. | | | | |
|---|---|---|---|---|
| **Name** | **Gene ID** | **Accession number** | **Score\*** | **$\Delta$\*\*** |
| BCL2L1 / BCL-X$_L$ | 598 | NM_001191 | 3 | -17.41 |
| WSB2 | 55884 | NM_018639 | 4 | -15.79 |
| RAC2 | 5880 | NM_002872 | 3 | -14.91 |
| LIPC | 3990 | NM_000236 (SEQ ID NO 1) | 3 | -13.79 |
| LRMP | 4033 | NM_006152 | 3 | -13.67 |
| USP8 | 9101 | NM_005154 | 3 | -13.60 |
| MAT2A | 4144 | NM_005911 | 3 | -13.51 |
| CYP11B2 | 1585 | NM_000498 | 3 | -12.77 |

(continued)

| Name | Gene ID | Accession number | Score* | Δ** |
|------|---------|------------------|--------|-----|
| Transcripts whose depletion with at least three out of four non-overlapping siRNAs yielded significant and consistent chemosensitization or cytoprotection against cisplatin (CDDP)-induced cell death in A549 cells. Gene ID, accession number, score and Δ are reported. | | | | |
| BTNL2 | 56244 | NM_019602 | 3 | -12.60 |
| ZDHHC9 | 51114 | NM_001008222 | 4 | -12.43 |
| TFF1 | 7031 | NM_003225 | 3 | -12.13 |
| PLK1S1 / C20orf19 | 55857 | NM_018474 | 3 | -12.10 |
| NANOS1 | 340719 | NM_199461 | 3 | -12.02 |
| LOC389727 | 389727 | Pseudogene | 3 | -11.75 |
| C10ORF92 | 54777 | NM_017609 | 3 | -11.73 |
| MCL1 | 4170 | NM_021960 | 3 | -11.71 |
| IL6R | 3570 | NM_000565 | 3 | -11.67 |
| PPM1J / PPP2CZ | 333926 | NM_005167 | 3 | -11.28 |
| NCOA3 | 8202 | NM_006534 | 3 | -11.16 |
| RRAD | 6236 | NM_004165 | 3 | -10.96 |
| PLD3 | 23646 | NM_012268 | 3 | -10.75 |
| ITGB6 | 3694 | NM_000888 | 4 | -10.70 |
| UBE2T | 29089 | NM_014176 | 3 | -10.70 |
| SEMG2 | 6407 | NM_003008 | 3 | -10.59 |
| HGD | 3081 | NM_000187 | 3 | -10.44 |
| HIRIP3 | 8479 | NM_003609 | 4 | -10.35 |
| EBP | 10682 | NM_006579 | 3 | -10.32 |
| DEFB4 | 1673 | NM_004942 | 3 | -10.25 |
| KHSRP | 8570 | NM_003685 | 3 | -10.19 |
| ADAR | 103 | NM_001111 | 3 | -9.46 |
| ACHE | 43 | NM_000665 | 3 | -9.41 |
| PRSS21 | 10942 | NM_006799 | 3 | -9.37 |
| FLOT2 | 2319 | NM_004475 | 4 | -9.25 |
| LOC390533 | 390533 | Discontinued | 3 | -9.25 |
| BMP7 | 655 | NM_001719 | 4 | -9.13 |
| CLIC1 | 1192 | NM_001288 | 4 | -9.00 |
| CD151 | 977 | NM_004357 | 3 | -8.96 |
| OSTCL / LOC202459 | 202459 | NM_145303 | 3 | -8.85 |
| IAPP | 3375 | NM_000415 | 4 | -8.82 |
| UBE2L3 | 7332 | NM_003347 | 3 | -8.70 |
| CD34 | 947 | NM_001773 | 3 | -8.48 |
| RAB3C | 115827 | NM_138453 | 3 | -8.24 |

(continued)

| Transcripts whose depletion with at least three out of four non-overlapping siRNAs yielded significant and consistent chemosensitization or cytoprotection against cisplatin (CDDP)-induced cell death in A549 cells. Gene ID, accession number, score and Δ are reported. | | | | |
|---|---|---|---|---|
| **Name** | **Gene ID** | **Accession number** | **Score\*** | **Δ\*\*** |
| NLRP1 | 22861 | NM_014922 | 3 | -8.13 |
| C1ORF91 | 56063 | NM_019118 | 3 | -7.97 |
| TCTE3 | 6991 | NM_174910 | 3 | -7.73 |
| KIA1161 | 57462 | NM_020702 | 3 | -7.64 |
| NCKAP1 | 10787 | NM_013436 | 3 | -7.63 |
| TMED1 | 11018 | NM_006858 | 3 | -7.51 |
| BAIAP3 | 8938 | NM_003933 | 3 | -7.47 |
| BSN | 8927 | NM_003458 | 3 | -7.40 |
| PPM1B | 5495 | NM_002706 | 3 | -6.98 |
| GUCA1A | 2978 | NM_000409 | 3 | -6.71 |
| SEMA3C | 10512 | NM_006379 | 3 | -6.17 |
| EEF2 | 1938 | NM_001961 | 3 | +6.32 |
| RAB40A | 142684 | NM_080879 | 3 | +6.47 |
| ALDH7A1 | 501 | NM_001182 | 3 | +6.80 |
| PDXK | 8566 | M_003681 (SEQ ID NO :3) | 3 | +7.06 |
| LOC440611 | 440611 | Discontinued | 3 | +8.10 |
| HDLBP | 3069 | NM_005336 | 3 | +8.15 |
| LOC442126 | 442126 | Discontinued | 3 | +8.57 |
| ASTL | 431705 | NM_001002036 | 3 | +8.64 |
| SLC22A18AS | 5003 | NM_007105 | 3 | +8.69 |
| C21ORF2 | 755 | NM_004928 | 3 | +8.74 |
| RPSAP32 / LOC402123 | 402123 | Pseudogene | 3 | +9.13 |
| FN3KRP | 79672 | NM_024619 | 3 | +9.76 |
| DHRSX | 207063 | NM_145177 | 3 | +9.96 |
| LOC441115 | 441115 | Discontinued | 3 | +10.31 |
| COX5B | 1329 | NM_001862 | 3 | +10.61 |
| COX7C | 1350 | NM_001867 | 4 | +10.85 |
| LOC440056 | 440056 | Discontinued | 3 | +11.88 |
| MPP6 | 51678 | NM_016447 | 3 | +10.89 |
| SLC39A5 | 283375 | NM_173596 | 3 | +10.96 |
| GRLF1 | 2909 | NM_004491 | 3 | +11.62 |
| ALK | 238 | NM_004304 | 3 | +12.03 |
| COMMD9 | 29099 | NM_014186 | 3 | +12.60 |

(continued)

| Name | Gene ID | Accession number | Score* | Δ** |
|---|---|---|---|---|
| Transcripts whose depletion with at least three out of four non-overlapping siRNAs yielded significant and consistent chemosensitization or cytoprotection against cisplatin (CDDP)-induced cell death in A549 cells. Gene ID, accession number, score and Δ are reported. | | | | |
| APAF1 | 317 | NM_001160 | 4 | +12.66 |
| ZNF878 / LOC342972 | 729747 | NM_001080404 | 3 | +13.13 |
| FBX042 | 54455 | NM_018994 | 3 | +13.47 |
| LOC388882 | 388882 | XR_042117 | 3 | +13.52 |
| RNF26 | 79102 | NM_032015 | 3 | +14.55 |
| TRIP4 | 9325 | NM_016213 | 3 | +14.89 |
| PSMD7 | 5713 | NM_002811 | 3 | +16.00 |
| OVCH1 | 341350 | NM_183378 | 3 | +16.24 |
| PSMC5 | 5705 | NM_002805 | 3 | +16.29 |
| SOCS6 | 9306 | NM_004232 | 3 | +17.10 |

* = n° of siRNAs (out of 4 tested) that significantly modulated cisplatin (CDDP)-induced cell death ** = upon background subtraction and inter-plate normalization, Δ = relative survival of siRNA-transfected cells (WST-1 signal from CDDP treated, siRNA-transfected cells / WST-1 signal from untreated, siRNA-transfected cells) - relative survival of cells transfected with a control siRNA (siUNR) (WST-1 signal from CDDP treated, siUNR-transfected cells / WST-1 signal from untreated, siUNR-transfected cells). Negative and positive Δ values signify chemosensitization and cytoprotection, respectively.

Preferably, the genes, in particular the at least one gene, for example two or three genes, the absence, presence or expression level of which are advantageously to be determined in a biological sample of the subject having a tumor can be selected from the group consisting of ACHE, ADAR, ALDH7A1, ALK, APAF1, ASTL, BAIAP3, BCL2L1, BMP7, BSN, BTNL2, C10ORF92, C1ORF91, C21ORF2, CD151, CD34, CLIC1, COMMD9, COX5B, COX7C, CYP11B2, DEFB4, DHRSX, EBP, EEF2, FBX042, FLOT2, FN3KRP, GRLF1, GUCA1A, HDLBP, HGD, HIRIP3, IAPP, IL6R, ITGB6, KHSRP, KIA1161, LIPC (sequence NM_000236 = SEQ ID NO:1 and corresponding 499 amino acids sequence = SEQ ID NO: 2), LOC388882, LOC389727, LOC390533, LOC440056, LOC440611, LOC441115, LOC442126, LRMP, MAT2A, MCL1, MPP6, NANOS1, NCKAP1, NCOA3, NLRP1, OSTCL, OVCH1, PDXK (sequence NM_003681 = SEQ ID NO :3 and corresponding 312 amino acids sequence = SEQ ID NO:4), PLD3, PLK1S1, PPM1B, PPM1J, PRSS21, PSMC5, PSMD7, RAB3C, RAB40A, RAC2, RNF26, RPSAP32, RRAD, SEMA3C, SEMG2, SLC22A18AS, SLC39A5, SOCS6, TCTE3, TFF1, TMED1, TRIP4, UBE2L3, UBE2T, USP8, WSB2, ZDHHC9, ZNF878, more preferably from the group consisting of ACHE, ADAR, ALDH7A1, ALK, APAF1, ASTL, BAIAP3, BCL2L1, BMP7, BSN, BTNL2, CD151, CD34, CLIC1, COMMD9, COX5B, COX7C, CYP11B2, DEFB4, DHRSX, EBP, EEF2, FBX042, FLOT2, FN3KRP, GREF1, GUCA1A, HDLBP, HGD, HIRIP3, IAPP, IL6R, ITGB6, KHSRP, LIPC, LRMP, MAT2A, MCL1, MPP6, NANOS1, NCKAP1, NCOA3, NLRP1, OSTCL, OVCH1, PDXK, PLD3, PLK1S1, PPM1B, PPM1J, PRSS21, PSMC5, PSMD7, RAB3C, RAB40A, RAC2, RNF26, RPSAP32, RRAD, SEMA3C, SEMG2, SLC22A18AS, SLC39A5, SOCS6, TCTE3, TFF1, TMED1, TRIP4, UBE2L3, UBE2T, USP8, WSB2, ZDHHC9 and ZNF878; and even more preferably from the group consisting of ACHE, ADAR, ALDH7A1, ALK, ASTL, BAIAP3, BMP7, BSN, BTNL2, CD151, CD34, CLIC1, COMMD9, COX5B, COX7C, CYP11B2, DEFB4, DHRSX, EBP, EEF2, FBX042, FLOT2, FN3KRP, GRLF1, GUCA1A, HDLBP, HGD, HIRIP3, IAPP, IL6R, ITGB6, KHSRP, LIPC, LRMP, MAT2A, MPP6, NANOS1, NCKAP1, NCOA3, NLRP1, OSTCL, OVCH1, PDXK, PLD3, PLK1S1, PPM1B, PPM1J, PRSS21, PSMC5, PSMD7, RAB3C, RAB40A, RAC2, RNF26, RPSAP32, RRAD, SEMA3C, SEMG2, SLC22A18AS, SLC39A5, SOCS6, TCTE3, TMLD1, TRIP4, UBE2L3, UBE2T, USP8, WSB2, ZDHHC9, and ZNF878.

Optionally, the method comprises determining the absence, presence or expression level of at least 1, 2, 3, 4, 5, 6, 7, 8, 9, 10, 50, 60, 65, 70, 74, 75, 80 or 85 genes from those identified in Table 1.

When several genes are studied in the method, said genes can be selected in such a way that they comprise at least one, preferably several, for example at least 1, 2, 3, 4, 5, 6, 7, 8, 9, 10, 50, 60, 65, 70, 74, 75, 80 or 85 over-expressed genes, and at least one, preferably several, for example at least 1, 2, 3, 4, 5, 6, 7, 8, 9, 10, 50, 60, 65, 70, 74, 75, 80 or 85, under-expressed ones. Alternatively, they can be selected in such a way that they comprise only over-expressed or under-expressed genes.

In particular, the non expression or under-expression of a gene selected from ALDH7A1, ALK, APAF1, ASTL, C21ORF2, COMMD9, COX5B, COX7C, DHRSX, EEF2, FBX042, FN3KRP, GRLF1, HDLBP, LOC388882, LOC440056, LOC440611, LOC441115, LOC442126, MPP6, OVCH1, PDXK, PSMC5, PSMD7, RAB40A, RNF26, RPSAP32, SLC22A18AS, SLC39A5, SOCS6, TRIP4, and ZNF878 and/or the over-expression of a gene selected from ACHE, ADAR, BAIAP3, BCL2L1, BMP7, BSN, BTNL2, C10ORF92, C1ORF91, CD151, CD34, CLIC1, CYP11B2, DEFB4, EBP, FLOT2, GUCA1A, HGD, HIRIP3, IAPP, IL6R, ITGB6, KHSRP, KIA1161, LIPC, LOC389727, LOC390533, LRMP, MAT2A, MCL1, NANOS1, NCKAP1, NCOA3, NLRP1, OSTCL, PLD3, PLK1S1, PPM1B, PPM1J, PRSS21, RAB3C, RAC2, RRAD, SEMA3C, SEMG2, TCTE3, TFF1, TMED1, UBE2L3, UBE2T, USP8, WSB2, and ZDHHC9 are indicative of a resistance to a cancer treatment, in particular of a resistance to a chemotherapy, more particularly of a resistance to a chemotherapy using a drug selected from an alkylating agent such as camptothecin, cyclophosphamide, mechlorethamine, uramustine, melphalan, chlorambucil, ifosfamide, carmustine, lomostine, streoptozocin, busulfan, thiotepa, dacarbazine, mitozolomide, temozolomide, procarbazine, altretamine, dacarbazine, or an alkylating-like agent, in particular a platin (or platinum-based agent) selected from cis-platinum (cisplatin or CDDP), carboplatin, nedaplatin, satraplatin and oxali-platinum (oxaliplatin or OXP), most preferably a platinum-based agent, in particular CDDP.

In a particular embodiment of the present invention, a preferred example of the at least one gene the absence, presence or expression level of which is advantageously to be determined in a biological sample of the subject having a tumor, is the hepatic lipase (LIPC) gene.

The presence of LIPC in a biological sample, preferably in a tumor sample of the subject, is indicative of a resistance of the subject to a conventional cancer treatment, and, in the specific context of a chemotherapy, of a deleterious effect thereof on the subject undergoing such a cancer treatment.

The absence of LIPC in a biological sample, preferably in a tumor sample of the subject, is the indication that a chemotherapy will be efficient in the subject.

In a particular aspect, the method previously described of assessing or monitoring the sensitivity of a subject having a tumor to a particular treatment of cancer, advantageously further comprises a step of comparing the expression level of the at least one gene in the biological sample of the subject to a reference expression level, for instance the expression level of the gene in a reference cell, typically a cancer cell, preferably a cell sensitive to the particular treatment of cancer. Alternatively, reference or control expression levels are determined with a sample of patients or subjects sensitive to the particular treatment.

An over-expressed gene thus herein refers to a gene having an increased expression in comparison to the expression level of this gene in a sensitive cell, and an under-expressed gene herein refers to a gene having a decreased expression in comparison to the expression level of this gene in a sensitive cell. However, the man skilled in art understands that other references can be used. For instance, the invention also contemplates a reference level corresponding to the expression level in a cell resistant to the particular treatment of cancer.

In the context of the herein described methods, the presence, absence, normal/functional/correct expression, abnormal/non functional expression, and/or expression level of a gene can typically be determined (detected and/or measured) by the presence, quantity and/or analysis of the functional expression of protein or mRNA encoded by said genes. Generally, the expression level as determined is a relative expression level.

The expression level of the selected gene(s) can be determined by measuring the amounts of RNA, in particular mRNA, DNA, in particular cDNA, or protein using a variety of techniques well-known by the man skilled in art.

In a particular embodiment, the under-expression of a gene can be indirectly assessed through the determination of the methylation status of its promoter. Indeed, a methylated promoter is indicative of an expression repression, and therefore of an under-expression. On the contrary, an unmethylated promoter is indicative of a normal expression. The methylation state of a promoter can be assessed by any method known by the one skilled in the art, for instance by the methods disclosed in the following documents: Frommer et al (Proc Natl Acad Sci U S A. 1992;89:1827-31) and Boyd et al (Anal Biochem. 2004; 326:278-80).

More preferably, the determination comprises contacting the sample with selective reagents such as probes, primers or ligands, and thereby detecting the presence, or measuring the amount, of proteins or nucleic acids of interest originally in the sample. Contacting may be performed in any suitable device, such as a plate, microtiter dish, test tube, well, glass, column, and so forth. In specific embodiments, the contacting is performed on a substrate coated with the reagent, such as a nucleic acid array or chip or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or proteins of the sample.

In a preferred embodiment, the expression level may be determined by determining the quantity of mRNA.

Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate). For instance, the probes and primers can be selected from the Taqman Applied ones cited in the present application.

The nucleic acid primers or probes used herein may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another preferred embodiment, the expression level is determined by DNA chip analysis. Such DNA chip or nucleic acid micro array consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the micro array in hybridization conditions, leading to the formation of complexes between target nucleic acids that are complementary to probe sequences attached to the micro array surface. The labelled hybridized complexes are then detected and can be quantified or semi-quantified. Labelling may be achieved by various methods, *e.g.* by using radioactive or fluorescent labelling. Many variants of the micro array hybridization technology are available to the man skilled in the art (see e.g. the review by Hoheisel, et 2006).

Other methods for determining the expression level of said genes include the determination of the quantity of proteins encoded by said genes.

Such methods comprise contacting a biological sample with a binding partner capable of selectively interacting with a marker protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The antibody (Ab) the presence of which is to be determined according to the previously described method may be selected from:

- aldehyde dehydrogenase family 7, member A1 (ALDH7A1) antibody (goat antiserum #SC-79398, Santa Cruz Biotechnology Inc., Santa Cruz, USA),
- apoptotic peptidase activating factor 1 (APAF1) antibody (mouse monoclonal $IgG_2$ #MAB868, R&D Systems),
- BCL-$X_L$ (BCL2L1) antibody (rabbit antiserum #2764; Cell Signaling Technology Inc., Danvers, USA),
- caspase-3 (CASP3) antibody (rabbit monoclonal IgG #9665; Cell Signaling Technology Inc.),
- caspase-9 (CASP9) antibody (rabbit antiserum #9502; Cell Signaling Technology Inc.),
- cysteine conjugate-β lyase (CCBL1) antibody (mouse monoclonal $IgG_2$a #AB76963, Abcam plc., Cambridge, UK),

- cytochrome c oxidase subunit Vb (COX5B) antibody (mouse antiserum #AB88440, Abcam plc.),
- cytochrome c oxidase subunit VIIc (COX7C) antibody (goat antiserum #SC-55711, Santa Cruz Biotechnology Inc.),
- hepatic lipase (LIPC) antibody (rabbit antiserum #SC-21007, Santa Cruz Biotechnology Inc.),
- interleukin 6 receptor (IL6R) antibody (goat antiserum #AB-227-A, R&D Systems),
- pyridoxal kinase (PDXK) antibody (rabbit antiserum #AB38208, Abcam plc.),
- pyridoxal phosphatase (PDXP) antibody (rabbit monoclonal IgG #4686, Cell Signaling Technology Inc.),
- phospho-TP53 (Ser15) antibody (rabbit antiserum #9284; Cell Signaling Technology Inc.),
- phospho-TP53 (Ser46) antibody (rabbit antiserum #2521; Cell Signaling Technology Inc.),
- TP53 antibody (rabbit antiserum #9282; Cell Signaling Technology Inc.), and
- ZDHHC9 antibody (rabbit antiserum #AB74504, Abcam plc.).

The aforementioned assays generally involve separation of unbound protein in a liquid phase from a solid phase support to which antigen-antibody complexes are bound. Solid supports which can be used in the practice of the invention include substrates such as nitrocellulose (*e. g.*, in membrane or microtiter well form); polyvinylchloride (*e. g.,* sheets or microtiter wells); polystyrene latex *(e.g.,* beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

The expression of a particular gene (from the genes listed in Table 1) is correct if the expressed protein is active or functional, i.e., in the context of the present invention, if a gene selected from ALDH7A1, ALK, APAF1, ASTL, C21ORF2, COMMD9, COX5B, COX7C, DHRSX, EEF2, FBX042, FN3KRP, GRLF1, HDLBP, LOC388882, LOC440056, LOC440611, LOC441115, LOC442126, MPP6, OVCH1, PDXK, PSMC5, PSMD7, RAB40A, RNF26, RPSAP32, SLC22A18AS, SLC39A5, SOCS6, TRIP4, and ZNF878 is able to directly or indirectly induce or enhance the action of the therapeutic treatment of a cancer in a subject or if a gene selected from ACHE, ADAR, BAIAP3, BCL2L1, BMP7, BSN, BTNL2, C10ORF92, C1ORF91, CD151, CD34, CLIC1, CYP11B2, DEFB4, EBP, FLOT2, GUCA1A, HGD, HIRIP3, IAPP, IL6R, ITGB6, KHSRP, KIA1161, LIPC, LOC389727, LOC390533, LRMP, MAT2A, MCL1, NANOS1, NCKAP1, NCOA3, NLRP1, OSTCL, PLD3, PLK1S1, PPM1B, PPM1J, PRSS21, RAB3C, RAC2, RRAD, SEMA3C, SEMG2, TCTE3, TFF1, TMED1, UBE2L3, UBE2T, USP8, WSB2, and ZDHHC9 is able to directly or indirectly inhibit or reduce the action of the therapeutic treatment of a cancer in a subject.

An *in vitro* or *ex vivo* method of assessing the prognosis of a cancer in a subject is further herein disclosed. This method comprises a step of determining, in a biological sample of the subject, the presence, absence or expression level of a compound selected from vitamin B6, pyridoxal (PL), pyridoxal-5-phosphate (PLP), pyridoxamine (PM), pyridoxamine-5'-phosphate, pyridoxine (PN), pyridoxine-5'-phosphate, L-2-aminoadipate and L-2-aminoadipate 6-semialdehyde, and/or of the expression product (as previously defined) of at least one gene selected from the genes identified in Table I, or a derivative product thereof, thereby assessing the prognosis of the cancer in the subject.

In a particular embodiment of the present invention, a preferred example of the at least one gene the absence, presence or expression level of which is advantageously to be determined in a biological sample of the subject having a tumor, is selected from the hepatic lipase (LIPC) gene and the pyridoxal kinase (PDXK) gene.

The presence of LIPC in a biological sample of a subject, preferably in a tumor sample of the subject, is indicative of a favourable prognosis of the cancer in the subject. On the contrary, the absence of LIPC in a biological sample of a subject, is indicative of an unfavourable prognosis of the cancer in the subject.

An over expression of PDXK in a biological sample of the subject, preferably in a tumor sample of the subject, when compared, as explained previously, to a reference expression level is indicative of a favourable prognosis of the cancer in the subject.

The non expression of PDXK in a biological sample of the subject, preferably in a tumor sample of the subject, or a reduced expression thereof when compared to a reference expression level is indicative of an unfavourable prognosis of the cancer in the subject

The method previously described of assessing the prognosis of a cancer in a subject thus advantageously further comprises a step of comparing, as explained previously, the expression level of the at least one gene in the biological sample of the subject to a reference expression level.

The presence, in at least one of the herein identified genes (see Table 1), of an alteration leading to its abnormal expression, may determine the inability of the subject to positively respond to a cancer treatment and/or may be used to assess the prognosis of a cancer in a subject. The alteration may be in a gene locus. The alteration may be a single nucleotide polymorphism (SNP).

The method of assessing or monitoring the sensitivity of a subject having a tumor to a treatment of cancer or of assessing the prognosis of a cancer in a subject, may also consist in detecting the presence of an altered nucleic acid in a biological sample from the subject (as defined previously), the presence of said altered nucleic acid, being indicative of the inability for the subject to respond to a treatment of a cancer.

This detection step may be performed before or after the administration to the subject having the tumor of at least part of the treatment of cancer, typically of at least part of a conventional treatment of cancer as previously explained.

A particular method herein described may comprise the following steps of (a) obtaining from the subject a test sample of tumoral DNA, cDNA or RNA, (b) contacting the test sample with at least one nucleic acid probe, wherein said nucleic acid is complementary to and specifically hybridises with a targeted altered nucleic acid sequence (one of the herein identified sequence) preferably comprising at least one point mutation, in particular a single nucleotide polymorphism (SNP), to form a hybridization sample, (c) maintaining the hybridization sample under conditions sufficient for the specific hybridization of the targeted nucleic acid sequence with the nucleic acid probe to occur, and (d) detecting whether there is specific hybridization of the altered targeted nucleic acid sequence with the nucleic acid probe.

Further herein described is a DNA chip comprising a solid support which carries nucleic acids that are specific to at least one, preferably at least 2, 3, 4, 5, 6, 7, 8, 9, 10, 20, 25, 50, 60, 65, 70, 74, 75, 80 or 85 genes, selected from the genes identified in Table I. The DNA chip can further comprise nucleic acid(s) for control gene, for instance a positive and negative control or a nucleic acid for an ubiquitous gene in order to normalize the results.

Also herein described is a kit for assessing or monitoring the sensitivity of a subject having a tumor to a treatment of cancer, in particular a chemotherapy, or the prognosis of a cancer in a subject, wherein the kit comprises (i) detection means selected from the group consisting of a pair of primers, a probe, at least one antibody specific to the expression product of a gene selected, in particular from the genes identified in Table I, and/or specific to a derivative product thereof, and a DNA chip as herein described, and, optionally, (ii) a leaflet providing the wild-type sequence of the gene and/or the control quantitative expression value corresponding to the expression product of the gene in a control population and/or corresponding to a derivative product thereof.

The kit can further comprise control reagents and other necessary reagents.

The present invention also relates to the use of a DNA chip or a kit of the invention for preparing a kit for (i) predictively determining if a subject having a tumor, will respond to a treatment of cancer, in particular to a conventional treatment of cancer, or will be resistant to said treatment, and/or for (ii) assessing the prognosis of a cancer in a subject having a tumor.

If the subject is identified, using a method according to the present invention, as resistant to a particular treatment of cancer (for example because this subject does not express or abnormally expresses the previously mentioned genes), the method advantageously further comprises a step of selecting a "compensatory molecule", to be used, alone or in combination with a conventional treatment of cancer as herein defined, in particular with the particular treatment of cancer mentioned previously, as the appropriate therapeutic treatment of cancer for the subject.

A method of selecting an appropriate, preferably optimal, therapeutic treatment of cancer for a subject having a tumor, is therefore in addition herein described, as well as compensatory molecules for use in such a treatment of cancer, preferably in combination with a conventional treatment of cancer, in particular a chemotherapeutic treatment of cancer, in a subject identified, using a method as herein described, as resistant to the conventional treatment of cancer.

In a particular embodiment, the method of selecting an appropriate treatment of cancer for a subject having a tumor, comprises a step of determining the presence, absence or expression level of the hepatic lipase (LIPC) in a biological sample of said subject, the absence of LIPC in the biological sample being the indication that a chemotherapy will be efficient in the subject, the presence of LIPC, or an over expression thereof when compared to a reference expression level, in the biological sample being, on the contrary, the indication that a chemotherapy, in particular a chemotherapy using cis-platinum (cisplatin or CDDP), more particularly as sole treatment, will be detrimental to the subject.

In another particular embodiment, the method comprises a step of determining the presence or absence of LIPC in a biological sample of said subject, the presence of LIPC in the biological sample, or an over expression thereof when compared to a reference expression level, being the indication that another molecule selected from in particular: an inhibitor of lipase such as orlistat®; an antagonist of IL-6R, such as an IL6- or an IL6R-neutralizing antibody, and/or a DNA damaging chemotherapeutic agent selected from camptothecin, cyclophosphamide, mechlorethamine, uramustine, melphalan, chlorambucil, ifosfamide, carmustine, lomostine, streoptozocin, busulfan, thiotepa, dacarbazine, mitozolomide, temozolomide, procarbazine, altretamine, dacarbazine, or an alkylating-like agent, in particular a platinum-based agent selected from cis-platinum (cisplatin or CDDP), carboplatin, nedaplatin, satraplatin and oxali-platinum (oxaliplatin or OXP), most preferably a platinum-based agent, in particular CDDP, should preferably be administered to the patient together with a conventional treatment of cancer such as a chemotherapy, in particular a chemotherapy using cis-platinum.

In a further particular embodiment, the method comprises a step of determining the expression level of a compound selected from PDXK, PDXP and aldehyde dehydrogenase 7 family, member A1 (ALDH7A1) in a biological sample of said subject, the non expression thereof or a reduced expression thereof, when compared to a reference expression level, being the indication that a compound selected from pyridoxine (PN, vitamin B6), pyridoxal (PL), pyridoxal-5-phosphate (PLP), pyridoxamine (PM), pyridoxamine-5'-phosphate, pyridoxine (PN), and pyridoxine-5'-phosphate (PNP), should preferably be administered to the patient together with a conventional treatment of cancer such as a chemotherapy, in particular a chemotherapy using cis-platinum, typically when the cancer is selected from a NSCLC, a cervical cancer, a carcinoma, a osteosarcoma and a wild-type colorectal cancer.

Further herein disclosed is the use of a compensatory molecule as herein described for improving the treatment of a cancer, preferably for treating the cancer, or to prepare a pharmaceutical composition for improving the treatment of a cancer, preferably for treating the cancer in a subject tested and identified, by a method as herein described, as resistant to a treatment of cancer, typically to a conventional treatment of cancer. Preferably, the pharmaceutical composition further comprises, as a combined preparation, a drug used in a conventional treatment of cancer, for simultaneous, separate or sequential use in the treatment of said cancer.

Herein described is, in addition, a method for preventing or treating a cancer comprising the administration to a subject in need thereof, as previously explained, of a compensatory molecule or compound, preferably together with (in combination with) a distinct therapeutic agent, typically an agent or a drug used in a conventional treatment of cancer (as a combined preparation), the compound and distinct therapeutic agent being simultaneously or separately administered in the context of a unique cancer protocol.

In a particular embodiment of the present invention, the previously described method for treating cancer is performed on a subject having a tumor before surgical resection of at least part thereof.
In another particular embodiment of the present invention, the previously described method for treating cancer is performed on a subject having a tumor after surgical resection of at least part thereof (adjuvant chemotherapy).

Herein disclosed is also a method for screening or identifying a compound suitable for improving the treatment of a cancer (also herein identified as "compensatory molecule") in a subject having a tumor, said method comprising determining the ability of a test compound (i) to modify the expression in particular of at least one gene selected from the genes identified in Table I, or compensate an abnormal or altered expression thereof, or (ii) to improve the treatment of a cancer with a conventional treatment as described previously, or to reduce the development of a resistance during such a conventional treatment of a cancer.

In a particular aspect, the method comprises: 1) providing a cell or cell-line with at least one gene over-expressed and/or under-expressed selected from the group of genes of Table 1; 2) contacting said cell or cell-line with a test compound; 3) determining the expression level of said at least one gene; and, 4) selecting the compound which inhibits the expression or decreases the expression level of the at least one over-expressed gene or increases the expression level of the at least one under-expressed gene.

In another particular aspect, the method comprises: 1) providing a cell or cell-line sensitive to a particular alkylating agent or alkylating-like agent, in particular a platin (for example CDDP), as herein identified; 2) contacting said cell or cell-line with a test compound and the particular alkylating agent or alkylating-like agent; 3) determining the expression level of said at least one gene selected from the genes listed in Table 1, and, 4) selecting the compound which inhibits the appearance of an over-expression and/or the appearance of an inhibition or under-expression of the at least one gene selected from the genes listed in Table 1.

In a further particular aspect, the method comprises a step of detecting and/or measuring the level of expression, in a cell or cell-line, of at least one gene selected from the genes identified in Table I in the presence of a test compound, wherein a modified expression in comparison with the expression obtained in a control cell or cell-line (the same cell or cell-line which has not been exposed to or contacted with the test compound), is indicative of the capacity of said test compound to modify the expression of said at least one gene in said cell or cell-line.

Also herein described is a method for screening a compound usable for treating a cancer, as a compensatory molecule or compound according to the present invention, in a subject having an altered nucleic acid, an altered nucleic acid expression, or an abnormal expression or activity of the protein encoded by said nucleic acid, said method comprising determining *in vitro, in vivo* or *ex vivo* the ability of a test compound to (i) restore a functional expression of said altered or abnormal protein (ii) modulate (i.e., induce, increase, or decrease) the expression or activity of said protein, or (iii) modulate the expression or activity of a ligand of said protein.

The compounds identified with one of the herein described screening methods may be used, in the context of the present invention, as compensatory molecules.

Other characteristics and advantages of the invention are given in the following experimental section (with reference to figures 1 to 13), which should be regarded as illustrative and not limiting the scope of the present application.

## EXPERIMENTAL PART

**Example 1: identification of chemotherapeutic agent response modifiers reveals new prognostic and predictive biomarkers in cancer.**

Personalized cancer therapy relies on biomarkers that predict the evolution and therapeutic response of individual tumors. Most on-small cell lung cancers (NSCLC) are treated with platinum-based agents such as cisplatin, yielding highly heterogeneous therapeutic responses. Here, inventors report a genome-wide siRNA-based screening that led to the identification of gene transcripts whose abundance affects the response of NSCLC cells to cisplatin. Functional and pharmacological validation experiments coupled to epistatic analyses led to the identification of two metabolic pathways affecting cisplatin responses *in vitro* and *in vivo.* One pathway involves pyridoxal kinase (PDXK), which generates the bioactive form of vitamin B6 and is required for optimal cisplatin responses. The other pathway involves hepatic lipase (LIPC), whose depletion or pharmacological inhibition has chemosensitizing effects. The expression levels of PDXK and LIPC had a prognostic impact on patients with NSCLC. Moreover, low LIPC expression levels were predictive for a positive effect of cisplatin-based adjuvant chemotherapy.

## MATERIALS AND METHODS

Unless otherwise specified, all experiments were performed in triplicate and repeated at least twice. Data are reported as means $\pm$ SEM.

### *Chemicals and cell cultures.*

Unless otherwise specified, chemicals were purchased from Sigma-Aldrich (St. Louis, USA), culture media and supplements for cell culture from Gibco-Invitrogen (Carlsbad, USA) and plasticware from Corning (Corning, USA). Anti-human interleukin 6 (IL6, goat antiserum #AB-206-A) and IL6 receptor (IL6R, goat antiserum #AB-227-A) neutralizing antibodies were purchased by R&D Systems (Minneapolis, USA). Orlistat (Xenical®) was obtained from Roche (Basel, Switzerland). The content of each capsule (120 mg) was resuspended in 1 mL 33% (v/v) ethanol in PBS for 30 min and carefully vortexed every 10 min. Undissolved material was removed by two sequential rounds of centrifugation at 12,000 G (5 min, room temperature, RT), supernatants were collected and stored at -80˚c under protection from light until use. In low-throughput experiments, all cell lines were treated with 2.5 mM pyridoxine (PN), exception made for A549 cells that received 5 mM PN.

Human wild type (WT) non-small cell lung cancer (NSCLC) A549 cells and six distinct cisplatin (CDDP)-resistant derivatives (kindly provided by Dr. Catherine Brenner Jan, Université Paris 11, Chatenay Malabry, France) were routinely maintained in Glutamax®-containing DMEM/F12 medium supplemented with 10% fetal bovine serum (FBS), 10 mM HEPES buffer, 100 units/mL penicillin G sodium and 100 μg/mL streptomycin sulfate. Human NSCLC HCC827, H1299, H1650 and H1975 cells were cultured in Glutamax®-containing RPMI medium, supplemented as above. Human cervical carcinoma HeLa and osteosarcoma U20S cells as well as murine Lewis lung carcinoma (LLC) cells were grown in endotoxin-free Glutamax®-containing DMEM, supplemented as above. Human WT and *TP53*^-/- colon carcinoma cells were maintained in Glutamax® -containing McCoy's 5A supplemented with 10% FBS, 10 mM HEPES buffer, 1mM sodium pyruvate, 100 units/mL penicillin G sodium and 100 μg/mL streptomycin sulfate. Cell lines were routinely maintained at +37 ˚C under 5% $CO_2$ in T175 flasks, and seeded into 10 cm Petri dishes or 6-, 12-, 24- or 96-wells plates 12-24 h before the experiments.

### *Transfections.*

The genome-wide siRNA library Human Whole Genome siRNA Set Version 1.0 (Qiagen, Hilden, Germany) was used in this study. The sequences of the siRNAs selected for low-throughput validation analyses, as well as those of other siRNAs that were employed in this study are reported in Table 2. To avoid depleting the siRNA library of selected sequences, siRNAs for validation experiments were purchased as custom molecules from Sigma-Aldrich. For low-throughput experiments, (see below for the transfection protocols employed in the siRNA screen), siRNA were transfected in A549 cells with Oligofectamine™ reagent (Invitrogen), following the manufacturer's instructions. Transfection with a non-targeting siRNA (siUNR) was employed as a negative control condition. Forty-eight hours after transfection, siRNA-mediated target downregulation was assessed by immunoblotting (see below).

*Genome-wide siRNA screens.*

High-throughput operations were carried out by means of a Microlab STAR automated workstation (Hamilton, Reno, USA) placed under a class II safety cabinet. In all screening rounds, each siRNA was tested in triplicates that were distributed to three different plates. Prior to cell seeding, transfection complexes were individually prepared in a V-bottomed 96-well deep well plate. In brief, each siRNA (as retrieved from the Human Whole Genome siRNA Set Version 1.0) was dissolved in a total volume of 122.5 μL serum-free phenol-red free DMEM/F12 medium (final concentration = 10 nM, conditions for 7 wells). Similarly, an appropriate amount of HiPerFect® transfection reagent (Qiagen) was diluted in serum-free phenol-red free DMEM/F12 medium (0.25 μL per well). These solutions were allowed to stand at RT for 5-10 min and then 122.5 μL of diluted HiPerFect® were added to each siRNA solution, mixed by automated pipetting and left for another 10-15 min at RT to facilitate complex formation. Meanwhile, A549 cells from maintenance cultures were collected and resuspended in phenol-red free DMEM/F12 medium supplemented with 10% fetal bovine serum (FBS), 10 mM HEPES buffer, 100 units/mL penicillin G sodium and 100 μg/mL streptomycin sulfate at a final concentration of 45,000 cells/mL. Thereafter, 455 μL of such suspension were added on top of the transfection complexes for each siRNA, mixed by automated pipetting and seeded in 100 μL aliquots, in 6 distinct flat-bottomed 96-well plates. In each plate set (6 plates), the following control conditions were included: empty wells (for background subtraction), untransfected cells and untransfected cells destined to treatment (for the control of cell death induction), UNR-transfected cells and UNR-transfected cells destined to treatment (negative control for transfection), untransfected cells destined to lysis (positive control for the assessment of plasma membrane breakdown), cells transfected with a BAK1-targeting siRNA and cells transfected with a BAK1-targeting siRNA destined to treatment (positive control of siRNA-mediated cytoprotection), cells transfected with a EG5-targeting siRNA (positive control of transfection efficiency). Forty-eight hours later, 10 μL of 0.5 mM CDDP (in phenol red-free culture medium) were administered to 3 out of 6 plates (final concentration = 50 μM). After additional 24 h, the 3 plates that were not subjected to cell death induction were assessed for both plasma membrane breakdown and residual cell proliferation, while the 3 plates that were treated with cell death-inducing stimuli were monitored for residual proliferation only (see next section).

*Colorimetric assessment of plasma membrane breakdown and residual proliferation.* Plasma membrane breakdown was assessed by quantifying the release of the intracellular enzyme lactate dehydrogenase (LDH) in culture supernatants by means of the Cytotoxicity Detection Kit (Roche), according to the manufacturer's instructions. A positive control for plasma membrane rupture was obtained by incubating cells with 0.1% (v/v) Triton® X-100 for 10 min before the beginning of the test.

Cell proliferation was quantified by means of a colorimetric assay based on the reduction of the colorless tetrazolium salt 4-[3-(4-iodophenyl)-2-(4-nitrophenyl)-2H-5-tetrazolio]-1,3-benzene disulfonate (WST-1, from Roche) to formazan (which exhibit an absorbance peak around 450 nm), according to the manufacturer's instructions.

*Mathematical and statistical analysis of screening results.*

Mean WST-1 and LDH signals for each experimental condition (triplicate wells in 3 distinct plates) were obtained by upon background subtraction and inter-plate normalization.

In the first round of screening (2 siRNAs x 23,078 transcripts = 43,156 siRNAs), siRNAs that *per se* exerted a significant cytotoxic (inducing an LDH release $\geq$ 15% than that provoked by Triton® X-100) or antiproliferative (reducing WST-1 conversion to $\leq$ 80% of that of siUNR-transfected cells) effect were excluded from further analysis (score = 0). The modulatory effect on CDDP-induced cell death of the remaining siRNAs was estimated by the introduction of the assay-independent indicator $\Delta$, as previously reported {de La Motte Rouge, 2007; Tajeddine, 2008}. In brief, for each siRNA, residual proliferation was calculated as the ratio between the WST-1 signal of treated, siRNA-transfected cells and that of untreated, siRNA-transfected cells. $\Delta$ was then calculated as the difference between the residual proliferation or siRNA-transfected cells and that of control, siUNR-transfected cells. Non-cytotoxic, non-antiproliferative siRNAs were assigned a score of -1 if they were associated with a significantly (Student's *t* test, $p < 0.05$) negative $\Delta$, of 0 if their $\Delta$ was non significant, and of +1 if they displayed a significantly positive $\Delta$. mRNAs were finally ranked by summing the score associated to each of the corresponding siRNAs, and 1,000 transcripts were selected based on the absolute value of $\Delta$ amongst mRNAs with a score of either - 2 or +2. The modulatory effects of these transcripts on CDDP-induced cell death were subjected to validation in a second round of screening.

In the second round of screen (4 siRNAs x 1,000 transcripts = 4,000 siRNAs), the effects of individual siRNAs were analyzed and scored as described above. Thus, mRNA scores could assume any integer figure ranging from -4 to +4. 85 mRNAs exhibiting a score with an absolute score value of either 3 or 4 (*i.e.,* -4, -3, + 3 and +4) were selected as final hits and validated in functional low-throughout assays.

*Epistatic screen.*

Every possible couple of siRNAs selected amongst the most efficient siRNAs corresponding to each of the 85 hits from the second round of screening plus 7 siRNAs targeting BAK1, BAX, BCL2, CASP2, STAT3, TP53 and VDAC1 (see also Table 2) was tested for its ability to modulate the response of A549 cells to CDDP, as described above. The sole variations in the screening protocol concerned the transfection solution (which contained 10 nM siRNA$_x$ + 10 nM siRNA$_y$ rather than 10 nM siRNA$_x$ only) and the assay outcome (WST-1 conversion only)

The following mathematical model was established for the estimation of the effects of siRNAs (alone or coupled) on CDDP-induced cell death, as compared to the negative control provided by siUNR:

$$Y_{ijk} = \mu \; x \; siRNAWell_{ij} \; x \; siRNACDDP_{jk} \; x \; \varepsilon_{ijk}$$

where $siRNA\ Well_{ij}$ = siRNA and well combined effect
$siRNACDDP_{jk}$ = siRNA and CDDP combined effect
$\varepsilon_{ijt}$ = model residues
i = 1 to 4280: siRNA alone or siRNA couple
j = well number
k = 0,1: untreated or treated plate.

For untreated plates, the CDDP effect is null, implying $siRNACDDP_{j0}$ = 1.
The effect of individual siRNA as compared to that of siUNR was then evaluated by:

$siRNACDDP_{j1} = siRNACDDP_{k1}$
j = 1 to 92: siRNA alone
k = siUNR.

To estimate the effects of siRNA couples as compared to those of the corresponding siRNAs alone, the following model was established:

$$W_{xy} = W_x + W_y + \varepsilon_{xy}$$

where $W_{xy}$ = siRNA$_x$ and siRNA$_y$ combined effect
$W_x$ = effect of siRNA$_x$ alone
$W_y$ = effect of siRNA$_y$ alone
$\varepsilon_{xy}$ = epistatic effect between siRNA$_x$ and siRNA$_y$.

In this model, $\varepsilon_{xy}$ can assume the following values:

$\varepsilon_{xy}$= 0: absence of epistasis
$\varepsilon_{xy}$ < 0: negative (alleviating) epistatic effect
$\varepsilon_{xy}$ > 0: positive (aggravating) epistatic effect.

Each siRNA couple was then subjected to a Student's *t* test for the hypothesis $\varepsilon_{xy}$ = 0, and the following distribution was obtained:

$\varepsilon_{xy}$ = 0 in 2,012 instances (out of 4,186 total siRNA interactions, *i.e.,* the number of combinations of 92 siRNAs taken 2 at a time, $^{92}C_2$)
$\varepsilon_{xy}$ < 0: in 310 instances
$\varepsilon_{xy}$ > 0: in 1,864 instances.

Epistasis could be further classified as:

masking epistasis, when $W_{xy} = W_x > W_y$
partially masking epistasis, when $W_{xy} > W_x > W_y$

complete suppression, when $W_{xy} = W_y < W_x$
partial suppression, when $W_y < W_{xy} < W_x$
coequal interaction, when $W_{xy} = W_x = W_y$.

Finally, siRNAs were clustered based on the epistatic effect ($\varepsilon$) by means of the Ward method and the Pearson's correlation, leading to the identification of 4 modules within each of which siRNAs fail to manifest any consistent degree of epistatic interaction (see also Figure 4 and Table 5).

### *Validation screens.*

Validation screens were performed with 1 siRNA x 85 targets as described above for the genome-wide screening, exception made for the amount of HiPerFect® transfection reagent per well (ranging from 0,5 to 2 $\mu$L, depending on cell type) and for the readout (WST-1 conversion only). In A549 cells, cell death induction was performed with 60 $\mu$M betulinic acid, 30 $\mu$M C$_2$-ceramide (C$_2$-CER), 150 $\mu$M carboplatin, 200 nM camptothecin, 40 $\mu$M cadmium dichloride (CdCl$_2$), 50 $\mu$M CDDP, 20 $\mu$M doxorubicin, 80 $\mu$M etoposide, 10 $\mu$M mitoxantrone, 3 $\mu$M mytomycin C, 80 $\mu$M oxaliplatin; 1.5 $\mu$M staurosporine or 8 $\mu$M thapsigargin. For cell lines other that A549, CDDP was used at the following final concentrations: 130 $\mu$M for A549 #1-3, 100 $\mu$M for A549 #4 and #5; 160 $\mu$M for A549 #6, 120 $\mu$M for HCC827, 100 $\mu$M for H1299, 80 $\mu$M for H1650, 60 $\mu$M for H1975, 40 $\mu$M for WT HCT 116 and 50 $\mu$M for $TP53^{-/-}$HCT 116 cells. $\Delta$ values were calculated as described above and subjected to unsupervised hierarchical clustering based on the Euclidean distance metric and average linkage clustering by the open-source MultiExperiment Viewer v. 4.1 software (part of the TM4 software suite) {Saeed, 2003}.

### *Transcriptome studies.*

NSCLC A549 cells were treated with 100 $\mu$M CDDP, 75 $\mu$M C$_2$-CER or 100 $\mu$M CdCl$_2$ for 12 h, or with 50 $\mu$M CDDP, C$_2$-CER or CdCl$_2$ for 24 h. Thereafter, cells were collected and lysed for RNA extraction of following established procedures {de La Motte Rouge, 2007}. mRNA expression changes were quantified on G4112A 44k Whole Human Genome Oligo Microarrays (Agilent Technologies, Santa Clara, USA), as previously reported {Castedo, 2006}. mRNA microarrays underwent standardized post-hybridization processing, image acquisition and analysis (see below) {Castedo, 2006}.
Data from microarray experiments were analyzed with the Rosetta Resolver® System (Rosetta Biosoftware, Seattle, USA). The threshold for statistical significance of probe signals (Intensity) was set at $p = 10^{-5}$. In case of replicate probes for the same transcript, average Intensity was calculated for probes exhibiting a $p$ value $< 10^{-5}$. For each mRNA, fold change (FC) was then defined as Intensity$_{sample}$/Intensity$_{control}$ when Intensity$_{sample}$ > Intensity$_{control}$, or as — (Intensity$_{control}$/Intensity$_{sample}$) when Intensity$_{sample}$ < Intensity$_{control}$. Ratios were calculated as Intensity$_{sample}$/Intensity$_{control}$.

### *Cytofluorometry.*

To measure apoptosis-related parameters, living cells were labeled for 30 min at +37 ˚C with 40 nM 3,3'dihexiloxalocarbocyanine iodide (DiOC$_6$(3), from Molecular Probes-Invitrogen, Eugene, USA), which quantifies the mitochondrial transmembrane potential ($\Delta_{\Psi m}$), plus 1 $\mu$g/mL propidium iodide (PI), which only accumulates in cells with ruptured plasma membrane {Galluzzi, 2009; Galluzzi, 2008; Galluzzi, 2007}. To assess cell cycle distribution, cells were collected, fixed by gentle vortexing in ice-cold 80% (v/v) ethanol (Carlo Erba Reagents, Milano, Italy) and stained with 50 $\mu$g/mL PI in 0.1 % (w/v) D-glucose in PBS supplemented with 1$\mu$g/mL (w/v) RNAse A {Mondragon, 2009; Mouhamad, 2007}. All cytofluorometric determinations were carried out a FACSCalibur or a FACScan cytofluorometer (BD Biosciences, San José, USA) equipped with a 70 $\mu$m nozzle.
First line statistical analysis of cytofluorometric results was performed by using the CellQuest™ software (BD Biosciences), by gating on the events characterized by normal forward scatter and side scatter parameters. Apoptosis- and cell cycle-related data were further analyzed with Microsoft Excel (Microsoft Co., Redmond, USA) and statistical significance was assessed by means of two-tailed Student's $t$ test ($p < 0.05$).

### *Immunoblotting.*

For the preparation of total protein extracts A549 cells were washed with cold PBS and lysed in a buffer containing 1% NP40, 20 mM HEPES (pH 7.9), 10 mM KCl, 1 mM EDTA, 10% glycerol, 1 mM orthovanadate, 1 mM PMSF, 1 mM dithiothreitol, 10 $\mu$g/mL aprotinin, 10 $\mu$g/mL leupeptin, and 10 $\mu$g/mL pepstatin, according to established protocols {Zermati, 2007}. Lysates were separated on pre-cast 4-12% polyacrylamide NuPAGE® Novex® Bis-Tris gels (Invitrogen), electrotransferred to polyvinylidene fluoride (PVDF) membranes (Bio-Rad, Hercules, USA) and probed with primary antibodies specific for aldehyde dehydrogenase family 7, member A1 (ALDH7A1, goat antiserum #SC-79398, Santa Cruz Biotechnology Inc., Santa Cruz, USA), apoptotic peptidase activating factor 1 (APAF1, mouse monoclonal IgG$_2$ #MAB868, R&D Systems), BCL-X$_L$ (BCL2L1, rabbit antiserum #2764; Cell Signaling Technology Inc., Danvers, USA), caspase-3 (CASP3, rabbit monoclonal IgG #9665; Cell Signaling Technology Inc.), caspase-9 (CASP9, rabbit antiserum #9502; Cell Signaling Technology Inc.), cysteine conjugate-β lyase (CCBL1, mouse monoclonal IgG$_2$a #AB76963, Abcam plc., Cambridge, UK), cytochrome c oxidase subunit Vb (COX5B, mouse antiserum #AB88440, Abcam plc.), cytochrome c oxidase subunit VIIc (COX7C, goat antiserum #SC-55711, Santa Cruz Biotechnology Inc.), hepatic lipase (LIPC, rabbit antiserum #SC-21007, Santa Cruz Biotechnology Inc.), interleukin 6 receptor (IL6R, goat antiserum #AB-227-A, R&D Systems), pyridoxal kinase (PDXK, rabbit antiserum #AB38208, Abcam plc.), pyridoxal phosphatase (PDXP, rabbit monoclonal IgG #4686, Cell Signaling Technology Inc.), phospho-TP53(Ser15) (rabbit antiserum #9284; Cell Signaling Technology Inc.), phospho- TP53(Ser46) (rabbit antiserum #2521; Cell Signaling Technology Inc.), TP53 (rabbit antiserum #9282; Cell Signaling Technology Inc.) and ZDHHC9 (rabbit antiserum #AB74504, Abcam plc.). Equal loading of lanes was checked by probing membranes with an antibody that specifically binds to glyceraldehyde 3-phosphate dehydrogenase (GAPDH, mouse monoclonal IgG$_1$ #MAB374, Millipore-Chemicon International, Temecula, USA) or to β-actin (rabbit monoclonal IgG #4970; Cell Signaling Technology Inc.). Finally membranes were incubated with suitable secondary IgG conjugated to horseradish peroxidase (Southern Biotech, Birmingham, USA), followed by chemiluminescence detection with the SuperSignal West Pico® reagent and CL-XPosure® X-ray films (both from Thermo Scientific-Pierce, Rockford, USA).

***Immunofluorescence microscopy for the detection of CDDP-GG DNA adducts.***

Immunofluorescence staining and measurement of specific DNA platination products was performed essentially as previously described {Dzagnidze, 2007; Liedert, 2006}, with minor modifications. Briefly, cells were spotted onto ImmunoSelect adhesion slides (Squarix GmbH, Marl, Germany), fixed overnight in ice-cold methanol, and subjected to proteolytic digestion with 60 μg/mL pepsin (100 μL per spot, 10 min, 37°C, in a moist chamber) and 40 μg/mL proteinase K (100 μL per spot, 10 min, 37°C, in a moist chamber). Upon blockage of unspecific binding sites with 5% (w/v) non-fat powdered milk in PBS, slides were incubated with a rat primary antibody that specifically recognizes CDDP-GG DNA adducts (RC-18) (at 37 °C for 2 h or at 4 °C overnight). Primary antibodies were revealed with anti-rat Cy3®-labeled antibodies (Molecular Probes-Invitrogen). Incubation in 1 μg/mL (w/v) 4',6-diamidino-2-phenylindole (DAPI, from Molecular Probes-Invitrogen) in PBS for 30 min at RT was employed for nuclear counterstaining. Images were acquired on an Axioplan fluorescence microscope (Carl Zeiss GmbH, Göttingen, Germany) coupled to a C4880 CCD camera (Hamamatsu Photonics, Herrsching, Germany).
For the detection of CDDP-GG DNA adducts by immunofluorescence microscopy, fluorescence signals were measured by quantitative digital image analysis with the ACAS 6.0 Cytometry Analysis System (ACAS II, Asbach, Germany). The levels of adducts in each sample were calculated as arbitrary fluorescence units (AFUs), upon normalization of integrated antibody-derived fluorescence from 200 nuclei/sample to the corresponding DNA content. Data are presented of mean AFUs $\pm$ SEM from n = 3 independent samples.

***Real-time measurements of cell adherence.***

5x10$^3$ A549 cells were seeded in standard culture conditions on 96-well plates coated with gold microelectrodes (E-plates™, Roche) and let adapt and recover growth for 40 h. Thereafter, cells were treated with 50 μM CDDP alone or in association with 5 mM PN for additional 48 h. Since plating, the adherence of cells to the plate was monitored every 15 min (as a function of plate impedance) by means of an xCELLigence™ Real-Time Cell Analyzer (RTCA, from Roche). Cell adherence was measured in 3 parallel wells for each experimental condition.
Results are reported as mean cell index (CI) values, which constitute an indication of electrode impedance, $\pm$ SEM.

***Quantification of intracellular PN.***

A549 cells were cultured in absence or the presence of 5 mM PN for 24 or 48 h, harvested, washed once in cold PBS and pelleted at 800 G for 10 min. Supernatants were discarded and pellets were dissolved in 500 μL of deionized H$_2$O by vortexing for 1 min followed by sonication for 10 min. Thereafter, lysates were centrifuged at 5,796 G for 10 min and 60 μL of the resulting supernatant were mixed with an equal volume of trichloroacetic acid (TCA). Upon additional centrifugation (5,796 G, 10 min), 50 μL of sample were injected onto the chromatographic column and PN was measured by liquid chromatography with tandem mass spectrometry (LC-MS/MS), as previously described {Johansson, 2010; Midttun, 2009}.

## *Yeast strains assays.*

The haploid WT Saccharomyces cerevisiae strain BY4741 (MATa; his3Δ1; leu2Δ0; met15Δ0; ura3Δ0), its diploid counterpart BY4743 (MATa/MATα; his3Δ1/his3Δ1; leu2Δ0/leu2Δ0; MET15/met15Δ0; LYS2/lys2Δ0; ura3Δ0/ura3Δ0) as well as the null mutants employed in this study were obtained from were obtained from Euroscarf (Frankfurt, Germany). For large-scale experiments (see also Figure 9), yeast cells were cultured on a routine basis at 28 ˚C, on yeast extract peptone dextrose (YEPD) medium containing 1% yeast extract (Difco Laboratories, Detroit, USA), 2% bacto peptone (Difco Laboratories) and 4% D-glucose {Madeo, 2002}. Stationary phase cells from an overnight culture (8-9 x 107 cells/mL) were inoculated in 10 mL of medium at a density of 2.5 x 105 cells/mL, let grow at 28 ˚C for additional 4 h (up to a final concentration of approximately 1 x 106 cells/mL) and treated for further 4 h with 0.5 mM CDDP, 40 μM C2-CER or 0.5 mM CdCl2. Control cultures were treated with an equal volume of solvent: N-N-dimethylformamide (DMF) for CDDP, dimethylsulfoxide (DMSO) for C2-CER and H2O for CdC12.

To achieve vitamin B6-depleted conditions, WT, Δsnol and Δsnzl cells {Rodriguez-Navarro, 2002} were grown in a vitamin B6-free medium composed of 1.7% vitamin-free yeast base (Formedium, Norfolk, UK) supplemented with 1% glucose, all vitamins but PN (according to the composition of the classical yeast nitrogen base, from Formedium), all amino acids, uracil and adenine {Madeo, 2002}. In this case, 0.10 mM CDDP (or an equal amount of DMF) was added shortly before cells ceased to grow (approximately 5 h after inoculation) and maintained for 22 h. When appropriate, WT or Δsnzl cells were pre-incubated for 1 h with 0.05 μM or 1 mM PLP, 1 mM PN or 1 mM aminooxyacetic acid (AOAA) before CDDP administration.

In all settings, cell number was then quantified by means of a CASY® cell counter (Schärfe System, Reutlingen, Germany) and 500 cells from each experimental condition were seeded on three parallel YEPD plates. Following 3 days of incubation at 28 ˚C, plates were finally subjected to the quantification of colony forming units (CFUs). For each genotype or experimental condition, clonogenic survival was calculated as the ratio between the number of CFUs observed in treated and untreated conditions.

## *Isolation of rat liver mitochondria and assessment of mitochondrial swelling.*

Mitochondria were isolated from the liver of male Wistar Kyoto rats (Charles River Laboratories Inc., Wilmington, USA) by differential centrifugation, as previously described {Zischka, 2008}. Briefly, immediately after organ collection, livers were homogenized with a glass teflon homogenizer in an isolation buffer containing 10 mM triethanolamine, 10 mM acetic acid, 280 mM sucrose, 0,2 mM EGTA (pH adjusted to 7,4 with KOH). Homogenates were cleared from debris and nuclei by two rounds of centrifugation at 750 G (10 min, 4˚C). Mitochondria then were pelleted by centrifugation at 9,000 G (10 min, 4˚C), washed three times in isolation buffer (once at 9,000 G, then at 15,000 G, 10 min, 4˚C) and resuspended in EGTA-free isolation buffer. The integrity and functionality of isolated mitochondria was routinely checked by standard respiratory measurements.

For large-amplitude swelling determinations, mitochondria were resuspended in swelling buffer (10 mM Tris-MOPS pH 7.4, 200 mM sucrose, 5 mM succinate, 1 mM phosphate, 10 μM EGTA and 2 μM rotenone) and pre-incubated for 5 min at RT with swelling buffer (negative control) or either of the following permeability transition inhibitors: 5 or 10 μM bongkrekic acid (BA), 1 or 5 μM cyclosporine A (CsA); 0.1 or 0.5 mM reduced glutathione (GSH) or 0.5 or 2 mM N-acetylcysteine (NAC). Then, swelling buffer (negative control) or either of the following chemicals were added: 100 μM Ca$^{2+}$ (positive control), 50 μM GD3 ganglioside, 100 or 200 μM CDDP, 50 or 100 μM C2-CER or 50 or 100 μM CdCl2. The final assay volume was 200 μL, containing 0.5 mg/mL mitochondria. Permeability transition-induced large-amplitude swelling was assessed by measuring light scattering of mitochondrial suspensions at 540 nm on a standard microplate absorbance reader (μ-Quant™, Bio-Tek, Bad Friedrichshall, Germany) every 5 min over a total assay time of 30 min (at RT), as previously described {Zischka, 2008}.

The following mathematical model has been generated from the entire absorbance dataset:

$$ABS_{yt} = \mu + \alpha_i + rep_{ij} + \beta_i \times t + \gamma_i \times \sqrt{t} + \varepsilon_{ijt}$$

where i = experiment number
j = replicate number
t = time
$\varepsilon_{ijt}$ = model residues,

leading to the definition of the following swelling classes (to which each inhibitor/inducer combination has been assigned):

| Class 0 (no swelling) = | $\gamma_1 + 6.5 \times \beta_1 > -0.01$ |
| Class A (linear decrease in absorbance) = | $\gamma_1 + 6.5 \times \beta_1 \geq +0.01$ and $-0.05 \leq \gamma_1 \leq 0.03$ |
| Class B (logarithmic decrease in absorbance) = | $\gamma_1 + 6.5 \times \beta_1 \geq +0.01$ and $-0.15 \leq \gamma_1 \leq 0.05$ |

Class A and B have been further subdivided according to the amplitude of absorbance decrease ($\xi = (ABS_{max} - ABS_{min})$ / $ABS_{max}$) into:

Subclass 1 = $\xi < 0.35$
Subclass 2 = $\xi \geq 0.35$.

### Mouse strains.

C57BL/6 (H-2b) and Swiss Nude mice were obtained from Janvier (Le Genest St. Isle, France) and Charles River, respectively. Animals were maintained in pathogen-free conditions, and all experiments were carried out (upon approval by the local ethical committee) according to the Federation of European Laboratory Animal Science Association (FELASA) guidelines. Mice were used between 6 and 12 weeks of age. During the experiments, animals were subjected to artificial light cycle (12 h lights on - 12 h lights off) and allowed food and drink *ad libitum.*

### Tumor graft models.

$5 \times 10^5$ LLC and A549 cells were subcutaneously xenografted (in 100 $\mu$L sterile PBS) in 25-30 immunocompetent (C57BL/6) and immunodeficient (Swiss Nude) mice, respectively. When the surface of tumors reached 25-40 mm$^2$, n = 4-6 mice were assigned to either of 4 different treatment groups: group 1 = 100$\mu$L intraperitoneal PBS; group 2 = 5 mg/kg intraperitoneal CDDP in 100$\mu$L PBS; group 3 = 240 mg/Kg intraperitoneal orlistat$^®$ in 40$\mu$L PBS; group 4 = 5 mg/kg intraperitoneal CDDP in 100$\mu$L PBS + intraperitoneal orlistat$^®$ in 40$\mu$L PBS. Treatments were administered three times a week (day 1, 3 and 5; day 8 = day 1). Tumor size was monitored on the day of treatment by means of a standard calliper and tumor volume was extrapolated as previously described {Streit, 1999}. Animals bearing tumors that exceeded 20-25% of total body mass or that exhibited large necrotic lesions were sacrificed. Statistical significance was evaluated by two-way ANOVA.

### Patients.

Tumors resected from 122 patients with NSCLC from the Institut Mutualiste Montsouris (Paris, France) were fixed in neutral buffered 10% formalin solution and paraffin-embedded. Inclusion criteria were: (1) histological diagnosis of NSCLC and (2) complete tumor resection. An informed written consent was obtained from patients according to the local ethical committee. Bioptic specimens were collected in the context of the CHEMORES initiative (an EU funded research collaboration aimed at improving cancer treatment by obtaining increased knowledge on mechanisms of chemotherapy resistance) between 2002 and 2006. The main patients' characteristics (*i.e.*, age, gender, smoking status, tumor histology, follow up, administration of adjuvant chemotherapy) are summarized in Table 6.

### Immunohistochemistry.

Paraffin blocks were sectioned (4-$\mu$m thick) on a standard microtome and applied onto histological glass slides (Thermo Fisher Scientific Inc., Waltham, USA). Tissue sections were then deparaffinized in xylene rehydrated by incubation in serial (95%, 70%, 50%, 30%, v/v in PBS) ethanol baths (2 min/bath). Immunostaining was performed following standard procedures {Loriot, 2010; Olaussen, 2006}. Briefly, epitope retrieval was performed by incubating slides in either Tris-EDTA (pH 7.8 or 9), 10 mM citrate buffer (pH 6.0 or pH 7.3) for 30-40 min. Endogenous peroxidase activity was inhibited by treatment with 3% H2O2 for 10 min. Unspecific binding sites were then blocked for 10 min with Protein Block reagent (San Ramon, UA, USA) and slides were incubated for 1 h at RT with primary antibodies that recognizes: ALDH7A1 (rabbit antiserum #AB51029, Abcam plc.; dilution 1:400), ALK (mouse monoclonal IgG$_3$ #M7195, Dako, Carpinteria, USA; dilution 1:30), BCL2L1 (mouse monoclonal IgG$_2$a #AHO0222, Invitrogen; dilution 1:50), LIPC (mouse monoclonal IgG$_1$ #SC-21741, Santa Cruz Biotechnology Inc., dilution 1:600), PDXK (rabbit antiserum #AP7167A, Abgent, San Diego, USA; dilution 1:50), PDXP (rabbit antiserum #HPA001099, Sigma-Aldrich; dilution 1:200) or WSB2 (rabbit antiserum #12124-2-AP, ProteinTech, Chicago, USA; dilution 1:200). Slides were then washed in PBS and incubated for 30 min at RT with secondary antibodies (Vectastain$^®$ Universal Elite ABC Kit, from Vector Laboratories Inc., Burlingame, USA). Revelation was performed by the streptavidin-biotin-peroxidase complex method with 3,3'-diaminobenzidine tetrahydrochloride (DAB) as chromogenic substrate. Slides were counterstained with Mayer's hematoxylin (Dako), mounted with glass coverslips (Thermo Fisher Scientific Inc.) and observed by means of a DM2000 microscope equipped with HC PL Fluotar 20X/0.50 and 40X/0.75 objectives and coupled to a DFC280 CCD camera (all from Leica Microsystems GmbH, Wetzlar, Germany).

For each antibody, staining intensity was quantified on a 0-3 scale, using as a reference the signal observed in fibroblasts or endothelial cells (score 2). The percentage of reactive tumor cells was scored on a 0%-100% scale. These variables were combined by calculating each marker's score (ranging from 0-300) as the product between staining intensity (0-3) and the percentage of stained cells (0-100). The cutoff value for patient stratification was arbitrarily chosen as the score median for all markers but LIPC, for which 0 was used (positive *versus* negative tumors). Outcome variables were overall survival (OS) and disease-free survival (DFS) starting from the surgery date. The objective was to explore whether or not the seven markers carry prognostic and/or predictive information relative to DFS and/or OS. Estimated distributions of time-to-event data are displayed as Kaplan-Meier plots, and survival curves were compared by means of the log-rank test. The Cox proportional hazard model was employed to estimate the interaction between the presence/absence of adjuvant chemotherapy and patient status relative to each marker. Statistical analyses were performed on the open-source software environment for statistical computing and graphics R {Schwarzer, 2007}.

## RESULTS AND DISCUSSION

### Genome-wide siRNA screening for the identification of new CDDP response modifiers.

A genome-wide panel of small interfering RNAs (two siRNAs specific for 23,078 transcripts) was transfected into human NSCLC A549 cells, which then were either left untreated or treated with 50 $\mu$M CDDP for 24 hours (which kills ~45% of cells). Finally, cells were assayed for the conversion of a tetrazolium salt (WST-1, which provides an estimate for cell density/survival) and lactate dehydrogenase (LDH) release (which provides an estimate for plasma membrane breakdown). Cytotoxic (0.4% of all siRNAs) and anti-proliferative (12.2%) siRNAs, *per se* leading to increased LDH release or decreased WST-1 conversion, respectively, were excluded from further analysis. One thousand transcripts for which both specific siRNAs yielded a concordant and significant modulatory effect on the CDDP-mediated reduction of WST-1 conversion were selected based on their biological potency in the primary screen and subjected to further analysis. Two additional siRNAs were used in further rounds of confirmation, and 85 transcripts for which at least three out of four non-overlapping siRNAs yielded significant and consistent results were selected (Fig. 1A-C, Table 1). 53 of the transcripts are "cytoprotectors" (because their depletion enhances CDDP-induced cell death) and 32 are "chemosensitizers" (because their knockdown reduces CDDP-mediated killing). To confirm the results obtained with WST-1, siRNA-transfected A549 cells were treated with a sub-apoptotic (25 $\mu$M) or highly efficient (75 $\mu$M) dose of CDDP for 48h, and then co-stained with the mitochondrial transmembrane potential ($\Delta\psi_m$-sensitive dye DiOC$_6$(3) and the vital dye propidium iodide (PI) to determine the frequency of dying (PI$^-$ DiOC$_6$(3)$^{low}$) and dead (PI$^+$ DiOC$_6$(3)$^{low}$) cells (Fig. 1D, Fig. 8A, Table 2). In addition, pharmacological inhibitors specific for druggable siRNA targets were used to validate the screening results (Fig. 1E). For example, both the depletion and inhibition of ZDHHC9 (a palmitoyl transferase of H-RAS and N-RAS), interleukin 6 receptor (IL6R), BCL2L1 (better known as BCL-X$_L$), and hepatic lipase (LIPC) sensitized to CDDP-induced cell death, while the depletion/inhibition of two distinct cytochrome c oxidase (COX) subunits and that of apoptotic peptidase activating factor 1 (APAF1) protected against CDDP (Fig. 1D, E, Fig. 8B). Thus, the screening effort identified known apoptosis modulators (such as BCL-X$_L$ and APAF1) as well as proteins that had not previously been implicated in CDDP-induced signalling pathways.

**Table 2: Main siRNAs used in this study**

Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany).

| Name | Accession number | Sense sequence | Source |
|---|---|---|---|
| siACHE_1 | NM_000665 | 5'-CGCCCTCTGGCTGCAAATAAA-3' (SEQ ID NO : 5) | |
| siACHE_2 | | 5'-CCAGAGTGTCTGCTACCAATA-3' (SEQ ID NO : 6) | |

(continued)

| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
| --- | --- | --- | --- |
| **Name** | **Accession number** | **Sense sequence** | **Source** |
| siADAR_1 | NM_001111 | 5'-TCCCTCGATAACAGTCAGCTA-3' (SEQ ID NO : 7) | This study* |
| siADAR_2 | | 5'-TTCCGTTACCGCAGGGATCTA-3' (SEQ ID NO : 8) | |
| siALDH7A1_1 | NM_001182 | 5'-TCCGATTCTCTATGTCTTTAA-3' (SEQ ID NO : 9) | |
| siAEDH7A1_2 | | 5'-AAGGATGATTGGAGGACCTAT-3'(SEQ ID NO: 10) | |
| SiALK_1 1 | NM_004304 | 5'-CACCTACGTATTTAAGATGAA-3' (SEQ ID NO : 11) | |
| siAEK_2 | | 5'-CTCGACCATCATGACCGACTA-3' (SEQ ID NO : 12) | |
| siAPAF1_1 | NM_001160 | 5'-CAGTGAAGGTATGGAATATTA-3' (SEQ ID NO : 13) | |
| siAPAF1_2 | | 5'-TAGGCAGAGTATAAAGTATTA-3' (SEQ ID NO: 14) | |
| siASTL_1 | NM_001002036 | 5'-CAGGCTTTGAAATCAACTTCA-3' (SEQ ID NO : 15) | |
| siASTL_2 | | 5'-TCCCATCTTCAGAAGCAGGAA-3' (SEQ ID NO : 16) | |
| siBAIAP3_1 | NM_003933 | 5'-GTCGACCTTGCTGGACATTAA-3' (SEQ ID NO : 17) | |
| siBAIAP3_2 | | 5'-CTCGCCTGACTCCATCCAGAA-3'(SEQ ID NO : 18) | |
| siBAK1_1 | NM_001188 | 5'-GCGAAGUCUUUGCCUUCUCdTdT-3'(SEQ ID NO : 19) | Qiagen** |
| siBAX_1 | NM_004324 | 5'-GGUGCCGGAACUGAUCAGAdTdT-3'(SEQ ID NO : 20) | (Vitale, |
| siBCL2_1 | NM_000657/NM_000633 | 5'-GCUGCACCUGACGCCCUUCdTdT-3'(SEQ ID NO : 21) | (Vicencio, |
| siBCL2L1_1 | NM_001191 | 5'-AAAGTGCAGTTCAGTAATAAA-3' (SEQ ID NO : 22) | |
| siBCL2L1_2 | | 5'-CAAGCTTTCCCAGAAAGGATA-3' (SEQ ID NO : 23) | |
| siBMP7 1 | NM_001719 | 5'-CCCGGAAGTTCCTGTAATAAA-3' (SEQ ID NO : 24) | |
| siBMP7_2 | | 5'-CTCGTGGAACATGACAAGGAA-3' (SEQ ID NO : 25) | |

(continued)

| | Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
|---|---|---|---|
| **Name** | **Accession number** | **Sense sequence** | **Source** |
| siBSN_1 | NM_003458 | 5'-AAAGGACTTGATATAAACAAA-3' (SEQ ID NO : 26) | This study* |
| siBSN_2 | | 5'-CACGCTGTGTCCCATATGCAA-3' (SEQ ID NO : 27) | |
| siBTNL2_1 | NM_019602 | 5'-ATGGAGGGACATGGAAGGAAA-3'(SEQ ID NO : 28) | |
| siBTNL2_2 | | 5'-CAGAGTGGGAGAAGATATACA-3'(SEQ ID NO : 29) | |
| siC100RF92_1 1 | NM_017609 | 5'-CAGGGCCTATTGTCACTTCAA-3' (SEQ ID NO : 30) | |
| siC100RF92_2 | | 5'-CAGGAGAGGCGAGGACCTGAA-3'(SEQ ID NO: 31) | |
| siC10RF91 | NM_019118 | 5'-ACCGTCTGTCTTCACAAAGTA-3' (SEQ ID NO : 32) | |
| siC1ORF91_2 | | 5'-CTGGCGAGTAGTAGCTGTCAA-3'(SEQ ID NO : 33) | |
| siC210RF2_1 | NM_004928 | 5'-AAGCGCTTTGTTGGAATGGTA-3' (SEQ ID NO : 34) | |
| siC21ORF2_2 | | 5'-CCGCATGAAGCTGACGCGGAA-3' (SEQ ID NO : 35) | |
| siCASP2_1 | NM_032982/NM_032983 | 5'-ACAGCUGUUGUUGAGCGAAdTdT-3'(SEQ ID NO : 36) | (de La Motte |
| siCCBL1_1 | NM_004059/NM_001122671 | 5'-CCAGUACACCAAGACAUUUdTdT-3'(SEQ ID NO : 37) | This study |
| siCCBL1_2 | | 5'-GGUCCAGAUCACAUCAUGAdTdT-3'(SEQ ID NO: 38) | |
| siCD151_1 | NM_004357 | 5'-CACCCTGTGCCATCACCATAA-3' (SEQ ID NO : 39) | This study* |
| siCD151_2 | | 5'-CTGCCTGTACAGGAGTCTCAA-3'(SEQ ID NO : 40) | |
| siCD34 1 | NM_001773 | 5'-CACGTGGTGGCTGATACCGAA-3' (SEQ ID NO : 41) | |
| siCD34_2 | | 5'-AACGGCCATTCAGCAAGACAA-3'(SEQ ID NO : 42) | |
| siCLIC1_1 | NM_001288 | 5'-AAGGTGTCTCTCAGAGGAAGT-3' (SEQ ID NO : 43) | |
| siCLIC1_2 | | 5'-TTCCTGCTGTATGGCACTGAA-3' (SEQ ID NO : 44) | |

(continued)

| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
|---|---|---|---|
| **Name** | **Accession number** | **Sense sequence** | **Source** |
| siCOMMD9_1 | NM_014186 | 5'-AAGCTCCTGAGTGCAACTTAA-3' (SEQ ID NO : 45) | This study* |
| siCOMMD9_2 | | 5'-CCCGCTGTTCTCACTCATCTT-3' (SEQ ID NO : 46) | |
| siCOX5B_1 | NM_001862 | 5'-CACCTGCACTAAATTACTCAA-3' (SEQ ID NO : 47) | |
| siCOX5B_2 | | 5'-AGGCACCAGGGAAGACCCTAA-3'(SEQ ID NO : 48) | |
| siCOX7C_1 | NM_001867 | 5'-CTAGATATGTTTGTCAATAAA-3' (SEQ ID NO : 49) | |
| siCOX7C_2 | | 5'-CAAGTGGTCGTTACTAGCTAA-3' (SEQ ID NO : 50) | |
| siCYP11B2 1 | NM_000498 | 5'-CACTAGATCATGGGATCCTAA-3' (SEQ ID NO : 51) | |
| siCYP11B2_2 | | 5'-AAGGCGGAACTGTCACTAGAA-3'(SEQ ID NO : 52) | |
| siDEFB4_1 | NM_004942 | 5'-GCCCTAGAAGGTATAAACAAA-3' (SEQ ID NO : 53) | |
| siDEFB4_2 | | 5'-TGCCTTAAGAGTGGAGCCATA-3' (SEQ ID NO : 54) | |
| siDHRSX_1 | NM_145177 | 5'-CCGCGTTAATACAACTGAGTA-3' (SEQ ID NO : 55) | |
| siDHRSX_2 | | 5'-TGCCCTGATAAGCAACTTAAA-3' (SEQ ID NO : 56) | |
| siEBP 1 | NM_006579 | 5'-ACTGGACAACTTTGTACCTAA-3' (SEQ ID NO : 57) | |
| siEBP_2 | | 5'-CACAGTGTGCATGGAAACCAT-3'(SEQ ID NO : 58) | |
| siEEF2 1 | NM_001961 | 5'-CCGCGCCATCATGGACAAGAA-3' (SEQ ID NO : 59) | |
| siEEF2_2 | | 5'-TGCCGTTTCTTTCAATATTTA-3' (SEQ ID NO : 60) | |
| siFBXO42_1 | NM_018994 | 5'-CTGGGATCAAATCTCCATTAA-3' (SEQ ID NO : 61) | |
| siFBXO42_2 | | 5'-CTCCTGTGTGATAGATGATAA-3' (SEQ ID NO : 62) | |
| siFLOT2_1 | NM_004475 | 5'-CTGCCTGTCCCTTCTGGTAAA-3' (SEQ ID NO : 63) | |
| siFLOT2_2 | | 5'-CACCAAGATTGCTGACTCTAA-3' (SEQ ID NO : 64) | |

(continued)

| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
|---|---|---|---|
| **Name** | **Accession number** | **Sense sequence** | **Source** |
| siFN3KRP_1 | NM_024619 | 5'-CCGGACTAGCTTAAGACCAAT-3' (SEQ ID NO : 65) | |
| siFN3KRP_2 | | 5'-ACGAGTGTTCGTGAAAGTGAA-3'(SEQ ID NO : 66) | |
| siGRLF1_1 | NM_004491 | 5'-CAGGATGTTCTGGGAGAGGAA-3'(SEQ ID NO : 67) | |
| siGRLF1_2 | | 5'-CACCACCGAAGAGGTGTTTAA-3' (SEQ ID NO : 68) | |
| SiGUCA1A_1 | NM_000409 | 5'-CACCGATACAGTGTTCTCCAA-3' (SEQ ID NO : 69) | |
| siGUCA1A_2 | | 5'-GCCGGTCGTTGTGGACTCTAA-3' (SEQ ID NO : 70) | |
| siHDLBP_1 | NM_005336 | 5'-AAGGATCTAATCATTGAGCAA-3' (SEQ ID NO : 71) | |
| siHDLBP_2 | | 5'-ATCCGTGAAATTCGTGACAAA-3' (SEQ ID NO : 72) | |
| siHGD_1 | NM_000187 | 5'-ACCCTACAAGTACAACCTGAA-3' (SEQ ID NO : 73) | |
| siHGD_2 | | 5'-CGGGAATTATACACCCTACAA-3' (SEQ ID NO : 74) | |
| siHIRIP3_1 | NM_003609 | 5'-TAGGAAGAAACCTGTGGTAAA-3' (SEQ ID NO : 75) | |
| siHIRIP3_2 | | 5'-CAGCCAAAGAGGAGAATCCAA-3'(SEQ ID NO : 76) | |
| siIAPP_1 | NM_000415 | 5'-TTAGAGGACAATGTAACTCTA-3' (SEQ ID NO : 77) | |
| siIAPP_2 | | 5'-ATGCAGGGTATTGCGAAACAA-3' (SEQ ID NO : 78) | |
| siIL6R_1 | NM_000565 | 5'-ACCCAGTTAGCTCTCAAGTTA-3' (SEQ ID NO : 79) | |
| siIL6R_2 | | 5'-CTGCGGGACCATGGAGTGGTA-3' (SEQ ID NO : 80) | |
| siITGB6_1 | NM_000888 | 5'-CTGGAATTACTTGTCAGCCCA-3' (SEQ ID NO : 81) | |
| siITGB6_2 | | 5'-AACCCTTGCAGTAGTATTCCA-3' (SEQ ID NO : 82) | |
| siKHSRP_1 | NM_003685 | 5'-CAAGAGGAGATTGAAACTGAA-3' (SEQ ID NO : 83) | |
| siKHSRP_2 | | 5'-CAGGATTCAGGCTGCAAAGTA-3' (SEQ ID NO : 84) | |

(continued)

| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
|---|---|---|---|
| **Name** | **Accession number** | **Sense sequence** | **Source** |
| siKIA1161_1 | NM_020702 | 5'-CAGGCCAGACTTCGTGCCTTA-3'(SEQ ID NO : 85) | |
| siKIA1161_2 | | 5'-CGCGGCCGCCATCAAAGTCAA-3' (SEQ ID NO : 86) | |
| siLIPC_1 | NM_000236 | 5'-CAGGAGAAACCCAGCAAAGAA-3' (SEQ ID NO : 87) | |
| siLIPC_2 | | 5'-CTGAAGACGATCAGAGTCAAA-3' (SEQ ID NO : 88) | |
| siLOC388882_1 | XR_042117 | 5'-CACCTTGGTGAGAATCAGGAA-3'(SEQ ID NO : 89) | |
| siLOC388882_2 | | 5'-CTGCTGTACATGACATCTCTA-3' (SEQ ID NO : 90) | |
| siLOC389727_1 | Pseudogene | 5'-CCACGCAGAGATGACAGCCAA-3'(SEQ ID NO : 91) | |
| siLOC389727_2 | | 5'-CTGGCGGTGAATGACTTTGAA-3'(SEQ ID NO : 92) | |
| siLOC390533_1 | Discontinued | 5'-CGGGTGGTATGGCCAAAGCAT-3' (SEQ ID NO : 93) | |
| siLOC390533_2 | | 5'-ATACAACAGCTTCTTAGGTAA-3' (SEQ ID NO : 94) | |
| siLOC440056_1 | Discontinued | 5'-CCCGAGGCTTCGCTCCGCAAA-3'(SEQ ID NO : 95) | |
| siLOC440056_2 | | 5'-CTCCGGCGCGAGCCTCCCAAA-3' (SEQ ID NO : 96) | |
| siLOC440611_1 | Discontinued | 5'-CACCGAAAGAGTGTTTCCAAA-3' (SEQ ID NO : 97) | |
| siLOC440611_2 | | 5'-AAAGAGTGTTTCAAACGTGAA-3' (SEQ ID NO : 98) | |
| siLOC441115_1 | Discontinued | 5'-CAGAGCTACCTCAAACAGGAA-3' (SEQ ID NO : 99) | |
| siLOC441115_2 | | 5'-AGGGAGGTCTTTGAAAGTTCA-3' (SEQ ID NO : 100) | |
| siLOC442126_1 | Discontinued | 5'-CAGGGCGGGCGCAGTCCTGGA-3' (SEQ ID NO : 101) | |
| siLOC442126_2 | | 5'-TCGCACGAGCGGGATACTCCA-3' (SEQ ID NO : 102) | |
| siLRMP_1 | NM_006152 | 5'-CTGGGAAAGTATAGCATGAAA-3' (SEQ ID NO : 103) | |
| siLRMP_2 | | 5'-CACAAACTATATGGTGGTCAA-3' (SEQ ID NO : 104) | |

(continued)

| | | | |
|---|---|---|---|
| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
| **Name** | **Accession number** | **Sense sequence** | **Source** |
| siMAT2A_1 | NM_005911 | 5'-CAGCAGGATCCTGATGCCAAA-3' (SEQ ID NO : 105) | This study* |
| siMAT2A_2 | | 5'-CAGGGAGTGTTCCCTATCCAA-3'(SEQ ID NO : 106) | |
| siMCL1_1 | NM_021960 | 5'-CTGGTTTGGCATATCTAATAA-3' (SEQ ID NO : 107) | |
| siMCL1_2 | | 5'-CGGGACTGGCTAGTTAAACAA-3' (SEQ ID NO : 108) | |
| siMPP6 1 | NM_016447 | 5'-TGGAAGGTGTACTAATATATA-3' (SEQ ID NO : 109) | |
| siMPP6_2 | | 5'-CTGATGATTCAGTAAGGTTAA-3' (SEQ ID NO: 110) | |
| siNANOS1_1 | NM_199461 | 5'-CAGGCAGTGCTCAAACTAGAA-3' (SEQ ID NO : 111) | |
| siNANOS1_2 | | 5'-CACCAGTTTACCCATAAGCAA-3' (SEQ ID NO: 112) | |
| siNCKAP1_1 | NM_013436 | 5'-ACGCATGAACTATGTCCAGAA-3' (SEQ ID NO: 113) | |
| siNCKAP1_2 | | 5'-CAGGCATATACTAGTGTCTCA-3' (SEQ ID NO: 114) | |
| siNCOA3_1 | NM_006534 | 5'-CAGGAGGAGATTATAATACTT-3' (SEQ ID NO : 115) | |
| siNCOA3_2 | | 5'-CCGACAGGCACTTGAATTGAA-3'(SEQ ID NO: 116) | |
| siNLRP1_1 | NM_014922 | 5'-CAGCTTCTGCTCGCCAATAAA-3' (SEQ ID NO : 117) | |
| siNLRP1_2 | | 5'-CTGGTTTGCCTTCCAGCACTA-3' (SEQ ID NO : 118) | |
| siOSTCL_1 | NM_145303 | 5'-AAGCATTGGCTCTATGACTGA-3' (SEQ ID NO : 119) | |
| siOSTCL_2 | | 5'-AAAGGCTGTCCAATCCTCTAA-3' (SEQ ID NO : 120) | |
| siOVCH1_1 | NM_183378 | 5'-TACGAGGTGCATTTGGTATAA-3' (SEQ ID NO: 121) | |
| siOVCH1_2 | | 5'-AGCGTGTAATACTGTGCTCAA-3' (SEQ ID NO : 122) | |
| siPDXK_1 | NM_003681 | 5'-TCGAGTGACTTTCTAACCCAA-3' (SEQ ID NO : 123) | |
| siPDXK_2 | | 5'-CCCTGTATTAAGCAAGAATTA-3' (SEQ ID NO : 124) | |

(continued)

Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany).

| Name | Accession number | Sense sequence | Source |
|---|---|---|---|
| siPDXP_1 | NM_020315 | 5'-GGUUUGAGGGCCCUUGCAAdTdT-3' (SEQ ID NO : 125) | This study |
| siPDXP_2 | | 5'-GUGAUAGUGACUCAUCAAUdTdT-3' (SEQ ID NO : 126) | |
| siPLD3_1 | NM_012268 | 5'-TTGAGGGAAGATGAAGCCTAA-3' (SEQ ID NO : 127) | This study* |
| siPLD3_2 | | 5'-CCGCATGGTGGACATGCAGAA-3' (SEQ ID NO : 128) | |
| siPLK1S1_1 | NM_018474 | 5'-AAGGAAATATGTGAATCTGAA-3' (SEQ ID NO : 129) | |
| siPLK1S1_2 | | 5'-CAGCGGTGCATTCGAGACAAA-3' (SEQ ID NO : 130) | |
| SiPPM1B_1 1 | NM_002706 | 5'-CAACCAAGTGTTTAGAATGAA-3' (SEQ ID NO: 131) | |
| siPPMIB_2 | | 5'-TAGCCTAACTACACACATCAA-3' (SEQ ID NO : 132) | |
| SiPPMIJ_1 | NM_005167 | 5'-AACGAGATGGATAAAGGTGAA-3'(SEQ ID NO : 133) | |
| SiPPM1J_2 | | 5'-AGGGATGTCCGCTATATCCAA-3' (SEQ ID NO : 134) | |
| siPRSS21_1 | NM_006799 | 5'-CCACTTTGAGTGGATCCAGAA-3' (SEQ ID NO : 135) | |
| siPRSS21_2 | | 5'-ACCCTTGGCCTGTAACAAGAA-3' (SEQ ID NO : 136) | |
| siPSMC5_1 | NM_002805 | 5'-TTGGTCAAGGTACATCCTGAA-3' (SEQ ID NO : 137) | |
| siPSMC5_2 | | 5'-CCGGGTGGCTCTAAGGAATGA-3'(SEQ ID NO : 138) | |
| siPSMD7_1 | NM_002811 | 5'-TTCCGTATTGGTCATCATTGA-3' (SEQ ID NO : 139) | |
| siPSMD7_2 | | 5'-CAGGCCCTAAACTACACAAGA-3' (SEQ ID NO : 140) | |
| siRAB3C_1 | NM_138453 | 5'-GTGGCAAATATGTGATCTTAA-3' (SEQ ID NO: 141) | |
| siRAB3C_2 | | 5'-AAGGACATTTGCCAACAATGA-3' (SEQ ID NO : 142) | |
| siRAB40A_1 | NM_080879 | 5'-ATGGGAGGAAGAAATAGTACA-3'(SEQ ID NO : 143) | |
| siRAB40A_2 | | 5'-TAGGCTGAATAATCTCCTGTA-3' (SEQ ID NO : 144) | |

(continued)

| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
|---|---|---|---|
| **Name** | **Accession number** | **Sense sequence** | **Source** |
| siRAC2_1 | NM_002872 | 5'-AAGGAGATTGACTCGGTGAAA-3' (SEQ ID NO : 145) | |
| siRAC2_2 | | 5'-CAATGTGATGGTGGACAGCAA-3' (SEQ ID NO : 146) | |
| siRNF26 1 | NM_032015 | 5'-CACTAGGGTCCAAATACAGAA-3' (SEQ ID NO : 147) | |
| siRNF26_2 | | 5'-GACCTTGGTGTTGGACCTCAA-3'(SEQ ID NO: 148) | |
| siRPSAP32_1 | Pseudogene | 5'-CACTACTGAAATCCTCACTAA-3' (SEQ ID NO : 149) | |
| siRPSAP32_2 | | 5'-CTGACTATCATTACTCTATGA-3' (SEQ ID NO: 150) | |
| siRRAD_1 | NM_004165 | 5'-CGCGGTGGTCTTTGACTGCAA-3'(SEQ ID NO: 151) | This study* |
| siRRAD_2 | | 5'-CCCGGACGCGATGACCCTGAA-3' (SEQ ID NO : 152) | |
| siSEMA3C_1 | NM_006379 | 5'-CAGAATATGATTCACTATTTA-3' (SEQ ID NO: 153) | |
| siSEMA3C_2 | | 5'-TCCCTGAATATTAACAATATA-3' (SEQ ID NO : 154) | |
| siSEMG2_1 | NM_003008 | 5'-CAGGTAACAATTCATAGTCAA-3' (SEQ ID NO: 155) | |
| siSEMG2_2 | | 5'-AAGGCAGTATTTCGATCCAAA-3' (SEQ ID NO : 156) | |
| siSLC22A18AS_1 | NM_007105 | 5'-CTGCAGCAACATAGAGTACAA-3' (SEQ ID NO : 157) | |
| siSLC22A18AS_2 | | 5'-CTCAGCCAGTCTTAAAGGCAA-3' (SEQ ID NO : 158) | |
| siSLC39A5_1 | NM_173596 | 5'-CTCCTGGGACCTCGTCTACTA-3' (SEQ ID NO : 159) | |
| siSLC39A5_2 | | 5'-CAGGCTTCTGTTGCTGGACCA-3'(SEQ ID NO : 160) | |
| siSOCS6_1 | NM_004232 | 5'-TTGATCTAATTGAGCATTCAA-3' (SEQ ID NO : 161) | |
| siSOCS6_2 | | 5'-TAGAATCGTGAATTGACATAA-3' (SEQ ID NO : 162) | |
| siSTAT3_1 | NM_003150 | 5'-CTGGTCTTAACTCTGATTGTA-3' (SEQ ID NO : 163) | |
| siTCTE3 1 | NM_174910 | 5'-TAGAATGGAGCCATTGAAGAA-3' (SEQ ID NO : 164) | |
| siTCTE3_2 | | 5'-CTCAATTGCTGATATAGGTAA-3' (SEQ ID NO : 165) | |

(continued)

| | | | |
|---|---|---|---|
| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
| **Name** | **Accession number** | **Sense sequence** | **Source** |
| siTFF1_1 | NM_003225 | 5'-CACCATGGAGAACAAGGTGAT-3'(SEQ ID NO : 166) | |
| siTFF1_2 | | 5'-ACCATCGACGTCCCTCCAGAA-3'(SEQ ID NO : 167) | |
| siTMED1_1 | NM_006858 | 5'-CCGGCCAAAGTCTAGGCAGAA-3'(SEQ ID NO : 168) | |
| siTMED1_2 | | 5'-GAGGAGATGCTGGATGTTAAA-3'(SEQ ID NO : 169) | |
| siTP53_1 | NM_000546 | 5'-GACUCCAGUGGUAAUCUACdTdT-3' (SEQ ID NO : 170) | (Hoffmann, |
| siTRIP4_1 | NM_016213 | 5'-CAGAAATCAGGCGACCATCTA-3' (SEQ ID NO : 171) | |
| siTRIP4_2 | | 5'-CAGCTGCGAATCCAGGATCAA-3' (SEQ ID NO : 172) | |
| siUBE2L3_1 | NM_003347 | 5'-ACCACCGAAGATCACATTTAA-3' (SEQ ID NO : 173) | |
| siUBE2L3_2 | | 5'-AAGGAGCAGCACCAAATCCAA-3' (SEQ ID NO : 174) | This study* |
| siUBE2T_1 | NM_014176 | 5'-TCGCAACTGTGTTGACCTCTA-3' (SEQ ID NO: 175) | |
| siUBE2T_2 | | 5'-AAGAGAGAGCTGCACATGTTA-3' (SEQ ID NO : 176) | |
| siUNR | -- | 5'-GCCGGUAUGCCGGUUAAGUdTdT-3' (SEQ ID NO : 177) | (Mondragon, |
| siUSP8_1 | NM_005154 | 5'-CAGGTTCAGGCAAGCCATTTA-3' (SEQ ID NO : 178) | This study* |
| siUSP8_2 | | 5'-AAGGCTCGTATTCATGCAGAA-3' (SEQ ID NO : 179) | |
| siVDAC1_1 | NM_003374/NM_003150 | 5'-GUACGGCCUGACGUUUACAdTdT-3'(SEQ ID NO : 180) | (Tajeddine, |
| siWSB2 1 | NM_018639 | 5'-CACGGCTTCTTACGATACCAA-3' (SEQ ID NO: 181) | |
| siWSB2_2 | | 5'-CACGTTAATTCGGAAGCTAGA-3' (SEQ ID NO: 182) | |
| siZDHHC9_1 | NM_001008222 | 5'-AACCAGATTGTGAAACTGAAA-3' (SEQ ID NO: 183) | This study* |
| siZDHHC9_2 | | 5'-AGCAGGAATGGCAGTAATAAA-3' (SEQ ID NO : 184) | |

(continued)

| | | | |
|---|---|---|---|
| Sense and antisense sequences of the main siRNAs used in this study. Accession number and source are provided. Please note that some of most (but not all) of these siRNAs were part of the Human Whole Genome siRNA Set Version 1.0 (Qiagen, Hilden, Germany). | | | |
| **Name** | **Accession number** | **Sense sequence** | **Source** |
| siZNF878 1 | NM_001080404 | 5'-AAGCATTATCTTATCTTGTAA-3' (SEQ ID NO : 185) | |
| siZNF878_2 | | 5'-TAGAGTTGCCTCACAACTTAA-3' (SEQ ID NO: 186) | |
| * = Human Whole Genome siRNA Set Version 1.0 (Qiagen, Hilden, Germany) ** = HP Validated siRNA (Qiagen, Hilden, Germany) | | | |

### *Identification of general cell death modulators versus Cisplatin (CDDP) Response Modifiers (CRMs).*

To discriminate between general modulators of cell death and specific CRMs, inventors characterized the effect of siRNAs identified by our primary screen in A549 cells responding to a phylogenetically conserved stress mediator, ceramide, and an environmental toxin, the heavy metal cadmium. All these agents including CDDP stimulate the intrinsic pathway of apoptosis. However, only cadmium can mediate direct mitochondrion-permeabilizing effects, as determined in cell-free experiments (Fig. 8C-E). CDDP elicited a transcriptional response, as measured in pre-apoptotic conditions, which was clearly different from that induced by ceramide and cadmium (Fig. 2A, B). Among the 53 cytoprotective transcripts (Table 1), only 4 were upregulated and 4 downregulated at 12 h of CDDP treatment (2 and 3 were up- and downregulated, respectively, at 24h) (Fig. 2C). Moreover, among the 32 chemosensitizing transcripts (Table 1), 2 were upregulated and 5 were downregulated at 12h (2 and 4 were up- and downregulated, respectively, at 24h) (Fig. 2C, Table 3).

Next, inventors compared the effects of the 85 siRNAs on the cytotoxic/antiproliferative effects of CDDP, ceramide and cadmium. The biological effects of siRNAs were sorted for CDDP (Fig. 2D) and then plotted in the same order for the other cell death inducers (Fig. 2E, F), clearly demonstrating that most siRNAs modulated inducer-specific rather than general cell death pathways. For example, knockdown of 2 distinct COX subunits protected against, while the depletion of ZDHHC9 and IL6R sensitized to, CDDP only, but not to ceramide and cadmium. On the other hand, knockdown of the essential caspase activator, APAF1, protected against all three cell death inducers. Similarly, the response to CDDP, ceramide and cadmium was assayed in a panel of yeast strains that were deficient for cell death-relevant genes, yielding rather distinct patterns in cell death modulation and hence corroborating the results generated in human cells (Fig. 9). Next, they tested the 85 siRNAs discovered in A549 cells for their capacity to modulate the CDDP response in six distinct CDDP-resistant A549 clones, four additional NSCLC cell lines and wild-type or *TP53*$^{-/-}$ HCT 116 colon cancer cells, revealing cell line-specific and cancer-type specific modulations (Fig. 3A, Table 4).

Moreover, on A549 cells responding to 11 different cytotoxic drugs, they found that the 85 siRNAs induced relatively similar modifications in the response to CDDP and carboplatin, yet rather dissimilar changes when CDDP and oxaliplatin were compared (Fig. 4A, Table 4). This method revealed the existence of general (*e.g.,* COX5B, PSMD7, TRIP4) and drug-specific (e.g., ALK, CLIC1, IL6R, etc.) modifiers of the chemotherapeutic response.

### Table 3: Overlap between siRNA screening hits and the transcriptional signatures of A549 cells treated with cisplatin (CDDP), C2-ceramide (C2-CER) and cadmium dichloride (CdCl2).

Each siRNA screen hit whose abundance was significantly changed in any of the experimental settings assayed for transcriptome modifications is reported in Table 4 together with accession number, score, Δ, as well as fold change (FC) and p value

* = upon background subtraction and inter-plate normalization, Δ = relative survival of siRNA-transfected cells (WST-1 signal from CDDP treated, siRNA-transfected cells / WST-1 signal from untreated, siRNA-transfected cells) - relative survival of cells transfected with a control siRNA (siUNR) (WST-1 signal from CDDP treated, siUNR-transfected cells / WST-1 signal from untreated, siUNR-transfected cells). Negative and positive Δ values signify chemosensitization and cytoprotection, respectively.

**= n° of siRNAs (out of 4 tested) that significantly modulated cisplatin (CDDP)-induced cell death

EP 2 426 216 A1

| N | Accession | Symbol | △* | Score** | CDDP 12h | | CDDP 24h | | C2-CER 12h | | C2-CER 24h | | CdCl₂ 12h | | CdCl₂ 24h | |
|---|---|---|---|---|---|---|---|---|---|---|---|---|---|---|---|---|
| | | | | | *p* value | Fold change | *p* value | Fold change | *p* value | Fold change | *p* value | Fold change | *p* value | Fold change | *p* value | Fold change |
| 1 | NM_018639 | WSB2 | -15.79 | 4 | n.s. | n.s. | 2.27E-06 | 1.35 | n.s. | n.s. | n.s. | n.s. | 7.95E-07 | 1.32 | n.s. | n.s. |
| 2 | NM_005911 | MAT2A | -13.51 | 3 | 1.58E-30 | -1.97 | n.s. | n.s. | 0.00E+00 | -3.47 | 3.75E-28 | -2.96 | 0.00E+00 | -4.30 | 0.00E+00 | -3.05 |
| 3 | NM_018474 | PLK1S1 | -12.10 | 3 | n.s. | n.s. | n.s. | n.s. | 7.16E-06 | 1.99 | 5.38E-08 | 1.93 | n.s. | n.s. | n.s. | n.s. |
| 2 | NM_372092 | LOC389727 | -11.75 | 3 | n.s. | n.s. | n.s. | n.s. | n.s. | n.s. | n.s. | n.s. | 2.7E-11 | 2.09 | n.s. | n.s. |
| 5 | NM_021960 | MCL1 | -11.71 | 3 | 9.24E-20 | 2.48 | n.s. | n.s. | 1.20E-06 | -1.35 | 2.43E-26 | -1.59 | 1.44E-11 | 1.37 | 1.26E-14 | 1.57 |
| 6 | NM_000565 | IL8R | -11.67 | 3 | n.s. | n.s. | n.s. | n.s. | 4.24E-06 | 2.94 | 1.53E-19 | 3.52 | n.s. | n.s. | n.s. | n.s. |
| 7 | NM_005167 | PPM1J | -11.28 | 3 | 8.27E-06 | 1.63 | n.s. | n.s. | n.s. | n.s. | n.s. | n.s. | n.s. | n.s. | n.s. | n.s. |
| 8 | NM_004165 | RRAD | -10.96 | 3 | 3.45E-31 | 6.59 | 6.32E-18 | 3.75 | n.s. | n.s. | 1.51E-12 | 1.55 | 4.79E-14 | 3.33 | 2.66E-10 | 2.04 |
| 9 | NM_014176 | UBE2T | -10.70 | 3 | n.s. | n.s. | n.s. | n.s. | 0.00E+00 | -2.92 | 7.02E-21 | -4.39 | 1.09E-11 | -2.06 | 8.08E-08 | -1.50 |
| 10 | NM_000187 | HGD | -10.44 | 3 | 2.17E-11 | -1.68 | 6.41E-12 | -1.50 | 0.00E+00 | -3.15 | 0.00E+00 | -4.23 | 3.00E-37 | -2.37 | 1.05E-43 | -2.34 |
| 11 | NM_006579 | EBP | -10.32 | 3 | n.s. | n.s. | n.s. | n.s. | 2.42E-40 | -2.80 | 0.00E+00 | -3.16 | n.s. | n.s. | n.s. | n.s. |
| 12 | NM_003885 | KHSRP | -10.19 | 3 | n.s. | n.s. | 6.37E-14 | -2.00 | 2.21E-10 | -2.29 | 2.51E-06 | -2.23 | n.s. | n.s. | n.s. | n.s. |
| 13 | NM_001111 | ADAR | -9.46 | 3 | n.s. | n.s. | n.s. | n.s. | n.s. | n.s. | 5.61E-06 | -1.45 | n.s. | n.s. | 7.73E-07 | -1.33 |
| 14 | NM_001288 | CLIC1 | -9.00 | 4 | n.s. | n.s. | n.s. | n.s. | n.s. | n.s. | n.s. | n.s. | 7.10E-17 | -1.60 | 4.74E-06 | -1.29 |
| 15 | NM_004357 | CD151 | -8.96 | 3 | n.s. | n.s. | 9.50E-19 | -2.13 | n.s. | n.s. | n.s. | n.s. | n.s. | n.s. | n.s. | n.s. |

| N | Accession | Symbol | △* | Score** | CDDP 12h | | CDDP 24h | | C2-CER 12h | | C2-CER 24h | | CdCl₂ 12h | | CdCl₂ 24h | |
|---|---|---|---|---|---|---|---|---|---|---|---|---|---|---|---|---|
| | | | | | $p$ value | Fold change | $p$ value | Fold change | $p$ value | Fold change | $p$ value | Fold change | $p$ value | Fold change | $p$ value | Fold change |
| 16 | NM_145303 | OSTCL | -8.85 | 3 | n.s. | n.s. | n.s. | n.s. | 2.27E-10 | 2.25 | 3.29E-27 | 3.08 | n.s. | n.s. | n.s. | n.s. |
| 17 | NM_003347 | UBE2L3 | -8.70 | 3 | 1.70E-30 | -1.76 | n.s. | n.s. | 5.91E-14 | -1.48 | 3.57E-09 | -1.47 | n.s. | n.s. | n.s. | n.s. |
| 18 | NM_006858 | TMED1 | -7.51 | 3 | 1.50E-07 | 1.35 | n.s. | n.s. | n.s. | n.s. | n.s. | n.s. | n.s. | n.s. | n.s. | n.s. |
| 19 | NM_006379 | SEMA3C | -6.17 | 3 | 6.25E-14 | -1.56 | n.s. | n.s. | n.s. | n.s. | n.s. | n.s. | n.s. | n.s. | n.s. | n.s. |
| 20 | NM_001961 | EEF2 | 6.32 | 3 | 2.09E-06 | 1.36 | 1.58E-06 | -1.31 | 2.01E-19 | 2.31 | 0.00E+00 | 2.62 | n.s. | n.s. | n.s. | n.s. |
| 21 | NM_001182 | ALDH7A1 | 6.80 | 3 | n.s. | n.s. | 2.37E-27 | 1.62 | 2.25E-25 | -1.85 | 5.74E-36 | -1.99 | 1.43E-19 | -1.65 | 1.27E-30 | -1.82 |
| 22 | NM_003681 | PDXK | 7.08 | 3 | 3.17E-24 | -1.77 | 1.16E-06 | -1.44 | 2.66EE-17 | -1.89 | 4.06E-08 | -1.91 | n.s. | n.s. | n.s. | n.s. |
| 23 | NM_005336 | HDLBP | 9.15 | 3 | n.s. | n.s. | n.s. | n.s. | 2.84E-21 | 1.69 | 1.84E-12 | 1.99 | n.s. | n.s. | n.s. | n.s. |
| 24 | NM_007105 | SLC22A18AS | 8.89 | 3 | 1.08E-09 | -1.36 | 7.13E-06 | -1.34 | 1.03E-10 | -1.40 | 1.83E-18 | -1.60 | 1.69E-08 | -1.43 | 6.25E-09 | -1.53 |
| 25 | NM_024619 | FN3KRP | 9.76 | 3 | 5.54E-06 | 1.33 | 4.70E-06 | 1.25 | 9.02E-08 | -1.35 | 1.19E-23 | -1.67 | 1.86E-25 | -3.34 | 1.55E-11 | -2.05 |
| 26 | NM_145177 | DHRSX | 9.96 | 3 | 0.00E+00 | -4.94 | 0.00E+00 | -5.40 | n.s. | n.s. | n.s. | n.s. | 6.54E-15 | 1.64 | 2.62-06 | 1.38 |
| 27 | NM_001862 | COX5B | 10.51 | 3 | n.s. | n.s. | n.s. | n.s. | 7.50E-26 | -1.62 | 6.29E-15 | -1.61 | n.s. | n.s. | n.s. | n.s. |
| 28 | NM_001867 | COX7C | 10.85 | 4 | n.s. | n.s. | n.s. | n.s. | 8.32E-09 | -1.33 | n.s. | n.s. | n.s. | n.s. | n.s. | n.s. |
| 29 | NM_016447 | MPP8 | 10.89 | 3 | n.s. | n.s. | n.s. | n.s. | n.s. | n.s. | n.s. | n.s. | 7.68E-07 | -3.80 | n.s. | n.s. |
| 30 | NM_014186 | COMMD9 | 12.60 | 3 | n.s. | n.s. | n.s. | n.s. | n.s. | n.s. | n.s. | n.s. | 5.31E-06 | 1.95 | 9.44E-09 | 1.87 |

(continued)

| N | Accession | Symbol | △* | Score** | CDDP 12h | | CDDP 24h | | C2-CER 12h | | C2-CER 24h | | CdCl$_2$ 12h | | CdCl$_2$ 24h | |
|---|---|---|---|---|---|---|---|---|---|---|---|---|---|---|---|---|
| | | | | | $p$ value | Fold change | $p$ value | Fold change | $p$ value | Fold change | $p$ value | Fold change | $p$ value | Fold change | $p$ value | Fold change |
| 31 | NM_032015 | RNF28 | 14.55 | 3 | n.s. | n.s. | n.s. | n.s. | n.s. | n.s. | 9.51E-06 | -2.11 | 9.46E-08 | -1.77 | 3.45E-09 | -1.51 |
| 32 | NM_016213 | TRIP4 | 14.89 | 3 | 3.33E-06 | -1.64 | n.s. | n.s. | 2.02E-07 | 1.83 | 7.34E-07 | 1.65 | n.s. | n.s. | n.s. | n.s. |
| 33 | NM_002805 | PSMC5 | 16.29 | 3 | n.s. | n.s. | n.s. | n.s. | 3.11E-31 | -2.20 | 5.36E-32 | -2.27 | n.s. | n.s. | n.s. | n.s. |
| 34 | NM_004232 | SOCS8 | 17.10 | 3 | 4.41E-08 | -1.26 | n.s. | n.s. | n.s. | n.s. | n.s. | n.s. | n.s. | n.s. | n.s. | n.s. |

**Table 4: Effects of the 85 siRNA screen hits in other experimental models of cancer cell death.**

Table 4A (in two parts): The 85 siRNAs discovered in A549 cells were assayed for their capacity to modulate the response to cisplatin (CDDP) in six distinct CDDP-resistant A549 clones (A549 #1-6), four additional NSCLC cell lines (i.e., HCC827, H1299, H1650, H1975) and wild-type or TP53-/- HCT 116 colon cancer cells.

**Table 4A (part I)**

| | A549* | | A549 #1 | | A549 #2 | | A549 #3 | | A549 #4 | | A549 #5 | | A549 #6 | |
|---|---|---|---|---|---|---|---|---|---|---|---|---|---|---|
| | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) |
| MCL1 | -0,37 | 0,04 | -0,18 | 0,08 | -0,15 | 0,06 | -0,20 | 0,05 | -0,18 | 0,06 | -0,18 | 0,07 | -0,02 | 0,07 |
| TFF1 | -0,36 | 0,05 | -0,27 | 0,06 | -0,19 | 0,06 | -0,27 | 0,04 | -0,26 | 0,05 | -0,27 | 0,10 | -0,24 | 0,06 |
| BCL2L1 | -0,35 | 0,04 | -0,25 | 0,06 | -0,20 | 0,05 | -0,28 | 0,04 | -0,18 | 0,08 | -0,35 | 0,06 | -0,29 | 0,06 |
| RAB3C | -0,33 | 0,06 | -0,24 | 0,06 | -0,15 | 0,06 | -0,21 | 0,05 | -0,27 | 0,05 | -0,26 | 0,06 | -0,07 | 0,07 |
| PPM1J | -0,33 | 0,04 | -0,22 | 0,06 | -0,19 | 0,06 | -0,22 | 0,05 | -0,25 | 0,06 | -0,20 | 0,08 | -0,07 | 0,06 |
| CD151 | -0,32 | 0,12 | -0,05 | 0,06 | -0,09 | 0,07 | -0,10 | 0,05 | -0,13 | 0,05 | -0,19 | 0,06 | -0,08 | 0,07 |
| BMP7 | -0,30 | 0,04 | -0,13 | 0,05 | -0,10 | 0,06 | -0,17 | 0,05 | -0,11 | 0,09 | -0,11 | 0,10 | 0,02 | 0,06 |
| RAC2 | -0,30 | 0,04 | -0,25 | 0,06 | -0,13 | 0,06 | -0,19 | 0,04 | -0,29 | 0,05 | -0,27 | 0,06 | -0,17 | 0,05 |
| WSB2 | -0,30 | 0,05 | -0,25 | 0,11 | -0,22 | 0,06 | -0,28 | 0,04 | -0,28 | 0,05 | -0,26 | 0,06 | -0,21 | 0,06 |
| LOC390533 | -0,29 | 0,09 | -0,12 | 0,07 | -0,14 | 0,06 | -0,16 | 0,05 | -0,16 | 0,06 | -0,13 | 0,07 | -0,11 | 0,07 |
| CYP11B2 | -0,28 | 0,05 | -0,19 | 0,07 | -0,13 | 0,05 | -0,08 | 0,06 | -0,11 | 0,05 | -0,16 | 0,07 | 0,05 | 0,08 |
| RRAD | -0,28 | 0,05 | -0,16 | 0,09 | -0,11 | 0,07 | -0,17 | 0,06 | -0,22 | 0,05 | -0,27 | 0,07 | -0,06 | 0,07 |
| MAT2A | -0,27 | 0,05 | -0,11 | 0,09 | -0,03 | 0,07 | -0,11 | 0,05 | -0,08 | 0,07 | -0,06 | 0,11 | 0,14 | 0,08 |
| USP8 | -0,27 | 0,04 | -0,24 | 0,05 | -0,20 | 0,06 | -0,01 | 0,07 | 0,20 | 0,11 | 0,03 | 0,14 | -0,01 | 0,08 |
| ITGB6 | -0,27 | 0,05 | -0,09 | 0,08 | -0,08 | 0,07 | -0,10 | 0,08 | -0,06 | 0,07 | -0,21 | 0,07 | 0,02 | 0,06 |
| DEFB4 | -0,27 | 0,04 | -0,19 | 0,06 | -0,10 | 0,06 | -0,02 | 0,09 | 0,07 | 0,05 | -0,03 | 0,13 | 0,06 | 0,08 |
| NCKAP 1 | -0,27 | 0,05 | -0,16 | 0,06 | -0,11 | 0,06 | -0,09 | 0,05 | -0,06 | 0,05 | -0,19 | 0,06 | -0,16 | 0,06 |
| IAPP | -0,26 | 0,04 | -0,09 | 0,09 | -0,07 | 0,06 | -0,15 | 0,05 | -0,20 | 0,05 | -0,26 | 0,06 | 0,05 | 0,10 |
| IL6R | -0,25 | 0,04 | -0,11 | 0,06 | -0,06 | 0,06 | -0,09 | 0,06 | 0,08 | 0,08 | -0,05 | 0,10 | 0,08 | 0,07 |
| PLK1S1 | -0,25 | 0,05 | -0,23 | 0,08 | -0,18 | 0,06 | -0,21 | 0,05 | -0,30 | 0,05 | -0,22 | 0,08 | -0,12 | 0,06 |
| BTNL2 | -0,25 | 0,05 | -0,15 | 0,07 | -0,08 | 0,09 | -0,16 | 0,06 | -0,13 | 0,09 | -0,22 | 0,06 | -0,02 | 0,06 |
| FLOT2 | -0,25 | 0,05 | -0,15 | 0,08 | -0,06 | 0,06 | -0,05 | 0,06 | 0,00 | 0,06 | -0,11 | 0,09 | 0,00 | 0,06 |

| | A549* | | A549 #1 | | A549 #2 | | A549 #3 | | A549 #4 | | A549 #5 | | A549 #6 | |
|---|---|---|---|---|---|---|---|---|---|---|---|---|---|---|
| | Δ**(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| SEMG2 | -0,24 | 0,05 | -0,10 | 0,07 | -0,06 | 0,07 | -0,13 | 0,05 | -0,22 | 0,05 | -0,31 | 0,06 | 0,08 | 0,08 |
| BSN | -0,24 | 0,05 | -0,27 | 0,08 | -0,10 | 0,06 | -0,19 | 0,03 | -0,18 | 0,08 | -0,33 | 0,07 | -0,02 | 0,05 |
| PLD3 | -0,24 | 0,05 | -0,15 | 0,06 | -0,12 | 0,06 | -0,11 | 0,05 | 0,01 | 0,10 | -0,12 | 0,08 | -0,11 | 0,07 |
| PRSS21 | -0,24 | 0,04 | -0,23 | 0,06 | -0,13 | 0,06 | -0,11 | 0,07 | -0,07 | 0,07 | -0,01 | 0,22 | -0,09 | 0,09 |
| ADAR | -0,24 | 0,04 | -0,15 | 0,08 | -0,04 | 0,06 | -0,07 | 0,06 | -0,01 | 0,07 | -0,14 | 0,10 | -0,05 | 0,06 |
| HSPC150 | -0,24 | 0,05 | -0,12 | 0,06 | -0,05 | 0,07 | -0,03 | 0,06 | -0,05 | 0,06 | -0,11 | 0,06 | -0,09 | 0,06 |
| HGD | -0,24 | 0,05 | -0,21 | 0,07 | -0,15 | 0,07 | -0,18 | 0,05 | -0,07 | 0,09 | -0,28 | 0,07 | -0,19 | 0,07 |
| CLIC1 | -0,23 | 0,04 | -0,14 | 0,09 | -0,07 | 0,08 | -0,20 | 0,04 | -0,23 | 0,06 | -0,17 | 0,07 | -0,12 | 0,09 |
| NALP1 | -0,22 | 0,04 | -0,14 | 0,10 | -0,09 | 0,05 | -0,13 | 0,05 | -0,12 | 0,07 | -0,04 | 0,08 | 0,07 | 0,06 |
| LRMP | -0,22 | 0,05 | -0,09 | 0,07 | -0,07 | 0,07 | -0,15 | 0,05 | -0,16 | 0,05 | -0,09 | 0,11 | 0,00 | 0,06 |
| C1ORF91 | -0,22 | 0,05 | -0,24 | 0,06 | -0,09 | 0,06 | -0,22 | 0,05 | -0,27 | 0,05 | -0,30 | 0,06 | -0,06 | 0,06 |
| LOC389727 | -0,21 | 0,05 | -0,05 | 0,08 | -0,06 | 0,06 | -0,04 | 0,05 | -0,02 | 0,06 | -0,15 | 0,09 | -0,02 | 0,07 |
| ACHE | -0,21 | 0,05 | -0,18 | 0,08 | -0,05 | 0,08 | -0,12 | 0,07 | -0,17 | 0,06 | -0,08 | 0,11 | -0,03 | 0,07 |
| BAIAP3 | -0,21 | 0,06 | -0,09 | 0,10 | 0,01 | 0,06 | -0,09 | 0,06 | -0,05 | 0,10 | -0,08 | 0,10 | -0,06 | 0,04 |
| HIRIP3 | -0,21 | 0,05 | -0,11 | 0,07 | -0,10 | 0,06 | -0,17 | 0,05 | -0,18 | 0,07 | -0,21 | 0,08 | 0,03 | 0,07 |
| PPM1B | -0,21 | 0,06 | -0,24 | 0,09 | -0,12 | 0,05 | -0,18 | 0,05 | -0,21 | 0,07 | -0,27 | 0,09 | -0,01 | 0,07 |
| KHSRP | -0,21 | 0,05 | -0,19 | 0,07 | -0,16 | 0,06 | -0,18 | 0,06 | -0,19 | 0,08 | -0,27 | 0,07 | 0,00 | 0,06 |
| ZDHHC9 | -0,20 | 0,05 | -0,12 | 0,08 | -0,11 | 0,06 | -0,21 | 0,05 | -0,28 | 0,05 | -0,34 | 0,06 | -0,04 | 0,08 |
| CD34 | -0,20 | 0,05 | -0,17 | 0,07 | -0,07 | 0,06 | -0,14 | 0,05 | -0,20 | 0,05 | -0,24 | 0,07 | -0,02 | 0,06 |
| TMED1 | -0,19 | 0,04 | -0,21 | 0,05 | -0,13 | 0,06 | -0,04 | 0,05 | -0,15 | 0,08 | -0,11 | 0,07 | -0,09 | 0,06 |
| NCOA3 | -0,19 | 0,04 | 0,00 | 0,09 | -0,10 | 0,08 | -0,13 | 0,05 | -0,05 | 0,06 | -0,19 | 0,06 | -0,03 | 0,09 |

EP 2 426 216 A1

(continued)

| | A549* | | A549 #1 | | A549 #2 | | A549 #3 | | A549 #4 | | A549 #5 | | A549 #6 | |
|---|---|---|---|---|---|---|---|---|---|---|---|---|---|---|
| | Δ**(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| TCTE3 | -0,19 | 0,05 | -0,07 | 0,10 | -0,04 | 0,07 | -0,11 | 0,06 | -0,03 | 0,05 | -0,20 | 0,09 | 0,09 | 0,07 |
| C10orf9 2 | -0,18 | 0,09 | -0,14 | 0,06 | -0,08 | 0,09 | -0,19 | 0,05 | -0,10 | 0,07 | -0,20 | 0,10 | -0,16 | 0,07 |
| UBE2L 3 | -0,17 | 0,04 | -0,21 | 0,06 | -0,10 | 0,06 | -0,12 | 0,05 | -0,02 | 0,07 | -0,10 | 0,07 | -0,07 | 0,07 |
| NANOS 1 | -0,17 | 0,06 | -0,03 | 0,07 | -0,02 | 0,06 | -0,02 | 0,06 | -0,14 | 0,05 | -0,04 | 0,07 | -0,06 | 0,08 |
| LIPC | -0,16 | 0,05 | -0,12 | 0,07 | -0,12 | 0,06 | -0,18 | 0,05 | -0,22 | 0,05 | -0,08 | 0,09 | -0,11 | 0,06 |
| OSTCL | -0,16 | 0,07 | 0,03 | 0,07 | 0,03 | 0,07 | 0,04 | 0,06 | 0,05 | 0,07 | -0,06 | 0,07 | 0,01 | 0,06 |
| GUCA1 A | -0,14 | 0,07 | -0,11 | 0,09 | 0,01 | 0,10 | -0,02 | 0,05 | 0,05 | 0,07 | -0,02 | 0,08 | 0,01 | 0,05 |
| EBP | -0,13 | 0,06 | -0,13 | 0,06 | -0,11 | 0,06 | -0,08 | 0,05 | -0,07 | 0,07 | -0,04 | 0,12 | -0,12 | 0,06 |
| SEMA3 C | -0,11 | 0,06 | -0,18 | 0,08 | -0,07 | 0,06 | -0,03 | 0,04 | -0,17 | 0,07 | -0,28 | 0,08 | 0,01 | 0,05 |
| KIAA11 61 | -0,02 | 0,06 | 0,21 | 0,12 | 0,11 | 0,07 | 0,09 | 0,06 | -0,11 | 0,06 | -0,01 | 0,06 | 0,05 | 0,07 |
| ALK | 0,01 | 0,05 | 0,15 | 0,09 | 0,14 | 0,05 | 0,11 | 0,05 | 0,09 | 0,09 | 0,10 | 0,10 | 0,27 | 0,07 |
| EEF2 | 0,02 | 0,06 | 0,13 | 0,10 | 0,26 | 0,20 | 0,34 | 0,11 | 0,32 | 0,09 | 0,24 | 0,09 | 0,14 | 0,06 |
| COX7C | 0,05 | 0,06 | 0,10 | 0,13 | 0,07 | 0,05 | 0,01 | 0,06 | -0,05 | 0,08 | -0,11 | 0,09 | 0,18 | 0,05 |
| C21orf2 | 0,06 | 0,06 | 0,24 | 0,09 | 0,19 | 0,05 | 0,15 | 0,06 | 0,22 | 0,11 | 0,03 | 0,13 | 0,12 | 0,06 |
| RPSAP3 2 | 0,07 | 0,07 | 0,10 | 0,08 | -0,03 | 0,05 | 0,02 | 0,05 | 0,15 | 0,10 | -0,09 | 0,08 | 0,01 | 0,03 |
| PDXK | 0,07 | 0,07 | 0,16 | 0,12 | 0,17 | 0,09 | 0,21 | 0,05 | 0,22 | 0,08 | 0,05 | 0,08 | 0,17 | 0,05 |
| MPP6 | 0,08 | 0,08 | 0,09 | 0,14 | 0,09 | 0,05 | 0,09 | 0,07 | 0,18 | 0,09 | 0,08 | 0,10 | 0,08 | 0,04 |
| RAB40 A | 0,09 | 0,07 | 0,33 | 0,10 | 0,31 | 0,08 | 0,26 | 0,06 | 0,45 | 0,07 | 0,31 | 0,12 | 0,22 | 0,05 |
| OVCH1 | 0,11 | 0,05 | 0,27 | 0,12 | 0,26 | 0,09 | 0,16 | 0,05 | 0,20 | 0,10 | 0,12 | 0,09 | 0,16 | 0,06 |

|  | A549* | | A549 #1 | | A549 #2 | | A549 #3 | | A549 #4 | | A549 #5 | | A549 #6 | |
|---|---|---|---|---|---|---|---|---|---|---|---|---|---|---|
|  | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) |
| GRLF1 | 0,11 | 0,07 | 0,13 | 0,10 | 0,09 | 0,10 | 0,16 | 0,08 | 0,15 | 0,09 | -0,04 | 0,12 | 0,10 | 0,07 |
| SLC22A 1LS | 0,11 | 0,08 | 0,32 | 0,13 | 0,19 | 0,07 | 0,30 | 0,08 | 0,56 | 0,17 | 0,35 | 0,13 | 0,23 | 0,03 |
| LOC442 126 | 0,12 | 0,07 | 0,24 | 0,18 | 0,29 | 0,16 | 0,30 | 0,07 | 0,44 | 0,13 | 0,39 | 0,12 | 0,23 | 0,06 |
| DHRSX | 0,13 | 0,07 | 0,27 | 0,11 | 0,23 | 0,06 | 0,22 | 0,05 | 0,32 | 0,07 | 0,23 | 0,11 | 0,18 | 0,04 |
| LOC440 056 | 0,14 | 0,06 | 0,39 | 0,13 | 0,33 | 0,09 | 0,26 | 0,06 | 0,37 | 0,08 | 0,34 | 0,10 | 0,27 | 0,04 |
| FBX04 2 | 0,14 | 0,06 | -0,01 | 0,12 | 0,13 | 0,06 | 0,14 | 0,06 | 0,19 | 0,09 | -0,03 | 0,10 | 0,03 | 0,08 |
| ALDH7 A1 | 0,14 | 0,06 | 0,24 | 0,19 | 0,31 | 0,06 | 0,50 | 0,06 | 0,58 | 0,08 | 0,49 | 0,12 | 0,20 | 0,06 |
| LOC440 611 | 0,14 | 0,06 | 0,18 | 0,17 | 0,20 | 0,06 | 0,23 | 0,06 | 0,20 | 0,10 | 0,32 | 0,13 | 0,05 | 0,06 |
| HDLBP | 0,15 | 0,06 | 0,27 | 0,17 | 0,24 | 0,08 | 0,27 | 0,12 | 0,42 | 0,08 | 0,34 | 0,12 | 0,17 | 0,05 |
| LOC441 115 | 0,16 | 0,07 | 0,19 | 0,17 | 0,32 | 0,08 | 0,15 | 0,05 | 0,15 | 0,09 | 0,08 | 0,09 | 0,07 | 0,07 |
| ASTL | 0,17 | 0,07 | 0,13 | 0,08 | 0,17 | 0,10 | 0,29 | 0,09 | 0,42 | 0,09 | 0,06 | 0,08 | 0,18 | 0,04 |
| FN3KR P | 0,17 | 0,07 | 0,40 | 0,09 | 0,21 | 0,08 | 0,27 | 0,09 | 0,29 | 0,10 | 0,31 | 0,14 | 0,25 | 0,10 |
| RNF26 | 0,18 | 0,07 | 0,24 | 0,11 | 0,16 | 0,07 | 0,28 | 0,07 | 0,49 | 0,13 | 0,40 | 0,11 | 0,13 | 0,07 |
| PSMC5 | 0,18 | 0,09 | 0,22 | 0,12 | 0,19 | 0,10 | 0,38 | 0,05 | 0,53 | 0,13 | 0,39 | 0,10 | 0,33 | 0,08 |
| TRIP4 | 0,20 | 0,07 | 0,32 | 0,13 | 0,30 | 0,13 | 0,36 | 0,07 | 0,41 | 0,08 | 0,42 | 0,08 | 0,29 | 0,06 |
| SOCS6 | 0,21 | 0,08 | 0,31 | 0,23 | 0,27 | 0,08 | 0,14 | 0,09 | 0,25 | 0,15 | -0,02 | 0,08 | 0,17 | 0,06 |
| PSMD7 | 0,22 | 0,05 | 0,23 | 0,16 | 0,39 | 0,07 | 0,25 | 0,08 | 0,49 | 0,12 | 0,22 | 0,08 | 0,33 | 0,17 |
| COX5B | 0,23 | 0,05 | 0,07 | 0,12 | 0,11 | 0,04 | 0,17 | 0,05 | 0,31 | 0,07 | 0,05 | 0,09 | 0,10 | 0,05 |

(continued)

| | A549* | | A549 #1 | | A549 #2 | | A549 #3 | | A549 #4 | | A549 #5 | | A549 #6 | |
|---|---|---|---|---|---|---|---|---|---|---|---|---|---|---|
| | Δ** (Mean) | Δ(SEM) | Δ (Mean) | Δ(SEM) | Δ (Mean) | Δ(SEM) | Δ(Mean) | Δ(SEM) | Δ(Mean) | Δ(SEM) | Δ(Mean) | Δ(SEM) | Δ(Mean) | Δ(SEM) |
| SLC39A5 | 0,23 | 0,07 | 0,38 | 0,14 | 0,45 | 0,16 | 0,45 | 0,12 | 0,52 | 0,09 | 0,60 | 0,11 | 0,29 | 0,06 |
| ZNF878 | 0,25 | 0,08 | 0,17 | 0,13 | 0,16 | 0,08 | 0,30 | 0,09 | 0,37 | 0,12 | 0,10 | 0,11 | 0,19 | 0,04 |
| APAF12 | 0,25 | 0,07 | 0,07 | 0,11 | 0,20 | 0,06 | 0,24 | 0,05 | 0,20 | 0,10 | 0,09 | 0,09 | 0,04 | 0,06 |
| LOC388882 | 0,26 | 0,06 | 0,32 | 0,15 | 0,37 | 0,12 | 0,37 | 0,08 | 0,53 | 0,09 | 0,23 | 0,14 | 0,26 | 0,07 |
| COMMD9 | 0,35 | 0,09 | 0,32 | 0,11 | 0,33 | 0,08 | 0,54 | 0,09 | 0,66 | 0,13 | 0,48 | 0,14 | 0,35 | 0,05 |

**Table 4A (part II)**

| | H1299 | | H1650 | | H1975 | | HCC827 | | HCT 116 | | p53-/- HCT 116 | |
|---|---|---|---|---|---|---|---|---|---|---|---|---|
| | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) |
| MCL1 | -0,13 | 0,08 | 0,00 | 0,12 | -0,06 | 0,07 | -0,05 | 0,13 | -0,10 | 0,06 | -0,12 | 0,10 |
| TFF1 | -0,40 | 0,06 | -0,13 | 0,11 | -0,27 | 0,07 | -0,18 | 0,10 | 0,31 | 0,06 | 0,09 | 0,10 |
| BCL2L1 | -0,23 | 0,06 | -0,14 | 0,09 | -0,08 | 0,07 | -0,31 | 0,11 | 0,04 | 0,05 | -0,05 | 0,10 |
| RAB3C | -0,14 | 0,08 | -0,11 | 0,07 | -0,19 | 0,08 | -0,08 | 0,10 | 0,01 | 0,06 | -0,12 | 0,09 |
| PPM1J | -0,23 | 0,06 | -0,14 | 0,11 | -0,23 | 0,06 | -0,09 | 0,11 | 0,17 | 0,06 | 0,00 | 0,11 |
| CD151 | 0,00 | 0,09 | -0,05 | 0,09 | -0,20 | 0,07 | -0,11 | 0,13 | 0,20 | 0,09 | 0,19 | 0,12 |
| BMP7 | -0,11 | 0,07 | -0,10 | 0,12 | -0,02 | 0,07 | -0,13 | 0,10 | -0,07 | 0,05 | 0,00 | 0,11 |
| RAC2 | -0,25 | 0,10 | -0,14 | 0,06 | -0,17 | 0,08 | -0,18 | 0,10 | 0,11 | 0,04 | 0,03 | 0,10 |
| WSB2 | -0,21 | 0,06 | -0,10 | 0,07 | -0,18 | 0,08 | 0,03 | 0,12 | 0,14 | 0,05 | 0,03 | 0,11 |
| LOC390533 | -0,28 | 0,07 | -0,13 | 0,07 | -0,08 | 0,06 | -0,14 | 0,09 | 0,13 | 0,06 | -0,10 | 0,13 |
| CYP11B2 | -0,19 | 0,06 | -0,12 | 0,08 | -0,10 | 0,09 | 0,07 | 0,11 | 0,05 | 0,05 | -0,02 | 0,13 |
| RRAD | -0,28 | 0,06 | 0,03 | 0,07 | 0,00 | 0,13 | 0,04 | 0,09 | 0,18 | 0,07 | 0,12 | 0,08 |
| MAT2A | -0,06 | 0,06 | 0,00 | 0,13 | -0,11 | 0,10 | 0,21 | 0,12 | 0,30 | 0,05 | 0,31 | 0,10 |
| USP8 | -0,15 | 0,06 | -0,05 | 0,10 | -0,16 | 0,12 | 0,01 | 0,13 | 0,17 | 0,07 | -0,06 | 0,08 |
| ITGB6 | 0,04 | 0,07 | 0,19 | 0,08 | -0,18 | 0,11 | 0,18 | 0,12 | 0,45 | 0,08 | 0,45 | 0,09 |
| DEFB4 | -0,30 | 0,05 | -0,03 | 0,07 | -0,07 | 0,07 | 0,02 | 0,09 | 0,16 | 0,10 | 0,09 | 0,15 |
| NCKAP1 | -0,17 | 0,06 | -0,02 | 0,09 | -0,04 | 0,10 | 0,10 | 0,09 | 0,32 | 0,08 | 0,22 | 0,13 |
| IAPP | -0,18 | 0,06 | 0,05 | 0,09 | -0,08 | 0,08 | -0,07 | 0,10 | 0,14 | 0,08 | 0,14 | 0,11 |
| IL6R | -0,08 | 0,06 | 0,06 | 0,13 | -0,13 | 0,07 | -0,03 | 0,15 | 0,03 | 0,04 | 0,12 | 0,12 |
| PLK1S1 | -0,26 | 0,06 | -0,07 | 0,06 | -0,17 | 0,06 | -0,11 | 0,09 | 0,06 | 0,05 | 0,04 | 0,09 |
| BTNL2 | -0,27 | 0,06 | 0,01 | 0,10 | -0,08 | 0,08 | -0,03 | 0,12 | -0,01 | 0,09 | 0,07 | 0,11 |
| FLOT2 | -0,13 | 0,06 | -0,03 | 0,12 | -0,15 | 0,07 | -0,01 | 0,09 | 0,07 | 0,04 | 0,07 | 0,12 |
| SEMG2 | -0,26 | 0,07 | -0,10 | 0,06 | -0,16 | 0,07 | -0,08 | 0,09 | -0,13 | 0,04 | -0,14 | 0,11 |
| BSN | -0,32 | 0,05 | -0,16 | 0,06 | -0,13 | 0,06 | -0,14 | 0,07 | -0,09 | 0,04 | -0,14 | 0,10 |

(continued)

| | H1299 | | H1650 | | H1975 | | HCC827 | | HCT 116 | | p53-/- HCT 116 | |
|---|---|---|---|---|---|---|---|---|---|---|---|---|
| | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) |
| PLD3 | -0,14 | 0,08 | 0,02 | 0,07 | -0,09 | 0,10 | 0,07 | 0,16 | 0,07 | 0,08 | 0,05 | 0,11 |
| PRSS21 | -0,38 | 0,06 | -0,13 | 0,06 | -0,04 | 0,09 | -0,11 | 0,10 | 0,36 | 0,07 | 0,21 | 0,12 |
| ADAR | 0,01 | 0,08 | -0,01 | 0,11 | -0,13 | 0,07 | -0,05 | 0,14 | 0,12 | 0,09 | 0,08 | 0,09 |
| HSPC150 | -0,08 | 0,06 | -0,01 | 0,08 | -0,14 | 0,07 | -0,06 | 0,10 | 0,19 | 0,05 | 0,07 | 0,12 |
| HGD | -0,31 | 0,06 | 0,03 | 0,06 | -0,21 | 0,10 | -0,06 | 0,13 | 0,23 | 0,10 | 0,12 | 0,14 |
| CLIC1 | -0,29 | 0,07 | -0,11 | 0,08 | -0,09 | 0,08 | -0,09 | 0,10 | 0,15 | 0,03 | 0,06 | 0,09 |
| NALP1 | -0,16 | 0,06 | 0,00 | 0,09 | -0,05 | 0,08 | -0,08 | 0,10 | 0,06 | 0,06 | 0,13 | 0,11 |
| LRMP | -0,13 | 0,06 | -0,03 | 0,11 | -0,02 | 0,07 | -0,03 | 0,11 | 0,05 | 0,07 | 0,08 | 0,16 |
| C10RF91 | 0,06 | 0,06 | 0,02 | 0,06 | -0,15 | 0,06 | 0,04 | 0,09 | 0,08 | 0,05 | -0,09 | 0,08 |
| LOC389727 | 0,02 | 0,09 | 0,00 | 0,09 | -0,13 | 0,09 | -0,03 | 0,13 | 0,28 | 0,09 | 0,20 | 0,12 |
| ACHE | -0,12 | 0,06 | -0,05 | 0,08 | -0,16 | 0,09 | 0,16 | 0,09 | -0,02 | 0,07 | -0,02 | 0,08 |
| BAIAP3 | -0,14 | 0,09 | -0,05 | 0,07 | -0,08 | 0,07 | 0,20 | 0,09 | 0,32 | 0,03 | 0,36 | 0,15 |
| HIRIP3 | -0,15 | 0,06 | -0,09 | 0,08 | -0,17 | 0,07 | -0,09 | 0,11 | -0,01 | 0,04 | 0,03 | 0,11 |
| PPM1B | -0,28 | 0,06 | -0,12 | 0,07 | -0,01 | 0,05 | 0,00 | 0,08 | 0,06 | 0,04 | 0,19 | 0,13 |
| KHSRP | -0,31 | 0,07 | -0,11 | 0,06 | -0,17 | 0,07 | -0,07 | 0,11 | 0,09 | 0,06 | 0,03 | 0,09 |
| ZDHHC9 | -0,25 | 0,07 | -0,05 | 0,11 | -0,19 | 0,06 | -0,03 | 0,12 | 0,16 | 0,06 | 0,10 | 0,09 |
| CD34 | -0,34 | 0,06 | 0,04 | 0,13 | -0,07 | 0,10 | -0,04 | 0,10 | -0,13 | 0,04 | -0,10 | 0,08 |
| TMED1 | -0,19 | 0,07 | -0,02 | 0,09 | -0,12 | 0,10 | -0,03 | 0,09 | 0,14 | 0,04 | 0,04 | 0,16 |
| NCOA3 | -0,15 | 0,08 | -0,07 | 0,07 | -0,10 | 0,06 | 0,00 | 0,16 | 0,18 | 0,07 | 0,08 | 0,09 |
| TCTE3 | -0,15 | 0,07 | 0,00 | 0,09 | -0,14 | 0,08 | -0,04 | 0,09 | 0,24 | 0,05 | 0,19 | 0,12 |
| C10orf92 | -0,17 | 0,08 | 0,07 | 0,09 | -0,07 | 0,07 | 0,12 | 0,13 | 0,37 | 0,06 | 0,28 | 0,18 |
| UBE2L3 | -0,17 | 0,06 | -0,03 | 0,06 | -0,10 | 0,11 | -0,03 | 0,10 | -0,05 | 0,05 | -0,05 | 0,10 |
| NANOS1 | -0,41 | 0,06 | -0,12 | 0,07 | -0,02 | 0,11 | -0,04 | 0,13 | -0,02 | 0,03 | -0,20 | 0,10 |
| LIPC | -0,38 | 0,06 | -0,05 | 0,10 | -0,08 | 0,08 | -0,09 | 0,09 | 0,12 | 0,05 | 0,02 | 0,09 |

| | H1299 | | H1650 | | H1975 | | HCC827 | | HCT 116 | | p53$^{-/-}$ HCT 116 | |
|---|---|---|---|---|---|---|---|---|---|---|---|---|
| | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| OSTCL | 0,00 | 0,09 | 0,05 | 0,07 | -0,09 | 0,07 | 0,13 | 0,11 | 0,02 | 0,05 | 0,02 | 0,09 |
| GUCA1A | -0,03 | 0,06 | -0,09 | 0,06 | -0,22 | 0,06 | 0,01 | 0,07 | 0,24 | 0,07 | 0,22 | 0,12 |
| EBP | 0,02 | 0,08 | -0,10 | 0,07 | -0,13 | 0,08 | -0,13 | 0,09 | 0,15 | 0,06 | 0,10 | 0,11 |
| SEMA3C | -0,15 | 0,05 | -0,11 | 0,06 | 0,03 | 0,05 | 0,01 | 0,08 | -0,01 | 0,05 | -0,04 | 0,07 |
| KIAA 1161 | 0,10 | 0,12 | 0,15 | 0,14 | -0,02 | 0,07 | 0,23 | 0,09 | 0,13 | 0,04 | 0,03 | 0,10 |
| ALK | 0,06 | 0,09 | 0,06 | 0,06 | 0,08 | 0,05 | 0,11 | 0,08 | 0,41 | 0,12 | 0,28 | 0,13 |
| EEF2 | 0,06 | 0,08 | -0,08 | 0,06 | 0,18 | 0,14 | 0,14 | 0,10 | 0,41 | 0,10 | 0,28 | 0,13 |
| COX7C | -0,07 | 0,07 | -0,04 | 0,09 | 0,00 | 0,08 | 0,02 | 0,08 | 0,24 | 0,03 | 0,13 | 0,07 |
| C21orf2 | 0,02 | 0,05 | 0,06 | 0,06 | -0,02 | 0,06 | 0,06 | 0,09 | 0,35 | 0,08 | 0,40 | 0,08 |
| RPSAP32 | 0,05 | 0,08 | -0,07 | 0,07 | -0,15 | 0,08 | 0,03 | 0,08 | 0,32 | 0,11 | 0,11 | 0,09 |
| PDXK | 0,10 | 0,06 | 0,07 | 0,07 | 0,00 | 0,07 | 0,04 | 0,08 | 0,39 | 0,06 | 0,29 | 0,15 |
| MPP6 | 0,08 | 0,06 | -0,09 | 0,07 | 0,08 | 0,09 | -0,04 | 0,10 | 0,11 | 0,06 | 0,24 | 0,15 |
| RAB40A | 0,16 | 0,06 | 0,05 | 0,09 | 0,18 | 0,07 | 0,09 | 0,06 | 0,28 | 0,07 | 0,15 | 0,11 |
| OVCH1 | 0,19 | 0,09 | 0,09 | 0,06 | 0,18 | 0,08 | 0,02 | 0,10 | 0,15 | 0,09 | 0,19 | 0,09 |
| GRLF1 | 0,06 | 0,07 | -0,02 | 0,06 | 0,17 | 0,10 | 0,02 | 0,06 | 0,36 | 0,06 | 0,14 | 0,10 |
| SLC22A1LS | 0,24 | 0,06 | 0,05 | 0,08 | 0,06 | 0,07 | 0,30 | 0,09 | 0,33 | 0,05 | 0,44 | 0,13 |
| LOC442126 | 0,14 | 0,07 | 0,10 | 0,06 | 0,04 | 0,08 | 0,05 | 0,12 | 0,25 | 0,08 | 0,40 | 0,10 |
| DHRSX | 0,09 | 0,09 | -0,02 | 0,06 | 0,08 | 0,06 | 0,00 | 0,08 | 0,08 | 0,05 | 0,07 | 0,07 |
| LOC440056 | 0,23 | 0,14 | 0,05 | 0,09 | -0,02 | 0,09 | 0,08 | 0,10 | 0,34 | 0,14 | 0,37 | 0,10 |
| FBX042 | 0,24 | 0,08 | 0,05 | 0,07 | 0,12 | 0,08 | 0,09 | 0,09 | 0,16 | 0,04 | 0,22 | 0,14 |
| ALDH7A1 | 0,15 | 0,08 | -0,01 | 0,07 | 0,03 | 0,06 | 0,13 | 0,09 | 0,26 | 0,09 | 0,13 | 0,09 |
| LOC440611 | 0,17 | 0,08 | -0,01 | 0,06 | 0,03 | 0,06 | 0,00 | 0,10 | 0,06 | 0,04 | -0,03 | 0,08 |
| HDLBP | 0,19 | 0,06 | 0,02 | 0,08 | 0,04 | 0,05 | 0,04 | 0,08 | 0,12 | 0,07 | 0,26 | 0,11 |
| LOC441115 | 0,24 | 0,08 | 0,01 | 0,06 | 0,15 | 0,10 | 0,02 | 0,09 | 0,31 | 0,05 | 0,12 | 0,09 |

EP 2 426 216 A1

47

| | H1299 | | H1650 | | H1975 | | HCC827 | | HCT 116 | | p53-/- HCT 116 | |
|---|---|---|---|---|---|---|---|---|---|---|---|---|
| | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| ASTL | 0,07 | 0,08 | 0,02 | 0,06 | 0,02 | 0,11 | 0,16 | 0,13 | 0,28 | 0,09 | 0,38 | 0,12 |
| FN3KRP | 0,09 | 0,06 | -0,05 | 0,07 | 0,06 | 0,06 | -0,04 | 0,12 | 0,13 | 0,06 | 0,08 | 0,09 |
| RNF26 | 0,33 | 0,10 | 0,05 | 0,07 | 0,03 | 0,10 | 0,05 | 0,06 | 0,21 | 0,04 | 0,28 | 0,15 |
| PSMC5 | -0,03 | 0,07 | -0,06 | 0,11 | -0,02 | 0,06 | 0,17 | 0,15 | 0,39 | 0,06 | 0,11 | 0,10 |
| TRIP4 | 0,31 | 0,13 | -0,02 | 0,07 | 0,12 | 0,08 | 0,02 | 0,14 | 0,44 | 0,15 | 0,42 | 0,10 |
| SOCS6 | -0,02 | 0,07 | -0,02 | 0,07 | 0,24 | 0,12 | 0,03 | 0,07 | 0,09 | 0,03 | 0,12 | 0,06 |
| PSMD7 | 0,10 | 0,11 | -0,01 | 0,08 | -0,16 | 0,05 | 0,23 | 0,10 | 0,28 | 0,05 | 0,21 | 0,10 |
| COX5B | 0,23 | 0,07 | 0,00 | 0,09 | -0,07 | 0,08 | 0,11 | 0,13 | 0,14 | 0,07 | 0,39 | 0,11 |
| SLC39A5 | 0,14 | 0,05 | -0,04 | 0,07 | 0,12 | 0,09 | 0,12 | 0,13 | 0,42 | 0,16 | 0,42 | 0,13 |
| ZNF878 | 0,16 | 0,10 | 0,10 | 0,08 | 0,12 | 0,08 | 0,17 | 0,17 | 0,27 | 0,06 | 0,19 | 0,11 |
| APAF12 | 0,26 | 0,09 | 0,13 | 0,08 | 0,29 | 0,06 | 0,03 | 0,09 | 0,11 | 0,05 | -0,12 | 0,07 |
| LOC388882 | 0,19 | 0,09 | 0,01 | 0,07 | 0,18 | 0,07 | 0,09 | 0,12 | 0,32 | 0,12 | 0,27 | 0,10 |
| COMMD9 | 0,33 | 0,09 | 0,03 | 0,07 | 0,12 | 0,10 | 0,10 | 0,10 | 0,37 | 0,11 | 0,32 | 0,13 |

\* = reference profile

\*\* = upon background subtraction and inter-plate normalization, Δ = relative survival of siRNA-transfected cells (WST-1 signal from CDDP treated, siRNA-transfected cells / WST-1 signal from untreated, siRNA-transfected cells) - relative survival of cells transfected with a control siRNA (siUNR) (WST-1 signal from CDDP treated, siUNR-transfected cells / WST-1 signal from untreated, siUNR-transfected cells). Negative and positive Δ values signify chemosensitization and cytoprotection, respectively.

SEM = standard error of the mean

Table 4B (in two parts): Alternatively, the siRNA screen hits were evaluated for their ability to modify the response of A549 to betulinic acid, carboplatin, camptothecin, doxorubicin, etoposide, mitoxantrone, mytomycin C, oxaliplatin, staurosporine, and thapsigargin. For each hit in each experimental settings, mean Δ and SEM are reported.

Table 4B (part I)

| | Betulinic acid | | Carboplatin | | Cisplatin* | | Camptothecin | | Doxorubicin | |
|---|---|---|---|---|---|---|---|---|---|---|
| | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) |
| MCL1 | -0,26 | 0,10 | -0,42 | 0,04 | -0,37 | 0,04 | -0,40 | 0,04 | -0,17 | 0,06 |
| TFF1 | -0,09 | 0,06 | -0,27 | 0,05 | -0,36 | 0,05 | -0,16 | 0,07 | -0,21 | 0,05 |
| BCL2L1 | -0,16 | 0,08 | -0,34 | 0,05 | -0,35 | 0,04 | -0,28 | 0,04 | -0,24 | 0,04 |
| RAB3C | -0,07 | 0,07 | -0,25 | 0,05 | -0,33 | 0,06 | -0,12 | 0,05 | -0,17 | 0,04 |
| PPM1J | -0,08 | 0,07 | -0,18 | 0,05 | -0,33 | 0,04 | -0,13 | 0,08 | -0,18 | 0,05 |
| CD151 | -0,09 | 0,10 | -0,33 | 0,04 | -0,32 | 0,12 | -0,30 | 0,04 | -0,03 | 0,03 |
| BMP7 | -0,06 | 0,08 | -0,19 | 0,05 | -0,30 | 0,04 | -0,18 | 0,04 | -0,05 | 0,05 |
| RAC2 | -0,08 | 0,09 | -0,31 | 0,05 | -0,30 | 0,04 | -0,26 | 0,06 | -0,12 | 0,07 |
| WSB2 | -0,04 | 0,07 | -0,36 | 0,04 | -0,30 | 0,05 | -0,30 | 0,04 | -0,18 | 0,06 |
| LOC390533 | -0,10 | 0,10 | -0,30 | 0,04 | -0,29 | 0,09 | -0,24 | 0,06 | -0,09 | 0,06 |
| CYP11B2 | -0,02 | 0,11 | -0,23 | 0,05 | -0,28 | 0,05 | -0,14 | 0,07 | -0,10 | 0,05 |
| RRAD | -0,14 | 0,08 | -0,23 | 0,08 | -0,28 | 0,05 | -0,22 | 0,06 | 0,02 | 0,06 |
| MAT2A | 0,03 | 0,09 | -0,11 | 0,04 | -0,27 | 0,05 | 0,04 | 0,09 | -0,08 | 0,04 |
| USP8 | -0,14 | 0,07 | -0,19 | 0,05 | -0,27 | 0,04 | -0,20 | 0,06 | -0,25 | 0,04 |
| ITGB6 | -0,21 | 0,06 | -0,32 | 0,03 | -0,27 | 0,05 | -0,35 | 0,05 | -0,03 | 0,03 |
| DEFB4 | -0,07 | 0,09 | -0,25 | 0,05 | -0,27 | 0,04 | -0,21 | 0,06 | -0,02 | 0,04 |
| NCKAP1 | -0,12 | 0,08 | -0,32 | 0,04 | -0,27 | 0,05 | -0,30 | 0,05 | -0,04 | 0,07 |
| IAPP | -0,05 | 0,08 | -0,26 | 0,04 | -0,26 | 0,04 | -0,24 | 0,04 | -0,05 | 0,08 |
| IL6R | -0,06 | 0,06 | -0,11 | 0,06 | -0,25 | 0,04 | -0,05 | 0,06 | 0,04 | 0,05 |
| PLK1S1 | -0,05 | 0,08 | -0,11 | 0,07 | -0,25 | 0,05 | -0,11 | 0,08 | -0,09 | 0,04 |
| BTNL2 | -0,03 | 0,09 | -0,18 | 0,05 | -0,25 | 0,05 | -0,06 | 0,05 | -0,01 | 0,06 |
| FLOT2 | -0,04 | 0,06 | -0,15 | 0,05 | -0,25 | 0,05 | -0,19 | 0,05 | -0,10 | 0,04 |
| SEMG2 | -0,06 | 0,06 | -0,17 | 0,04 | -0,24 | 0,05 | -0,13 | 0,04 | -0,06 | 0,04 |
| BSN | -0,04 | 0,07 | -0,20 | 0,04 | -0,24 | 0,05 | -0,23 | 0,06 | -0,03 | 0,07 |

| | Betulinic acid | | Carboplatin | | Cisplatin* | | Camptothecin | | Doxorubicin | |
|---|---|---|---|---|---|---|---|---|---|---|
| | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) |
| PLD3 | -0,08 | 0,06 | -0,28 | 0,05 | -0,24 | 0,05 | -0,33 | 0,04 | -0,12 | 0,05 |
| PRSS21 | -0,08 | 0,07 | -0,13 | 0,06 | -0,24 | 0,04 | -0,19 | 0,06 | -0,05 | 0,07 |
| ADAR | -0,16 | 0,06 | -0,18 | 0,06 | -0,24 | 0,04 | -0,13 | 0,07 | 0,03 | 0,04 |
| HSPC150 | -0,06 | 0,08 | -0,27 | 0,04 | -0,24 | 0,05 | -0,25 | 0,04 | -0,05 | 0,05 |
| HGD | -0,14 | 0,05 | -0,31 | 0,05 | -0,24 | 0,05 | -0,35 | 0,05 | -0,22 | 0,03 |
| CLIC1 | -0,08 | 0,08 | -0,13 | 0,05 | -0,23 | 0,04 | 0,01 | 0,06 | -0,07 | 0,03 |
| NALP1 | -0,07 | 0,07 | -0,11 | 0,04 | -0,22 | 0,04 | -0,10 | 0,04 | -0,16 | 0,03 |
| LRMP | -0,08 | 0,07 | -0,13 | 0,05 | -0,22 | 0,05 | -0,01 | 0,06 | 0,04 | 0,04 |
| C10RF91 | -0,07 | 0,06 | -0,17 | 0,04 | -0,22 | 0,05 | -0,14 | 0,04 | -0,07 | 0,03 |
| LOC389727 | -0,09 | 0,06 | -0,18 | 0,04 | -0,21 | 0,05 | -0,14 | 0,04 | -0,12 | 0,03 |
| ACHE | -0,06 | 0,10 | -0,16 | 0,06 | -0,21 | 0,05 | -0,12 | 0,05 | 0,04 | 0,06 |
| BAIAP3 | 0,01 | 0,09 | -0,21 | 0,05 | -0,21 | 0,06 | -0,20 | 0,08 | -0,07 | 0,05 |
| HIRIP3 | -0,06 | 0,07 | -0,19 | 0,03 | -0,21 | 0,05 | -0,12 | 0,06 | -0,06 | 0,05 |
| PPM1B | 0,05 | 0,11 | -0,17 | 0,04 | -0,21 | 0,06 | -0,13 | 0,08 | -0,01 | 0,07 |
| KHSRP | -0,08 | 0,08 | -0,26 | 0,06 | -0,21 | 0,05 | -0,13 | 0,05 | -0,11 | 0,05 |
| ZDHHC9 | -0,05 | 0,08 | -0,15 | 0,04 | -0,20 | 0,05 | -0,12 | 0,07 | 0,01 | 0,05 |
| CD34 | -0,03 | 0,08 | -0,19 | 0,05 | -0,20 | 0,05 | -0,10 | 0,04 | -0,02 | 0,04 |
| TMED1 | -0,08 | 0,07 | -0,27 | 0,05 | -0,19 | 0,04 | -0,24 | 0,04 | 0,06 | 0,03 |
| NCOA3 | -0,06 | 0,08 | -0,33 | 0,05 | -0,19 | 0,04 | -0,32 | 0,07 | 0,06 | 0,06 |
| TCTE3 | -0,05 | 0,09 | -0,16 | 0,05 | -0,19 | 0,05 | -0,08 | 0,07 | 0,00 | 0,03 |
| C10orf92 | -0,17 | 0,08 | -0,19 | 0,06 | -0,18 | 0,09 | -0,36 | 0,05 | -0,12 | 0,07 |
| UBE2L3 | -0,03 | 0,11 | -0,27 | 0,04 | -0,17 | 0,04 | -0,23 | 0,05 | -0,04 | 0,05 |
| NANOS1 | 0,02 | 0,09 | -0,15 | 0,05 | -0,17 | 0,06 | -0,11 | 0,06 | -0,05 | 0,05 |
| LIPC | -0,14 | 0,07 | -0,14 | 0,04 | -0,16 | 0,05 | -0,09 | 0,06 | -0,19 | 0,04 |

(continued)

| | Betulinic acid | | Carboplatin | | Cisplatin* | | Camptothecin | | Doxorubicin | |
|---|---|---|---|---|---|---|---|---|---|---|
| | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) |
| OSTCL | -0,03 | 0,09 | -0,15 | 0,04 | -0,16 | 0,07 | -0,21 | 0,06 | 0,01 | 0,04 |
| GUCA1 A | -0,04 | 0,09 | -0,13 | 0,05 | -0,14 | 0,07 | -0,28 | 0,06 | -0,04 | 0,05 |
| EBP | 0,00 | 0,07 | -0,19 | 0,06 | -0,13 | 0,06 | -0,19 | 0,06 | -0,14 | 0,03 |
| SEMA3C | 0,08 | 0,07 | -0,10 | 0,04 | -0,11 | 0,06 | -0,18 | 0,08 | 0,01 | 0,06 |
| KIAA1161 | 0,06 | 0,08 | -0,20 | 0,06 | -0,02 | 0,06 | -0,19 | 0,06 | 0,14 | 0,07 |
| ALK | 0,01 | 0,07 | -0,08 | 0,05 | 0,01 | 0,05 | -0,15 | 0,11 | 0,15 | 0,07 |
| EEF2 | -0,03 | 0,10 | -0,01 | 0,07 | 0,02 | 0,06 | 0,02 | 0,13 | 0,08 | 0,05 |
| COX7C | 0,07 | 0,10 | 0,07 | 0,05 | 0,05 | 0,06 | -0,01 | 0,09 | 0,26 | 0,06 |
| C21orf2 | 0,00 | 0,07 | -0,17 | 0,04 | 0,06 | 0,06 | -0,06 | 0,09 | 0,14 | 0,07 |
| RPSAP32 | 0,01 | 0,09 | -0,02 | 0,04 | 0,07 | 0,07 | -0,02 | 0,06 | -0,05 | 0,05 |
| PDXK | 0,03 | 0,06 | 0,05 | 0,06 | 0,07 | 0,07 | -0,07 | 0,07 | 0,01 | 0,04 |
| MPP6 | 0,13 | 0,07 | 0,04 | 0,05 | 0,08 | 0,08 | 0,01 | 0,08 | 0,04 | 0,06 |
| RAB40A | 0,00 | 0,07 | -0,03 | 0,04 | 0,09 | 0,07 | -0,13 | 0,08 | 0,13 | 0,06 |
| OVCH1 | 0,01 | 0,07 | -0,11 | 0,04 | 0,11 | 0,05 | -0,22 | 0,09 | 0,04 | 0,05 |
| GRLF1 | 0,09 | 0,07 | -0,05 | 0,04 | 0,11 | 0,07 | 0,02 | 0,08 | 0,16 | 0,06 |
| SLC22A1LS | -0,02 | 0,10 | 0,00 | 0,05 | 0,11 | 0,08 | -0,05 | 0,06 | 0,12 | 0,08 |
| LOC442126 | 0,00 | 0,06 | -0,06 | 0,04 | 0,12 | 0,07 | -0,21 | 0,07 | 0,19 | 0,09 |
| DHRSX | 0,05 | 0,07 | 0,05 | 0,04 | 0,13 | 0,07 | -0,03 | 0,07 | 0,12 | 0,05 |
| LOC440056 | -0,10 | 0,06 | 0,03 | 0,04 | 0,14 | 0,06 | -0,07 | 0,08 | 0,18 | 0,05 |
| FBX042 | 0,06 | 0,06 | -0,09 | 0,04 | 0,14 | 0,06 | -0,06 | 0,06 | 0,01 | 0,05 |
| ALDH7A1 | 0,06 | 0,07 | -0,02 | 0,05 | 0,14 | 0,06 | -0,15 | 0,07 | 0,16 | 0,05 |
| LOC440611 | 0,13 | 0,07 | 0,00 | 0,05 | 0,14 | 0,06 | -0,08 | 0,06 | 0,02 | 0,05 |
| HDLBP | 0,04 | 0,08 | 0,10 | 0,05 | 0,15 | 0,06 | 0,03 | 0,07 | 0,06 | 0,06 |
| LOC441115 | -0,01 | 0,08 | -0,09 | 0,06 | 0,16 | 0,07 | -0,22 | 0,07 | 0,09 | 0,07 |

| | Betulinic acid | | Carboplatin | | Cisplatin* | | Camptothecin | | Doxorubicin | |
|---|---|---|---|---|---|---|---|---|---|---|
| | Δ** (Mean) | Δ (SEM) | Δ (Mean) | Δ (SEM) | Δ (Mean) | Δ (SEM) | Δ (Mean) | Δ (SEM) | Δ (Mean) | Δ (SEM) |
| ASTL | -0,04 | 0,08 | 0,04 | 0,06 | 0,17 | 0,07 | -0,10 | 0,06 | 0,01 | 0,05 |
| FN3KRP | 0,11 | 0,09 | -0,06 | 0,04 | 0,17 | 0,07 | -0,08 | 0,08 | 0,10 | 0,06 |
| RNF26 | -0,01 | 0,07 | -0,04 | 0,05 | 0,18 | 0,07 | -0,14 | 0,06 | 0,05 | 0,07 |
| PSMC5 | -0,05 | 0,08 | -0,03 | 0,04 | 0,18 | 0,09 | -0,02 | 0,09 | 0,02 | 0,06 |
| TRIP4 | 0,03 | 0,08 | 0,04 | 0,06 | 0,20 | 0,07 | 0,08 | 0,08 | 0,03 | 0,07 |
| SOCS6 | 0,01 | 0,07 | -0,01 | 0,05 | 0,21 | 0,08 | -0,06 | 0,09 | 0,13 | 0,05 |
| PSMD7 | 0,00 | 0,07 | 0,07 | 0,04 | 0,22 | 0,05 | 0,06 | 0,08 | 0,11 | 0,06 |
| COX5B | 0,07 | 0,07 | 0,08 | 0,06 | 0,23 | 0,05 | -0,02 | 0,08 | 0,12 | 0,06 |
| SLC39A5 | 0,09 | 0,07 | 0,09 | 0,05 | 0,23 | 0,07 | 0,11 | 0,08 | 0,19 | 0,06 |
| ZNF878 | 0,14 | 0,09 | 0,12 | 0,05 | 0,25 | 0,08 | 0,01 | 0,08 | -0,01 | 0,05 |
| APAF12 | 0,04 | 0,06 | -0,06 | 0,06 | 0,25 | 0,07 | -0,10 | 0,06 | 0,06 | 0,04 |
| LOC388882 | -0,04 | 0,06 | 0,01 | 0,06 | 0,26 | 0,06 | -0,05 | 0,07 | 0,11 | 0,07 |
| COMMD9 | 0,08 | 0,10 | 0,06 | 0,04 | 0,35 | 0,09 | -0,02 | 0,07 | 0,08 | 0,04 |

**Table 4B (part II):**

| | Etoposide | | Mitoxantrone | | Mytomycin C | | Oxaliplatin | | Staurosporine | | Thapsigargin | |
|---|---|---|---|---|---|---|---|---|---|---|---|---|
| | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| MCL1 | -0,35 | 0,06 | -0,18 | 0,05 | -0,21 | 0,08 | -0,24 | 0,05 | -0,25 | 0,05 | -0,11 | 0,07 |
| TFF1 | -0,05 | 0,06 | -0,20 | 0,04 | -0,23 | 0,08 | -0,11 | 0,05 | -0,07 | 0,07 | -0,08 | 0,06 |
| BCL2L1 | -0,12 | 0,07 | -0,17 | 0,03 | -0,22 | 0,10 | -0,25 | 0,05 | -0,20 | 0,06 | -0,06 | 0,03 |
| RAB3C | -0,06 | 0,08 | -0,09 | 0,04 | -0,20 | 0,11 | -0,11 | 0,06 | -0,16 | 0,05 | 0,06 | 0,08 |
| PPM1J | -0,13 | 0,09 | -0,12 | 0,04 | -0,21 | 0,08 | -0,02 | 0,05 | -0,18 | 0,05 | 0,15 | 0,06 |
| CD151 | -0,03 | 0,08 | -0,03 | 0,07 | -0,08 | 0,09 | -0,08 | 0,06 | -0,17 | 0,05 | 0,02 | 0,08 |
| BMP7 | -0,06 | 0,07 | -0,10 | 0,06 | 0,00 | 0,09 | 0,01 | 0,07 | -0,17 | 0,05 | 0,16 | 0,03 |
| RAC2 | -0,06 | 0,10 | -0,12 | 0,03 | -0,24 | 0,09 | -0,10 | 0,06 | -0,07 | 0,06 | -0,02 | 0,07 |
| WSB2 | -0,04 | 0,13 | -0,18 | 0,04 | -0,27 | 0,10 | -0,25 | 0,05 | 0,04 | 0,07 | -0,10 | 0,05 |
| LOC390533 | -0,12 | 0,08 | -0,14 | 0,05 | -0,08 | 0,10 | -0,09 | 0,06 | -0,13 | 0,05 | -0,02 | 0,09 |
| CYP11B2 | 0,00 | 0,06 | -0,16 | 0,04 | -0,04 | 0,13 | -0,07 | 0,05 | -0,17 | 0,05 | 0,20 | 0,06 |
| RRAD | -0,09 | 0,06 | -0,18 | 0,03 | -0,11 | 0,09 | -0,07 | 0,05 | 0,05 | 0,07 | -0,10 | 0,05 |
| MAT2A | 0,10 | 0,08 | -0,03 | 0,05 | 0,02 | 0,09 | 0,14 | 0,05 | -0,05 | 0,06 | 0,17 | 0,05 |
| USP8 | -0,13 | 0,07 | -0,25 | 0,03 | -0,24 | 0,19 | -0,06 | 0,05 | -0,16 | 0,06 | -0,03 | 0,07 |
| ITGB6 | -0,10 | 0,09 | -0,07 | 0,07 | -0,19 | 0,09 | -0,09 | 0,05 | -0,09 | 0,05 | -0,06 | 0,06 |
| DEFB4 | -0,18 | 0,06 | -0,06 | 0,04 | -0,07 | 0,09 | -0,05 | 0,05 | -0,17 | 0,05 | 0,29 | 0,09 |
| NCKAP1 | -0,15 | 0,09 | -0,06 | 0,05 | -0,04 | 0,09 | -0,08 | 0,06 | -0,06 | 0,06 | -0,05 | 0,09 |
| IAPP | -0,12 | 0,09 | -0,05 | 0,04 | -0,10 | 0,08 | -0,05 | 0,06 | -0,15 | 0,04 | 0,08 | 0,03 |
| IL6R | -0,12 | 0,07 | 0,08 | 0,04 | -0,01 | 0,09 | 0,02 | 0,05 | 0,00 | 0,06 | -0,02 | 0,06 |
| PLK1S1 | -0,11 | 0,06 | -0,13 | 0,03 | -0,12 | 0,15 | 0,03 | 0,05 | -0,21 | 0,05 | 0,01 | 0,03 |
| BTNL2 | -0,04 | 0,08 | -0,06 | 0,04 | 0,01 | 0,09 | 0,03 | 0,05 | 0,13 | 0,07 | 0,04 | 0,06 |
| FLOT2 | -0,07 | 0,08 | -0,13 | 0,04 | 0,06 | 0,10 | -0,04 | 0,06 | -0,18 | 0,06 | 0,24 | 0,04 |
| SEMG2 | -0,05 | 0,06 | -0,01 | 0,03 | -0,02 | 0,09 | 0,00 | 0,05 | -0,03 | 0,06 | 0,30 | 0,03 |
| BSN | -0,19 | 0,08 | -0,06 | 0,05 | -0,13 | 0,06 | -0,05 | 0,05 | -0,11 | 0,05 | 0,04 | 0,06 |

| | Etoposide | | Mitoxantrone | | Mytomycin C | | Oxaliplatin | | Staurosporine | | Thapsigargin | |
|---|---|---|---|---|---|---|---|---|---|---|---|---|
| | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| PLD3 | -0,14 | 0,07 | -0,17 | 0,05 | -0,14 | 0,11 | -0,19 | 0,05 | -0,13 | 0,05 | -0,08 | 0,04 |
| PRSS21 | -0,02 | 0,07 | -0,12 | 0,03 | -0,05 | 0,17 | -0,02 | 0,06 | 0,01 | 0,05 | -0,06 | 0,05 |
| ADAR | -0,15 | 0,08 | -0,11 | 0,03 | -0,07 | 0,10 | -0,01 | 0,06 | 0,01 | 0,05 | 0,07 | 0,05 |
| HSPC150 | -0,10 | 0,09 | -0,14 | 0,04 | 0,00 | 0,10 | -0,10 | 0,05 | -0,14 | 0,05 | 0,20 | 0,07 |
| HGD | -0,25 | 0,08 | -0,23 | 0,04 | -0,17 | 0,09 | -0,11 | 0,05 | -0,20 | 0,05 | -0,18 | 0,04 |
| CLIC1 | -0,11 | 0,07 | -0,07 | 0,04 | 0,06 | 0,15 | 0,11 | 0,06 | 0,04 | 0,07 | -0,10 | 0,05 |
| NALP1 | -0,06 | 0,06 | -0,11 | 0,04 | 0,11 | 0,10 | 0,01 | 0,05 | 0,00 | 0,05 | 0,23 | 0,05 |
| LRMP | -0,11 | 0,07 | 0,04 | 0,05 | 0,01 | 0,09 | 0,06 | 0,07 | -0,08 | 0,05 | 0,34 | 0,05 |
| C10RF91 | -0,08 | 0,07 | -0,04 | 0,03 | 0,01 | 0,09 | -0,07 | 0,05 | -0,13 | 0,06 | 0,03 | 0,04 |
| LOC389727 | -0,14 | 0,08 | -0,11 | 0,04 | -0,05 | 0,09 | -0,01 | 0,05 | -0,01 | 0,06 | 0,07 | 0,06 |
| ACHE | -0,03 | 0,07 | -0,03 | 0,05 | -0,10 | 0,13 | -0,01 | 0,05 | -0,10 | 0,06 | -0,03 | 0,06 |
| BAIAP3 | -0,06 | 0,09 | -0,03 | 0,05 | -0,10 | 0,07 | -0,07 | 0,05 | 0,02 | 0,07 | 0,01 | 0,05 |
| HIRIP3 | -0,13 | 0,09 | -0,08 | 0,04 | -0,13 | 0,12 | -0,01 | 0,06 | -0,10 | 0,06 | 0,25 | 0,04 |
| PPM1B | 0,13 | 0,12 | -0,05 | 0,05 | -0,01 | 0,07 | 0,03 | 0,06 | -0,02 | 0,05 | 0,14 | 0,06 |
| KHSRP | -0,15 | 0,08 | -0,13 | 0,05 | -0,06 | 0,10 | -0,15 | 0,05 | -0,10 | 0,07 | 0,14 | 0,05 |
| ZDHHC9 | -0,10 | 0,07 | -0,05 | 0,05 | 0,05 | 0,10 | 0,04 | 0,08 | -0,16 | 0,06 | 0,17 | 0,11 |
| CD34 | -0,08 | 0,05 | -0,04 | 0,06 | -0,04 | 0,09 | -0,01 | 0,05 | -0,01 | 0,06 | 0,13 | 0,04 |
| TMED1 | -0,07 | 0,07 | -0,03 | 0,04 | -0,12 | 0,08 | -0,05 | 0,07 | 0,11 | 0,07 | -0,10 | 0,05 |
| NCOA3 | -0,09 | 0,07 | 0,03 | 0,05 | -0,04 | 0,10 | -0,21 | 0,06 | -0,17 | 0,05 | 0,06 | 0,06 |
| TCTE3 | -0,03 | 0,06 | 0,01 | 0,03 | -0,05 | 0,09 | 0,01 | 0,06 | -0,10 | 0,05 | 0,25 | 0,09 |
| Cl0orf92 | -0,09 | 0,08 | -0,14 | 0,03 | -0,15 | 0,11 | -0,14 | 0,07 | 0,09 | 0,08 | -0,05 | 0,08 |
| UBE2L3 | -0,13 | 0,06 | -0,08 | 0,05 | -0,03 | 0,09 | -0,01 | 0,05 | -0,05 | 0,06 | 0,08 | 0,08 |
| NANOS1 | -0,02 | 0,06 | -0,09 | 0,04 | -0,08 | 0,18 | -0,06 | 0,06 | -0,13 | 0,05 | 0,18 | 0,11 |
| LIPC | -0,06 | 0,08 | -0,18 | 0,04 | -0,04 | 0,08 | 0,02 | 0,05 | -0,12 | 0,06 | -0,02 | 0,05 |

(continued)

| | Etoposide | | Mitoxantrone | | Mytomycin C | | Oxaliplatin | | Staurosporine | | Thapsigargin | |
|---|---|---|---|---|---|---|---|---|---|---|---|---|
| | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) | Δ(Mean) | Δ(SEM) |
| OSTCL | -0,08 | 0,08 | -0,09 | 0,04 | 0,02 | 0,08 | 0,02 | 0,05 | -0,09 | 0,04 | 0,18 | 0,05 |
| GUCA1 A | 0,08 | 0,11 | -0,05 | 0,07 | 0,01 | 0,07 | 0,05 | 0,06 | -0,02 | 0,04 | -0,04 | 0,05 |
| EBP | -0,07 | 0,07 | -0,07 | 0,04 | 0,02 | 0,11 | -0,06 | 0,05 | -0,13 | 0,06 | 0,23 | 0,06 |
| SEMA3C | -0,05 | 0,09 | 0,05 | 0,05 | -0,07 | 0,06 | -0,01 | 0,05 | -0,12 | 0,04 | 0,09 | 0,05 |
| KIAA1161 | -0,04 | 0,09 | 0,05 | 0,04 | -0,13 | 0,09 | -0,12 | 0,06 | -0,09 | 0,05 | 0,11 | 0,06 |
| ALK | 0,07 | 0,11 | 0,17 | 0,10 | -0,11 | 0,10 | 0,07 | 0,07 | 0,06 | 0,05 | 0,07 | 0,09 |
| EEF2 | 0,20 | 0,16 | 0,07 | 0,09 | -0,04 | 0,11 | 0,07 | 0,07 | 0,04 | 0,05 | 0,02 | 0,06 |
| COX7C | 0,18 | 0,11 | 0,12 | 0,09 | 0,04 | 0,07 | 0,11 | 0,06 | 0,04 | 0,04 | 0,13 | 0,07 |
| C21orf2 | -0,05 | 0,10 | 0,13 | 0,09 | -0,06 | 0,07 | -0,01 | 0,06 | 0,14 | 0,04 | 0,15 | 0,04 |
| RPSAP32 | -0,03 | 0,11 | -0,04 | 0,07 | -0,16 | 0,14 | -0,05 | 0,06 | -0,14 | 0,05 | 0,22 | 0,07 |
| PDXK | -0,03 | 0,09 | 0,11 | 0,08 | 0,02 | 0,08 | 0,09 | 0,07 | 0,01 | 0,05 | 0,24 | 0,06 |
| MPP6 | -0,02 | 0,10 | 0,10 | 0,07 | 0,00 | 0,06 | 0,04 | 0,06 | -0,10 | 0,04 | 0,23 | 0,07 |
| RAB40A | -0,06 | 0,10 | 0,06 | 0,07 | -0,10 | 0,07 | 0,05 | 0,06 | 0,04 | 0,05 | 0,18 | 0,05 |
| OVCH1 | -0,10 | 0,10 | 0,20 | 0,07 | 0,19 | 0,07 | 0,01 | 0,05 | -0,05 | 0,05 | 0,19 | 0,10 |
| GRLF1 | 0,07 | 0,09 | 0,22 | 0,08 | 0,10 | 0,07 | 0,12 | 0,06 | -0,11 | 0,04 | 0,21 | 0,05 |
| SLC22A1LS | 0,06 | 0,12 | -0,03 | 0,06 | -0,05 | 0,06 | 0,02 | 0,07 | -0,13 | 0,03 | -0,01 | 0,04 |
| LOC442126 | -0,07 | 0,10 | 0,15 | 0,07 | -0,04 | 0,06 | 0,05 | 0,05 | 0,15 | 0,04 | 0,16 | 0,07 |
| DHRSX | 0,01 | 0,11 | 0,15 | 0,08 | 0,05 | 0,06 | 0,09 | 0,06 | -0,02 | 0,04 | 0,24 | 0,08 |
| LOC440056 | 0,03 | 0,15 | 0,12 | 0,07 | -0,01 | 0,07 | -0,03 | 0,05 | 0,19 | 0,05 | 0,19 | 0,08 |
| FBX042 | -0,01 | 0,10 | 0,18 | 0,07 | -0,03 | 0,08 | -0,02 | 0,06 | -0,09 | 0,04 | 0,04 | 0,04 |
| ALDH7A1 | 0,06 | 0,10 | 0,05 | 0,07 | -0,01 | 0,06 | -0,01 | 0,05 | -0,19 | 0,04 | 0,23 | 0,06 |
| LOC440611 | 0,09 | 0,09 | 0,12 | 0,07 | 0,04 | 0,07 | 0,01 | 0,06 | 0,04 | 0,04 | 0,25 | 0,05 |
| HDLBP | 0,11 | 0,11 | 0,18 | 0,07 | -0,02 | 0,09 | 0,11 | 0,07 | 0,06 | 0,04 | 0,19 | 0,08 |
| LOC441115 | 0,05 | 0,16 | 0,24 | 0,11 | 0,00 | 0,06 | -0,05 | 0,06 | -0,07 | 0,05 | 0,09 | 0,06 |

(continued)

| | Etoposide | | Mitoxantrone | | Mytomycin C | | Oxaliplatin | | Staurosporine | | Thapsigargin | |
|---|---|---|---|---|---|---|---|---|---|---|---|---|
| | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) | Δ (Mean) | Δ (SEM) |
| ASTL | 0,03 | 0,10 | 0,00 | 0,07 | -0,02 | 0,07 | 0,10 | 0,05 | -0,03 | 0,05 | 0,07 | 0,07 |
| FN3KRP | 0,06 | 0,12 | 0,18 | 0,09 | 0,02 | 0,07 | -0,02 | 0,12 | 0,08 | 0,11 | 0,21 | 0,07 |
| RNF26 | -0,09 | 0,10 | -0,04 | 0,05 | 0,04 | 0,07 | -0,03 | 0,06 | -0,09 | 0,04 | 0,19 | 0,06 |
| PSMC5 | 0,04 | 0,12 | -0,11 | 0,05 | -0,01 | 0,09 | 0,10 | 0,05 | -0,18 | 0,04 | 0,01 | 0,06 |
| TRIP4 | 0,04 | 0,13 | 0,02 | 0,06 | 0,12 | 0,08 | 0,01 | 0,05 | -0,02 | 0,04 | 0,24 | 0,08 |
| SOCS6 | 0,00 | 0,09 | 0,25 | 0,13 | -0,01 | 0,06 | -0,04 | 0,06 | 0,00 | 0,05 | 0,18 | 0,09 |
| PSMD7 | 0,09 | 0,11 | 0,13 | 0,07 | 0,04 | 0,06 | 0,12 | 0,05 | -0,02 | 0,03 | 0,29 | 0,06 |
| COX5B | 0,11 | 0,09 | 0,06 | 0,08 | 0,10 | 0,07 | 0,12 | 0,07 | 0,12 | 0,05 | 0,02 | 0,05 |
| SLC39A5 | 0,11 | 0,10 | 0,13 | 0,08 | 0,01 | 0,07 | 0,13 | 0,07 | 0,15 | 0,03 | 0,22 | 0,06 |
| ZNF878 | 0,09 | 0,10 | 0,06 | 0,06 | 0,04 | 0,09 | 0,14 | 0,05 | -0,11 | 0,03 | 0,28 | 0,06 |
| APAF12 | -0,03 | 0,10 | 0,06 | 0,04 | -0,02 | 0,07 | 0,01 | 0,07 | -0,08 | 0,03 | 0,17 | 0,05 |
| LOC388882 | 0,00 | 0,11 | 0,13 | 0,08 | 0,16 | 0,07 | 0,03 | 0,06 | -0,12 | 0,03 | 0,03 | 0,06 |
| COMMD9 | 0,11 | 0,11 | 0,07 | 0,07 | 0,00 | 0,07 | 0,02 | 0,06 | -0,04 | 0,05 | 0,09 | 0,06 |

\* = reference profile

\*\* = upon background subtraction and inter-plate normalization, Δ = relative survival of siRNA-transfected cells (WST-1 signal from CDDP treated, siRNA-transfected cells / WST-1 signal from untreated, siRNA-transfected cells) - relative survival of cells transfected with a control siRNA (siUNR) (WST-1 signal from CDDP treated, siUNR-transfected cells / WST-1 signal from untreated, siUNR-transfected cells). Negative and positive Δ values signify chemosensitization and cytoprotection, respectively.

SEM = standard error of the mean

*Epistatic screening of CDDP response modifiers.*

To gain further insights into the functional relationship between the CRMs identified in this screen, inventors performed a systematic epistatic analysis. For this, they selected the most efficient siRNAs corresponding to each of the 85 hits (Fig. 1) and to 7 additional genes (BAK1, BAX, BCL2, CASP2, STAT3, TP53, VDAC1) identified as possible CRMs in the past.

A549 cells were transfected with 92 x 92 couples of siRNAs, treated with CDDP, and characterized for the combined effects of the siRNAs on WST-1 conversion. The resultant matrix of epistatic effects (Fig. 4A) was subjected to unsupervised hierarchical clustering (following the Ward method and the Pearson correlation), leading to the identification of four separate clusters (Table 5). Within each cluster, combined knockdown of two transcripts mostly failed to yield significant epistatic interactions, while the knockdown of transcript belonging to distinct clusters caused epistasis. Some of these results were expected (such as the classification of the anti-apoptotic proteins BCL2, BCL-X$_L$ and MCL1 in cluster 1, that of the COX subunits Vb and VIId in cluster 3, and that of the pro-apoptotic proteins BAX, BAK1, APAF1, TP53, VDAC1, CASP2, the BCL2 interactor NLRP1 ,and two proteasome subunits in cluster 4), while others were not (such as the presence of IL6R, STAT3 and CLIC1 in cluster 1).

These results were validated using siRNAs (Fig. 4B, 4D, 4F) and pharmacological inhibitors (Fig. 4C, 4E, 4G), followed by DiOC$_6$(3)/PI co-staining as an alternative readout. Thus the combined depletion/inhibition of ZDHHC9 (which is inhibited by 2-bromopalmitate) and IL6R (which can be blocked by an extracellular antibody), both belonging to cluster 1, yields no significant epistatic interaction (Fig. 4B, 4C). In contrast, simultaneous depletion/inhibition of IL6R (in cluster 1) and LIPC (which can be inhibited by orlistat and belonged to cluster 2) advantageously synergistically sensitized to CDDP-induced killing (Fig. 4C, 4D). Moreover, concomitant knockdown or inhibition of COX (in cluster 3) with sodium azide and TP53 (in cluster 4) with cyclic pifithrin-$\alpha$ yielded synergistic protection against CDDP (Fig. 4F, 4G). Altogether, these results demonstrate that (at least) four different signalling modules determine CDDP responses.

**Table 5: Epistatic clustering.**

| Epistatic analyses upon co-transfection of the 85 siRNA screen allowed for the identification of 4 clusters of cisplatin response modifiers (CRMs). For each the hit, accession number and cluster are reported. See also Figures 4. | | |
|---|---|---|
| **Name** | **Accession number** | **Cluster** |
| ACHE | NM_000665 | 1 |
| ADAR | NM_001111 | 1 |
| ALDH7A1 | NM_001182 | 4 |
| ALK | NM_004304 | 3 |
| APAF1 | NM_001160 | 4 |
| ASTL | NM_001002036 | 4 |
| BAIAP3 | NM_003933 | 1 |
| BAK1 | NM_001188 | 4 |
| BAX | NM_004324 | 4 |
| BCL2 | NM_000657/NM_000633 | 1 |
| BCL2L1 | NM_001191 | 1 |
| BMP7 | NM_001719 | 2 |
| BSN | NM_003458 | 1 |
| BTNL2 | NM_019602 | 1 |
| C100RF92 | NM_017609 | 1 |
| C10RF91 | NM_019118 | 1 |
| C21ORF2 | NM_004928 | 4 |
| CASP2 | NM_032982/NM_032983 | 4 |
| CD151 | NM_004357 | 1 |
| CD34 | NM_001773 | 1 |

(continued)

| Epistatic analyses upon co-transfection of the 85 siRNA screen allowed for the identification of 4 clusters of cisplatin response modifiers (CRMs). For each the hit, accession number and cluster are reported. See also Figures 4. | | |
|---|---|---|
| **Name** | **Accession number** | **Cluster** |
| CLIC1 | NM_001288 | 1 |
| COMMD9 | NM_014186 | 4 |
| COX5B | NM_001862 | 3 |
| COX7C | NM_001867 | 3 |
| CYP11B2 | NM_000498 | 1 |
| DEFB4 | NM_004942 | 1 |
| DHRSX | NM_145177 | 3 |
| EBP | NM_006579 | 1 |
| EEF2 | NM_001961 | 3 |
| FBX042 | NM_018994 | 4 |
| FLOT2 | NM_004475 | 1 |
| FN3KRP | NM_024619 | 4 |
| GRLF1 | NM_004491 | 4 |
| GUCA1A | NM_000409 | 3 |
| HDLBP | NM_005336 | 4 |
| HGD | NM_000187 | 2 |
| HIRIP3 | NM_003609 | 1 |
| IAPP | NM_000415 | 2 |
| IL6R | NM_000565 | 1 |
| ITGB6 | NM_000888 | 1 |
| KHSRP | NM_003685 | 1 |
| KIA1161 | NM_020702 | 4 |
| LIPC | NM_000236 | 2 |
| LOC388882 | XR_042117 | 4 |
| LOC389727 | Pseudogene | 2 |
| LOC390533 | Discontinued | 2 |
| RPSAP32 | Pseudogene | 4 |
| LOC440056 | Discontinued | 4 |
| LOC440611 | Discontinued | 3 |
| LOC441115 | Discontinued | 4 |
| LOC442126 | Discontinued | 4 |
| LRMP | NM_006152 | 2 |
| MAT2A | NM_005911 | 1 |
| MCL1 | NM_021960 | 1 |
| MPP6 | NM_016447 | 4 |
| NANOS1 | NM_199461 | 1 |

(continued)

| Name | Accession number | Cluster |
|---|---|---|
| NCKAP1 | NM_013436 | 1 |
| NCOA3 | NM_006534 | 1 |
| NLRP1 | NM_014922 | 4 |
| OSTCL | NM_145303 | 1 |
| OVCH1 | NM_183378 | 4 |
| PDXK | NM_003681 | 4 |
| PLD3 | NM_012268 | 1 |
| PLK1S1 | NM_018474 | 1 |
| PPM1B | NM_002706 | 1 |
| PPM1J | NM_005167 | 1 |
| PRSS21 | NM_006799 | 2 |
| PSMC5 | NM_002805 | 4 |
| PSMD7 | NM_002811 | 4 |
| RAB3C | NM_138453 | 2 |
| RAB40A | NM_080879 | 4 |
| RAC2 | NM_002872 | 1 |
| RNF26 | NM_032015 | 4 |
| RRAD | NM_004165 | 1 |
| SEMA3C | NM_006379 | 2 |
| SEMG2 | NM_003008 | 1 |
| SLC22A18AS | NM_007105 | 4 |
| SLC39A5 | NM_173596 | 4 |
| SOCS6 | NM_004232 | 4 |
| STAT3 | NM_003150 | 1 |
| TCTE3 | NM_174910 | 2 |
| TFF1 | NM_003225 | 1 |
| TMED1 | NM_006858 | 4 |
| TP53 | NM_000546 | 4 |
| TRIP4 | NM_016213 | 4 |
| UBE2L3 | NM_003347 | 1 |
| UBE2T | NM_014176 | 1 |
| USP8 | NM_005154 | 1 |
| VDAC1 | NM_003374/NM_003150 | 4 |
| WSB2 | NM_018639 | 1 |
| ZDHHC9 | NM_001008222 | 1 |
| ZNF878 | NM_001080404 | 4 |

Epistatic analyses upon co-transfection of the 85 siRNA screen allowed for the identification of 4 clusters of cisplatin response modifiers (CRMs). For each the hit, accession number and cluster are reported. See also Figures 4.

### Impact of intermediate metabolism on CDDP responses.

Inventors observed that pyridoxal kinase (PDXK) and aldehyde dehydrogenase family 7, member A1 (ALDH7A1, an evolutionarily conserved protein also known as antiquitin), two enzymes involved in vitamin B6 metabolism (Fig. 5A), were both found in cluster 4 (Fig. 4A), suggesting that they act on similar pathway to modulate CDDP responses. Therefore, they decided to investigate the impact of the enzymatically active form of vitamin B6 (pyridoxal-5-phosphate, PLP) and its closest precursor (the B6 vitamer pyridoxine, PN) on CDDP responses.

PDXK, converts vitamin B6 precursors into their enzymatically active derivative, PLP {Lee, 2000}. ALDH7A1 metabolizes L-2-aminoadipate 6-semialdehyde (AAS, also known as piperideine-6-carboxylate) which reacts with PLP by Knoevenagel condensation {Mills, 2006}. The depletion of ALDH7A1 hence indirectly causes a reduction in PLP levels. Acute addition of PN (but not that of PLP, which cannot cross the plasma membrane) increased the intracellular PN concentration (Fig. 5B) and exacerbated CDDP-induced cell death, as quantified by $DiOC_6(3)$/PI co-staining (Fig. 5C) or by measuring impedance caused by attached, viable cells (Fig. 8F). Chronic treatment with increasing amounts of B6 vitamer sensitized both NSCLC (Fig. 5D) and yeast cells (Fig. 5E) to CDDP, but the same effect could not be observed when yeast cells were killed by cadmium (Fig. 5E). Inventors then transfected A549 cells with a series of siRNA targeting B6-relevant enzymes (Fig. 5F) including PDXK, ALDH7A1, as well as PDX phosphatase (PDXP, which functionally antagonizes PDXK, Fig. 5A) and tested their response to CDDP. As expected, PDXK or ALDH7A1 depletion reduced the cytotoxic effect of CDDP, while the knockdown of PDXP provided chemosensitization (Fig. 5G). The effects of PN were lost upon PDXK depletion (Fig. 5H), in accord with the notion that PN must be converted into PLP to become active (Fig. 5I). PLP is the enzymatic co-factor of cysteine conjugate-β lyase (CCBL1), an enzyme that has previously been implicated in the renal toxicity of CDDP {Zhang, 2003}. siRNA-mediated depletion of CCBL1 (Fig. 5J) or addition of the CCBL1 inhibitor aminooxyacetic acid (AOAA) reduced the toxicity of CDDP in both A549 (Fig. 5K) and yeast cells (Fig. 5L). In yeast, the knockout of vitamin B6-related genes *(snol, snzl)* as well as that of the *bona fide PDXK* ortholog *bud17,* rescued from CDDP (but not cadmium)-induced cell death (Fig. 8G). PN exerted chemosensitizing effects on three distinct CDDP-resistant A549 clones, on four additional NSCLC cell lines, as well as on cervical carcinoma, osteosarcoma and wild type (but not $TP5^{-/-}$) colorectal cancer cells (Fig. 10A-C).

Non-oxidized glutathione (GSH) or *N*-acetylcysteine (NAC) blocked the apoptosis-inducing effect of CDDP (irrespective of the presence of PN), be it quantified by $DiOC_6(3)$/PI co-staining (Fig. 8G) or as DNA loss (sub-$G_1$ peak in Fig. 6A). However, NAC failed to reverse the cell cycle arrest induced by CDDP, an effect that was not affected by the presence of PN (Fig. 6A, Fig. 8H). Therefore, they further focused on the cell death-sensitizing effects of PN. In the presence of PN, cells treated by a sub-apoptotic concentration of CDDP exhibited an increased activating phosphorylation of TP53 on serines 15 and 46 (note that TP53 is in the same epistatic cluster as PDXK, Fig. 4, and that TP53 is required for PN-mediated sensitization to CDDP, Fig. 10C), and an increased activation of caspases-3 and -9 (Fig. 6B). Inhibition of caspases, indeed, reduced killing by CDDP alone or in combination with PN, although it did not completely abrogate PN-mediated chemosensitization (Fig. 8G). PN significantly increased CDDP-DNA adducts, as quantified by immunofluorescence staining of cells cultured in the presence of CDDP (Fig. 6C) followed by automated image analysis (Fig. 6D). Inventors also investigated another druggable metabolic pathway and surprisingly discovered that both the knockdown and pharmacological inhibition (with orlistat®) of LIPC enhanced the cytotoxiticy of CDDP against A549 cells *in vitro* (Fig. 1). In line with these observations, systemic injections of orlistat® also advantageously augmented the antitumor effects of CDDP-based chemotherapy *in vivo,* in A549 cells growing on immunodeficient Swiss nude mice, as well as in murine Lewis lung carcinoma cells transplanted into immunocompetent C57BL/6 mice (Fig. 6E).

### Prognostic and predictive impact of PDXK and LIPC on non-small cell lung cancer.

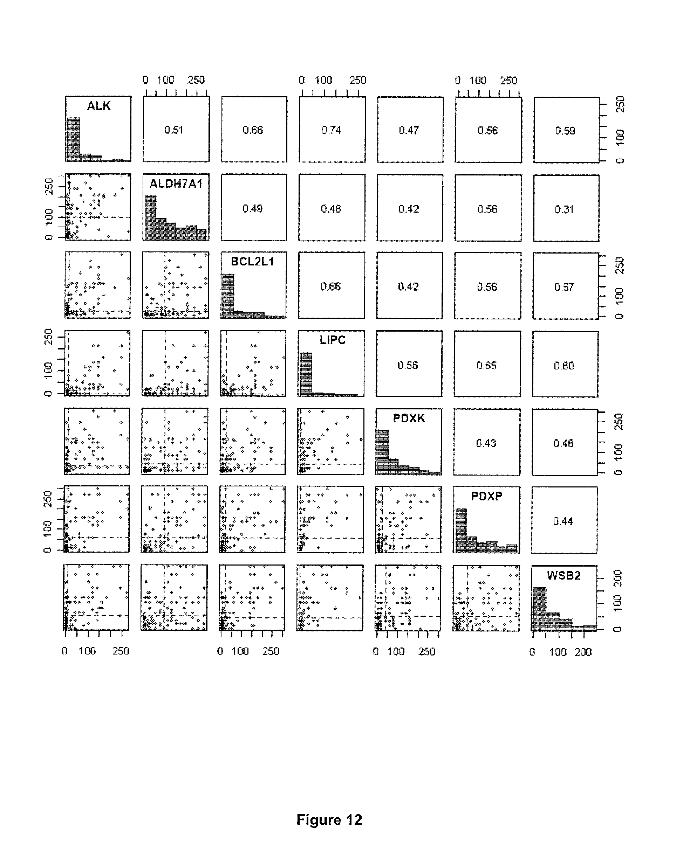

To finally validate the results of the siRNA screen on human NSCLC tumor samples, inventors took advantage of 122 operable NSCLC specimens that were or were not treated with adjuvant CDDP-based chemotherapy (Table 6). Sections of each tumor were stained with antibodies specific for ALDH7A1, ALK, BCL2L1, LIPC, PDXK, PDXP and WSB2. As quantified by immunohistochemistry (Fig. 7A, Fig. 11), the expression levels of each of these proteins failed to exhibit a major positive or negative correlation among each other (Fig. 12). Tumor samples were then arbitrarily divided into two groups with high and low expression of each of the proteins of interest, and their impact on overall survival (OS) and disease-free survival (DFS) was determined, either for all patients or for subgroups that received chemotherapy or were left untreated. The protein expression levels of ALDH7A1, ALK, BCL2L1, PDXP and WSB2 had no impact on OS, DFS or therapeutic responses (Fig. 13).

In contrast, low expression of PDXK had a significant negative impact on DFS (Fig. 7B), irrespective of whether the patients were treated with CDDP or not. Indeed the expression of PDXK did not affect the therapeutic response to CDDP as estimated with survival curves based on a Cox proportional hazard model (Fig. 7C). Thus PDXK constitutes a prognostic biomarker. The absence of LIPC had a significant negative effect on OS when all patients were analyzed together (Fig. 7D). However, LIPC⁻ tumors tended to respond to chemotherapy, while LIPC⁺ ones exhibited a paradoxical behavior in thus far that CDDP-treated patients tended to show a higher frequency of events than untreated patients (Fig. 7E). The interaction between LIPC expression levels and treatment outcome were significant, indicating that LIPC constitutes a predictive biomarker.

**Table 6: Patient characteristics.**

| The cohort of patients included in this study is classified with respect to age, gender, smoking status, histological parameters and treatment with adjuvant chemotherapy. | | |
|---|---|---|
| **N(%)** | | |
| Adjuvant chemotherapy | No | 64 (52.45) |
|  | Yes | 58 (47.55) |
| Histology | ADC | 56 (45.90) |
|  | LCC | 13(10.66) |
|  | SCC | 50 (40.98) |
|  | Others | 3(2.46) |
| Gender | Men | 33 (27,05) |
|  | Women | 89 (72,95) |
| Smoking status | Current | 47 (38,52) |
|  | Former | 66 (54,10) |
|  | Never | 7 (5,74) |
|  | Unknown | 2(1.64) |
| **Median (range) years**\*\* | | |
| Age | 63(41-85) | |
| Follow-up | 2.7 (0.5 - 8.3) | |
| \* = absolute number (percentage); \*\* = median (range); ADC, adenocarcinoma; LCC, large cell carcinoma; SCC, squamous cell carcinoma. | | |

**Concluding remarks**

In this work, inventors identified in particular 85 CRMs by a non-saturating genome-wide siRNA screen and several subsequent and variant rounds of validation. These include known apoptosis regulators (negative: MCL1, BCL2L1, TFF1; positive: APAF1) {Bossenmeyer-Pourie, 2002; Kroemer, 2007}, pro-survival proteins (IL6R), as well as multiple proteins involved in intermediate metabolism (LIPC; homogentisate 1,2-dioxygenase, HGD; high density lipoprotein binding protein, HDLBP; fructosamine-3-kinase-related protein, FN3KRP; COX subunits). A majority of the 85 transcripts/proteins had cytoprotective properties, meaning that their knockdown or their pharmacological inhibition enhances cisplatin-induced killing. Inventors herein demonstrate that these cytoprotective proteins constitute novel targets for chemosensitization. Epistatic analyses indicate that dual interventions on at least two among these transcripts/ proteins induce synergistic chemosensitizing effects. In this example, inventors in particular showed that orlistat®, an inhibitor of LIPC, can improve the efficacy of cisplatin in a preclinical setting, in mice bearing murine or human NSCLC. The siRNA screening also led to the identification of multiple transcripts/proteins whose depletion reduces cisplatin responses, implying that they directly or indirectly contribute to CDDP toxicity. Based on the obtained results, inventors identified ALK inhibitors as detrimental, when used in combination with CDDP, for the therapy of NSCLC. Moreover, the knockdown of two subunits of the respiratory chain complex COX or chemical COX inhibition reduces the toxicity of cisplatin. Thus, a progressive reduction of oxidative phosphorylation, as it occurs during oncogenesis and tumor progression {Kroemer, 2008}, might reduce CDDP responses as a 'natural' resistance mechanism. The knockdown of PDXK (and that of another vitamin B6-related enzyme, ALDH7A1) reduced the cytotoxic activity of CDDP, leading to the discovery that PDXP depletion (the phosphatase that functionally antagonizes PDXK) can stimulate cisplatin-induced tumor cell killing. In line with these observations, inventors found that exogenous administration of a cell-permeable vitamin B6 precursor, PN, sensitized cultured NSCLC cells to CDDP, provided that PDXK was present.

The transcription of most of the 85 CRMs reported here was not affected by CDDP (Figure 2 and Table 3). Indeed, the percentage of CRMs whose mRNA levels were significantly modulated by CDDP (< 10%) was not higher than that affected by the general stress mediator ceramide. Moreover, most CRMs affected the CDDP response (and that of carboplatin as well as camptothecin) in a specific fashion, yet had discordant effects on ceramide, cadmium, pro-apoptotic triggers (such as staurosporine or thapsigargin) or unrelated chemotherapeutics (such as betulinic acid) (Fig. 3). In contrast, the 85 CRMs had similar effects on a range of NSCLC (but not on colorectal cancer) cells.

Driven by the availability of monoclonal antibodies suitable for immunohistochemistry, inventors determined the expression level of 7 CRMs on a panel of 120 NSCLC biopsies. Although there was some degree of heterogeneity in their expression levels, 5 out of these 7 markers had no predictive or prognostic impact, implying that the abundance of these 5 proteins does not have sufficient biological impact to affect the fate of a heterogeneous collection of NSCLC patients. However, 2 proteins, in particular, that they characterized did have a prognostic impact.

Reduced PDXK expression negatively affected DFS. In this context, inventors highlight the paradox of the results provided by Johansson {Johansson, 2010} showing that circulating vitamin B6 levels negatively correlate with the risk of developing clinically manifest NSCLC, and herein demonstrate that vitamin B6 itself - and the enzyme that renders it bioactive - have a profound impact on the biology of NSCLC. Although PDXK affected prognosis, it had no predictive value, meaning that patients expressing high or low levels of PDXK responded similarly to adjuvant chemotherapy. As a possibility, the levels of PDXK may reflect profound differences in the natural history of NSCLC that largely supersede its hypothetical CRM effect in importance.

No expression of LIPC had a negative impact on OS. At first glance, this result appears paradoxical because the depletion of LIPC had chemosensitizing properties *in vitro* and its pharmacological inhibition improved the CDDP response *in vitro* and *in vivo*. Only upon further analyses, when patient groups were divided into those who received chemotherapy and those who did not, it turned out that the clinical impact of LIPC expression concords with its biological properties. The absence of LIPC, which among untreated patients have a negative prognostic impact, predict that adjuvant chemotherapy improves patient survival. In contrast, LIPC expression predicts that adjuvant chemotherapy has no beneficial effect and actually adversely affects patient survival.

## REFERENCES

Beers, M.H. (2008). Lung Carcinoma. In The Merck manual of diagnosis and therapy, R.S. Porter, and T.V. Jones, eds. (Rahway, Merck & Co., Inc.), p. 2992.

Bertolini, G., Roz, L., Perego, P., Tortoreto, M., Fontanella, E., Gatti, L., Pratesi, G., Fabbri, A., Andriani, F., Tinelli, S., et al. (2009). Highly tumorigenic lung cancer CD133+ cells display stem-like features and are spared by cisplatin treatment. Proc Natl Acad Sci U S A 106, 16281-16286.

Bossenmeyer-Pourie, C., Kannan, R., Ribieras, S., Wendling, C., Stoll, I., Thim, L., Tomasetto, C., and Rio, M.C. (2002). The trefoil factor 1 participates in gastrointestinal cell differentiation by delaying G1-S phase transition and reducing apoptosis. J Cell Biol 157, 761-770.

Boyd, V.L. and Zon, G. (2004). Bisulfite conversion of genomic DNA for methylation analysis: protocol simplification with higher recovery applicable to limited samples and increased throughput. Anal Biochem. 326, 278-280.

Breen, D., and Barlesi, F. (2008). The place of excision repair cross complementation 1 (ERCC1) in surgically treated non-small cell lung cancer. Eur J Cardiothorac Surg 33, 805-811.

Castedo, M., Coquelle, A., Vivet, S., Vitale, I., Kauffmann, A., Dessen, P., Pequignot, M.O., Casares, N., Valent, A., Mouhamad, S., et al. (2006). Apoptosis regulation in tetraploid cancer cells. EMBO J 25,2584-2595.

Cosaert, J., and Quoix, E. (2002). Platinum drugs in the treatment of non-small-cell lung cancer. Br J Cancer 87, 825-833.

Dai, C.H., Li, J., Shi, S.B., Yu, L.C., Ge, L.P., and Chen, P. (2010). Survivin and Smac gene expressions but not livin are predictors of prognosis in non-small cell lung cancer patients treated with adjuvant chemotherapy following surgery. Jpn J Clin Oncol 40, 327-335.

de La Motte Rouge, T., Galluzzi, L., Olaussen, K.A., Zermati, Y., Tasdemir, E., Robert, T., Ripoche, H., Lazar, V., Dessen, P., Harper, F., et al. (2007). A novel epidermal growth factor receptor inhibitor promotes apoptosis in non-small cell lung cancer cells resistant to erlotinib. Cancer Res 67, 6253-6262.

Dzagnidze, A., Katsarava, Z., Makhalova, J., Liedert, B., Yoon, M.S., Kaube, H., Limmroth, V. and Thomale J. (2007) Repair capacity for platinum-DNA adducts determines the severity of cisplatin-induced peripheral neuropathy. J Neurosci. 27, 9451-9457.

Frommer, M., McDonald, L.E., Millar, D.S., Collis, C.M., Watt, F., Grigg, G.W., Molloy, P.L. and Paul, CL. (1992). A genomic sequencing protocol that yields a positive display of 5-methylcytosine residues in individual DNA strands. Proc Natl Acad Sci U S A. 89, 1827-1831.

Galluzzi, L., Zamzami, N., de La Motte Rouge, T., Lemaire, C., Brenner, C.and Kroemer, G. (2007). Methods for the assessment of mitochondrial membrane permeabilization in apoptosis. Apoptosis. 12, 803-813.

Galluzzi, L., Vitale, I., Kepp, O., Séror, C., Hangen, E., Perfettini, J.L., Modjtahedi, N. and Kroemer, G. (2008). Methods to dissect mitochondrial membrane permeabilization in the course of apoptosis. Methods Enzymol. 442, 355-374.

Galluzzi, L., Aaronson, S.A., Abrams, J., Alnemri, E.S., Andrews, D.W., Baehrecke, E.H., Bazan, N.G., Blagosklonny, M.V., Blomgren, K., Borner, C. et al (2009). Guidelines for the use and interpretation of assays for monitoring cell death in higher eukaryotes. Cell Death Differ. 16, 1093-1107.

Hoffmann, J., Vitale, I., Buchmann, B., Galluzzi, L., Schwede, W., Senovilla, L., Skuballa, W., Vivet, S., Lichtner, R.B., Vicencio, J.M., et al. (2008). Improved cellular pharmacokinetics and pharmacodynamics underlie the wide anticancer activity of sagopilone. Cancer Res 68, 5301-5308.

Jemal, A., Thun, M.J., Ries, L.A., Howe, H.L., Weir, H.K., Center, M.M., Ward, E., Wu, X.C., Eheman, C., Anderson, R., et al. (2008). Annual report to the nation on the status of cancer, 1975-2005, featuring trends in lung cancer, tobacco use, and tobacco control. J Natl Cancer Inst 100, 1672-1694.

Johansson, M., Relton, C., Ueland, P.M., Vollset, S.E., Midttun, O., Nygard, O., Slimani, N., Boffetta, P., Jenab, M., Clavel-Chapelon, F., et al. (2010). Serum B vitamin levels and risk of lung cancer. JAMA 303, 2377-2385.

Kancha, R.K., von Bubnoff, N., Peschel, C., and Duyster, J. (2009). Functional analysis of epidermal growth factor receptor (EGFR) mutations and potential implications for EGFR targeted therapy. Clin Cancer Res 15, 460-467.

Kroemer, G., Galluzzi, L., and Brenner, C. (2007). Mitochondrial membrane permeabilization in cell death. Physiol Rev 87, 99-163.

Kroemer, G., and Pouyssegur, J. (2008). Tumor cell metabolism: cancer's Achilles' heel. Cancer Cell 13, 472-482.

Lee, H.S., Moon, B.J., Choi, S.Y., and Kwon, O.S. (2000). Human pyridoxal kinase: overexpression and properties of the recombinant enzyme. Mol Cells 10, 452-459.

Liedert, B., Pluim, D., Schellens, J. and Thomale, J. (2006) Adduct-specific monoclonal antibodies for the measurement of cisplatin-induced DNA lesions in individual cell nuclei. Nucleic Acids Res. 34, e47.

Loriot, Y., Mordant, P., Dorvault, N., De la motte Rouge, T., Bourhis, J., Soria, J.C. and Deutsch, E. (2010). BMS-690514, a VEGFR and EGFR tyrosine kinase inhibitor, shows anti-tumoural activity on non-small-cell lung cancer xenografts and induces sequence-dependent synergistic effect with radiation. Br J Cancer. 103, 347-53.

Madeo, F., Herker, E., Maldener, C., Wissing, S., Lächelt, S., Herlan, M., Fehr, M., Lauber, K., Sigrist, S.J., Wesselborg, S. et al. (2002). A caspase-related protease regulates apoptosis in yeast. Mol Cell. 9, 911-917.

Mandic, A., Hansson, J., Linder, S., and Shoshan, M.C. (2003). Cisplatin induces endoplasmic reticulum stress and nucleus-independent apoptotic signaling. J Biol Chem 278, 9100-9106.

Martin, L.P., Hamilton, T.C., and Schilder, R.J. (2008). Platinum resistance: the role of DNA repair pathways. Clin Cancer Res 14, 1291-1295.

Midttun, Ø., Hustad, S. and Ueland, P.M. (2009) Quantitative profiling of biomarkers related to B-vitamin status, tryptophan metabolism and inflammation in human plasma by liquid chromatography/tandem mass spectrometry.

Rapid Commun Mass Spectrom. 23, 1371-1379.

Mills, P.B., Struys, E., Jakobs, C., Plecko, B., Baxter, P., Baumgartner, M., Willemsen, M.A., Omran, H., Tacke, U., Uhlenberg, B., et al. (2006). Mutations in antiquitin in individuals with pyridoxine-dependent seizures. Nat Med 12, 307-309.

Mondragon, L., Galluzzi, L., Mouhamad, S., Orzaez, M., Vicencio, J.M., Vitale, I., Moure, A., Messeguer, A., Perez-Paya, E., and Kroemer, G. (2009). A chemical inhibitor of Apaf-1 exerts mitochondrioprotective functions and interferes with the intra-S-phase DNA damage checkpoint. Apoptosis 14, 182-190.

Mouhamad, S., Galluzzi, L., Zermati, Y., Castedo, M. and Kroemer, G. (2007). Apaf-1 Deficiency Causes Chromosomal Instability. Cell Cycle. 6, 3103-3107.

Olaussen, K.A., Dunant, A., Fouret, P., Brambilla, E., Andre, F., Haddad, V., Taranchon, E., Filipits, M., Pirker, R., Popper, H.H., et al. (2006). DNA repair by ERCC1 in non-small-cell lung cancer and cisplatin-based adjuvant chemotherapy. N Engl J Med 355, 983-991.

Osoegawa, A., Yoshino, I., Tanaka, S., Sugio, K., Kameyama, T., Yamaguchi, M., and Maehara, Y. (2004). Regulation of p27 by S-phase kinase-associated protein 2 is associated with aggressiveness in non-small-cell lung cancer. J Clin Oncol 22, 4165-4173.

Ota, S., Ishii, G., Goto, K., Kubota, K., Kim, Y.H., Kojika, M., Murata, Y., Yamazaki, M., Nishiwaki, Y., Eguchi, K., et al. (2009). Immunohistochemical expression of BCRP and ERCC1 in biopsy specimen predicts survival in advanced non-small-cell lung cancer treated with cisplatin-based chemotherapy. Lung Cancer 64, 98-104.

Rodriguez-Navarro, S., Llorente, B., Rodriguez-Manzaneque, M.T., Ramne, A., Uber, G., Marchesan, D., Dujon, B., Herrero, E., Sunnerhagen, P. and Pérez-Ortín, J.E. (2002). Functional analysis of yeast gene families involved in metabolism of vitamins B1 and B6. Yeast. 19, 1261-1276.

Saeed, AI, Sharov, V, White, J, Li, J, Liang, W, Bhagabati, N, Braisted, J, Klapa, M, Currier, T, Thiagarajan, M, et al. (2003). TM4: a free, open-source system for microarray data management and analysis. Biotechniques 34(2): 374-8.

Schwarzer, G. (2007) Meta: An R Package for Meta-Analysis. R News, 7/3, 40-45.

Seve, P., and Dumontet, C. (2005). Chemoresistance in non-small cell lung cancer. Curr Med Chem Anticancer Agents 5, 73-88.

Soda, M., Choi, Y.L., Enomoto, M., Takada, S., Yamashita, Y., Ishikawa, S., Fujiwara, S., Watanabe, H., Kurashina, K., Hatanaka, H., et al. (2007). Identification of the transforming EML4-ALK fusion gene in non-small-cell lung cancer. Nature 448, 561-566.

Streit, M., Riccardi, L., Velasco, P., Brown, L.F., Hawighorst, T., Bornstein, P. and Detmar, M. (1999) Thrombospondin-2: a potent endogenous inhibitor of tumor growth and angiogenesis. Proc Natl Acad Sci U S A. 96, 14888-14893.

Tajeddine, N., Galluzzi, L., Kepp, O., Hangen, E., Morselli, E., Senovilla, L., Araujo, N., Pinna, G., Larochette, N., Zamzami, N., et al. (2008). Hierarchical involvement of Bak, VDAC1 and Bax in cisplatin-induced cell death. Oncogene 27, 4221-4232.

Vicencio, J.M., Ortiz, C., Criollo, A., Jones, A.W., Kepp, O., Galluzzi, L., Joza, N., Vitale, I., Morselli, E., Tailler, M., et al. (2009). The inositol 1,4,5-trisphosphate receptor regulates autophagy through its interaction with Beclin 1. Cell Death Differ 16, 1006-1017.

Vitale, I., Galluzzi, L., Vivet, S., Nanty, L., Dessen, P., Senovilla, L., Olaussen, K.A., Lazar, V., Prudhomme, M., Golsteyn, R.M., et al. (2007). Inhibition of Chkl kills tetraploid tumor cells through a p53-dependent pathway. PLoS One 2, e1337.

Zermati Y., Mouhamad S., Stergiou L., Besse B., Galluzzi L., Boehrer S., Pauleau A.L., Rosselli F., D'Amelio M.,

Amendola R. et al. (2007). Nonapoptotic role for Apaf-1 in the DNA damage checkpoint. Mol Cell. 28, 624-37.

Zhang, L., and Hanigan, M.H. (2003). Role of cysteine S-conjugate beta-lyase in the metabolism of cisplatin. J Pharmacol Exp Ther 306, 988-994.

Zischka, H., Larochette, N., Hoffmann, F., Hamöller, D., Jägemann, N., Lichtmannegger, J., Jennen, L., Müller-Höcker, J., Roggel, F., Göttlicher, M. et al. (2008). Electrophoretic analysis of the mitochondrial outer membrane rupture induced by permeability transition. Anal Chem. 80, 5051-5058.

## Claims

1.  An *in vitro* method of assessing or monitoring the sensitivity of a subject having a tumor to a chemotherapy, which method comprises a step of determining, in a biological sample of said subject, the absence, presence or expression level of the expression product of at least one gene selected from the genes identified in Table I, or a step of detecting an alteration, such as a single nucleotide polymorphism (SNP), in at least one of said genes, thereby assessing or monitoring whether the subject having a tumor is responsive or resistant to the chemotherapy.

2.  The method according to claim 1, wherein the method further comprises a step of comparing the expression level of the at least one gene in the biological sample of the subject to a reference expression level.

3.  The method according to claim 1, wherein the at least one gene is the hepatic lipase (LIPC) gene.

4.  The method according to claim 3, wherein the presence of LIPC in the biological sample, is indicative of a resistance of the subject to the chemotherapy.

5.  A method of selecting an appropriate treatment of a cancer for a subject having a tumor, which method comprises a step of determining the presence or absence of LIPC in a biological sample of said subject, the absence of LIPC in the biological sample being the indication that a chemotherapy will be efficient in the subject, the presence of LIPC in the biological sample being the indication that a chemotherapy will be detrimental to the subject.

6.  A method of assessing the prognosis of a cancer in a subject, the method comprising a step of determining, in a biological sample of said subject, the absence, presence or expression level of a compound selected from vitamin B6, pyridoxal (PL), pyridoxal-5-phosphate (PLP), pyridoxamine (PM), pyridoxamine-5'-phosphate, pyridoxine (PN), pyridoxine-5'-phosphate, L-2-aminoadipate and L-2-aminoadipate 6-semialdehyde, and/or of the expression product of at least one gene selected from the genes identified in Table I, or a derivative product thereof, thereby assessing the prognosis of the cancer in the subject.

7. The method according to claim 6, wherein the method further comprises a step of comparing the expression level of the at least one gene in the biological sample of the subject to a reference expression level.

8. The method according to claim 6, wherein the at least one gene is the hepatic lipase (LIPC) gene and the presence of LIPC is indicative of a favourable prognosis of the cancer in the subject.

9. The method according to claim 6, wherein the at least one gene is the hepatic lipase (LIPC) gene and the absence of LIPC is indicative of an unfavourable prognosis of the cancer in the subject.

10. The method according to claim 7, wherein the at least one gene is the pyridoxal kinase (PDXK) gene and an over expression thereof when compared to a reference expression level is indicative of a favourable prognosis of the cancer in the subject.

11. The method according to claim 7, wherein the at least one gene is the pyridoxal kinase (PDXK) gene and the non expression thereof or a reduced expression thereof when compared to a reference expression level is indicative of an unfavourable prognosis of the cancer in the subject.

12. The method according to anyone of the preceding claims, wherein the chemotherapy is using an alkylating agent such as camptothecin, cyclophosphamide, mechlorethamine, uramustine, melphalan, chlorambucil, ifosfamide, carmustine, lomostine, streoptozocin, busulfan, thiotepa, dacarbazine, mitozolomide, temozolomide, procarbazine, altretamine, dacarbazine, or an alkylating-like agent, in particular a platinum-based agent selected from cis-platinum (cisplatin or CDDP), carboplatin, nedaplatin, satraplatin and oxali-platinum (oxaliplatin or OXP).

13. The method according to anyone of the preceding claims, wherein the cancer is selected from non-small cell lung cancer (NSCLC), small cell lung cancer (SCLC), head and neck cancer, cervical carcinoma, ovarian cancer, osteosarcoma, melanoma and colorectal cancer.

14. The method according to anyone of the preceding claims, wherein the biological sample of the subject is selected from a tumor sample, a blood sample, a serum sample and a bronchoalveolar lavage (BAL).

15. A method of selecting an appropriate treatment of a cancer for a subject having a tumor, which method comprises a step of determining the expression level of a compound selected from PDXK, PDXP and aldehyde dehydrogenase 7 family, member A1 (ALDH7A1) in a biological sample of said subject, the non expression thereof or a reduced expression thereof, when compared to a reference expression level, being the indication that a compound selected from vitamin B6, pyridoxal (PL), pyridoxal-5-phosphate (PLP), pyridoxamine (PM), pyridoxamine-5'-phosphate, pyridoxine (PN), and pyridoxine-5'-phosphate (PNP), should be administered to the patient preferably alone or together with a conventional treatment of cancer such as a chemotherapy.

16. A method of selecting an appropriate treatment of a cancer for a subject having a tumor, which method comprises a step of determining the presence, absence or expression level of LIPC in a biological sample of said subject, the presence of LIPC in the biological sample, or an over expression thereof when compared to a reference expression level, being the indication that orlistat® should be administered to the patient together with a conventional treatment of cancer such as a chemotherapy, in particular a chemotherapy using cis-platinum (cisplatin or CDDP).

17. A kit for assessing or monitoring the sensitivity of a subject having a tumor to a chemotherapy, or the prognosis of a cancer in a subject, wherein the kit comprises detection means selected from the group consisting of a pair of primers, a probe, at least one antibody specific to the expression product of a gene selected from the genes identified in Table I or to a derivative product thereof, and a DNA chip, and, optionally, a leaflet providing the wild-type sequence of the gene and/or the control quantitative expression value corresponding to the expression product of the gene in a control population and/or corresponding to a derivative product thereof.

18. A DNA chip comprising a solid support which carries nucleic acids that are specific to at least two genes selected from the genes identified in Table I.

19. A method for screening or identifying a compound suitable for improving the treatment of a cancer in subject having a tumor, said method comprising determining the ability of a test compound to modify the expression of at least one gene selected from the genes identified in Table I, or compensate an abnormal expression thereof.

Figure 1

Figure 1

Figure 2

**Figure 2**

Figure 2

Figure 3

Figure 4

Figure 4

D

E

Figure 4

**F**

**G**

Figure 4

Figure 5

Figure 5

Figure 5

Figure 6

Figure 6

Figure 7

**A**

**B**

Figure 8

EP 2 426 216 A1

Figure 8

141

Figure 8

Figure 9

Figure 10

Figure 11

Figure 12

Figure 13

## EUROPEAN SEARCH REPORT

Application Number

EP 10 30 5937

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARCZMAREK-BOROWSKA B ET AL: "Estimation of prognostic value of Bcl-xL gene expression in non-small cell lung cancer", LUNG CANCER, vol. 51, no. 1, 1 January 2006 (2006-01-01), pages 61-69, XP024893833, ELSEVIER, AMSTERDAM, NL ISSN: 0169-5002, DOI: 10.1016/J.LUNGCAN.2005.08.010 [retrieved on 2006-01-01] * abstract * * page 62, paragraph 2.1 * * page 63, paragraphs 2.2, 2.2.1, 2.2.2 * * page 65, paragraph 3.3 - page 66 * ----- | 1,2,6,7, 12-14,17 | INV. C12Q1/68 |
| X | PEREGO P ET AL: "Role of apoptosis and apoptosis-related proteins in the cisplatin-resistant phenotype of human tumor cell lines.", APOPTOSIS : AN INTERNATIONAL JOURNAL ON PROGRAMMED CELL DEATH, vol. 2, no. 6, 1997, pages 540-548, XP002615910, ISSN: 1360-8185 * page 541, left-hand column, paragraph bridging - right-hand column * * page 542, left-hand column, paragraph bridging - right-hand column * * page 542, right-hand column, paragraph 1st full * * page 545, right-hand column, paragraph 1st full * * figure 4 * ----- -/-- | 1,2,6,7, 12-14,17 | TECHNICAL FIELDS SEARCHED (IPC) C12Q |

~~The present search report has been drawn up for all claims~~

| Place of search | Date of completion of the search | Examiner |
|---|---|---|
| Munich | 9 May 2011 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

## EUROPEAN SEARCH REPORT

Application Number

EP 10 30 5937

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEI XIAOYONG ET AL: "Bcl-XL small interfering RNA sensitizes cisplatin-resistant human lung adenocarcinoma cells", ACTA BIOCHIMICA ET BIOPHYSICA SINICA, vol. 39, no. 5, May 2007 (2007-05), pages 344-350, XP009142986, ISSN: 1672-9145 * page 345, left-hand column, paragraph bridging - right-hand column * * page 345, right-hand column, paragraph 1st full * * page 346, left-hand column, paragraph 1st and 3rd full * * page 348, left-hand column, paragraph bridging - right-hand column * * figure 1 * ----- | 1,2,6,7, 12-14,17 | |
| X | DATABASE Geo [Online] 11 March 2002 (2002-03-11), "[HG-U133A] Affymetrix Human Genome U133A Array", XP002527544, retrieved from NCBI Database accession no. GLP96 * the whole document * ----- -/-- | 17,18 | TECHNICAL FIELDS SEARCHED (IPC) |

The present search report has been drawn up for all claims

| Place of search | Date of completion of the search | Examiner |
|---|---|---|
| Munich | 9 May 2011 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

**EUROPEAN SEARCH REPORT**

Application Number

EP 10 30 5937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WENDT MICHAEL D ET AL: "Discovery and structure-activity relationship of antagonists of B-cell lymphoma 2 family proteins with chemopotentiation activity in vitro and in vivo", JOURNAL OF MEDICINAL CHEMISTRY, vol. 49, no. 3, February 2006 (2006-02), pages 1165-1181, XP002615911, ISSN: 0022-2623 * section "structure - activity relationships"; page 1168 - page 1171 * * page 1171 - page 1172 * ----- | 19 | |
| X Y | US 2009/123925 A1 (COLLIE-DUGUID ELAINA S R [GB] ET AL) 14 May 2009 (2009-05-14) * paragraph [0111] * * table 2 * * claims 1,17,19 * ----- | 12,15 1,2,6,7, 13,14, 17-19 | |
| Y | OLAUSSEN KEN A ET AL: "DNA repair by ERCC1 in non-small-cell lung cancer and cisplatin-based adjuvant chemotherapy", NEW ENGLAND JOURNAL OF MEDICINE,, vol. 355, no. 10, 1 September 2006 (2006-09-01), pages 983-991, XP002612486, * abstract * ----- | 1,2,6,7, 12-14, 17-19 | TECHNICAL FIELDS SEARCHED (IPC) |
| Y | US 2007/196840 A1 (TARON ROCA MIGUEL [ES] ET AL) 23 August 2007 (2007-08-23) * paragraph 58 - sentence 74 * * claim 1 * ----- -/-- | 1,2,6,7, 12-14, 17-19 | |

~~The present search report has been drawn up for all claims~~

| Place of search | Date of completion of the search | Examiner |
|---|---|---|
| Munich | 9 May 2011 | Ripaud, Leslie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

**Europäisches Patentamt**
**European Patent Office**
**Office européen des brevets**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/095607 A1 (ERLANDER MARK [US] ET AL) 5 May 2005 (2005-05-05)<br>* figure 1 *<br>* paragraphs [0013], [0106] - [0108] *<br>* page 19; table 3 *<br>----- | 6,7,14 | |
| X | US 2007/269432 A1 (NAKAMURA YUSUKE [JP] ET AL) 22 November 2007 (2007-11-22)<br>* paragraphs [0026], [0190], [0221] *<br>* table 11 *<br>* claim 85 *<br>----- | 6,7,10,11,14,19 | |
| X | US 2009/291853 A1 (KIM BYUNG-CHUL [KR] ET AL) 26 November 2009 (2009-11-26)<br>* claims 1,2,4,21,23 *<br>* paragraphs [0009] - [0016], [0056] *<br>* page 10; table 2 *<br>----- | 1,2,6,7,13,14,17 | |
| X | US 2009/105167 A1 (POTTI ANIL [US] ET AL) 23 April 2009 (2009-04-23)<br>* paragraphs [0078], [ 139] - [0141] *<br>* table 1 *<br>----- | 1,2,6,7,13,14 | TECHNICAL FIELDS SEARCHED (IPC) |
| X | MISSNER ENRICO ET AL: "Off-Target Decoding of a Multitarget Kinase Inhibitor by Chemical Proteomics",<br>CHEMBIOCHEM,<br>vol. 10, no. 7, May 2009 (2009-05), pages 1163-1174, XP002633933,<br>ISSN: 1439-4227<br>* page 1170, paragraph bridging - page 1171 *<br>----- | 17 | |

-/--

~~The present search report has been drawn up for all claims~~

| Place of search | Date of completion of the search | Examiner |
|---|---|---|
| Munich | 9 May 2011 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EUROPEAN SEARCH REPORT

Application Number

EP 10 30 5937

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE Genecards [Online]<br><br>31 December 2010 (2010-12-31), "pyridoxal (pyridoxine, vitamin B6) kinase",<br>XP002633934,<br>Database accession no. GC21P030506<br>* pages 1,24 * | 1,2,6,7, 10-15, 17-19 | |
| A | anonymous: "Lung Carcinoma",<br>Merck manual professional<br>,<br>March 2008 (2008-03), XP002633935,<br>Retrieved from the Internet:<br>URL:http://www.merckmanuals.com/profession al/print/sec05/ch062/ch062b.html<br>[retrieved on 2011-04-21]<br>* table 3 * | 1,2,6,7, 10-15, 17-19 | |
| A | SHOEMAKER ALEX R ET AL: "A small-molecule inhibitor of Bcl-X-L potentiates the activity of cytotoxic drugs in vitro and in vivo",<br>CANCER RESEARCH,<br>vol. 66, no. 17, September 2006 (2006-09), pages 8731-8739, XP002615912,<br>ISSN: 0008-5472<br>* abstract * | 1,2,6,7, 12-14, 17-19 | TECHNICAL FIELDS SEARCHED (IPC) |

-/--

~~The present search report has been drawn up for all claims~~

| Place of search | Date of completion of the search | Examiner |
|---|---|---|
| Munich | 9 May 2011 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BARTZ STEVEN R ET AL: "SMALL INTERFERING RNA SCREENS REVEAL ENHANCED CISPLATIN CYTOTOXICITY IN TUMOR CELLS HAVING BOTH BRCA NETWORK AND TP53 DISRUPTIONS", MOLECULAR AND CELLULAR BIOLOGY, vol. 26, no. 24, 1 December 2006 (2006-12-01), pages 9377-9386, XP009077057, AMERICAN SOCIETY FOR MICROBIOLOGY, WASHINGTON, US ISSN: 0270-7306, DOI: 10.1128/MCB.01229-06 * abstract * | 1,2,6,7, 10-15, 17-19 | |
| A | JI DIANA ET AL: "A screen of shRNAs targeting tumor suppressor genes to identify factors involved in A549 paclitaxel sensitivity", ONCOLOGY REPORTS, vol. 18, no. 6, December 2007 (2007-12), pages 1499-1505, XP002633936, ISSN: 1021-335X * abstract * | 1,2,6,7, 10-15, 17-19 | |
| A | JOHANSSON MATTIAS ET AL: "Serum B Vitamin Levels and Risk of Lung Cancer", JAMA (JOURNAL OF THE AMERICAN MEDICAL ASSOCIATION), vol. 303, no. 23, June 2010 (2010-06), pages 2377-2385, XP002633937, ISSN: 0098-7484 * abstract * | 1,2,6,7, 10-15, 17-19 | TECHNICAL FIELDS SEARCHED (IPC) |

-/--

~~The present search report has been drawn up for all claims~~

| Place of search | Date of completion of the search | Examiner |
|---|---|---|
| Munich | 9 May 2011 | Ripaud, Leslie |

EPO FORM 1503 03.82 (P04C01)

Europäisches
Patentamt

European
Patent Office

Office européen
des brevets

## EUROPEAN SEARCH REPORT

Application Number

EP 10 30 5937

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAPENNA SILVIA ET AL: "Cell cycle kinases as therapeutic targets for cancer.", NATURE REVIEWS. DRUG DISCOVERY, vol. 8, no. 7, July 2009 (2009-07), pages 547-566, XP002633938, ISSN: 1474-1784 * page 536; table 2 * | 1,2,6,7, 10-15, 17-19 | |
| A | FAN LI ET AL: "Oral Xeloda plus bi-platinu two-way combined chemotherapy in treatment of advanced gastrointestinal malignancies.", WORLD JOURNAL OF GASTROENTEROLOGY : WJG, vol. 11, no. 28, 28 July 2005 (2005-07-28), pages 4300-4304, XP002633939, ISSN: 1007-9327 * abstract * | 1,2,6,7, 10-15, 17-19 | |
| A | WO 2008/043561 A2 (MAX PLANCK GESELLSCHAFT [DE]; MEYER THOMAS F [DE]; KARLAS ALEXANDER [D) 17 April 2008 (2008-04-17) * sequences 1795,1798,7119,7121 * | 1,2,6,7, 10-15, 17-19 | TECHNICAL FIELDS SEARCHED (IPC) |

~~The present search report has been drawn up for all claims~~

| Place of search | Date of completion of the search | Examiner |
|---|---|---|
| Munich | 9 May 2011 | Ripaud, Leslie |

EPO FORM 1503 03.82 (P04C01)

Europäisches
Patentamt

European
Patent Office

Office européen
des brevets

Application Number

EP 10 30 5937

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

10, 11(completely); 1, 2, 6, 7, 12-15, 17-19(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 10 30 5937

The Search Division considers that the present European patent application does not comply with the
requirements of unity of invention and relates to several inventions or groups of inventions, namely:

1. claims: 1, 2, 6, 7, 12-14, 17-19(all partially)

     concerns a method of assessing or monitoring the sensitivity
     of a subject having a tumour to a chemotherapy, comprising
     determining the absence, presence or level of the expression
     product of a marker gene or detecting an alteration in said
     marker gene; a method of assessing the prognosis of a cancer
     in a subject comprising determining the absence, presence or
     level of the expression product of said marker gene; a kit
     and a DNA chip for detecting said expression product; and a
     method for screening a compound having the ability to modify
     the expression of said gene; said methods, kit and chip
     being characterised in that the marker gene is BCL2L1.
     ---

2-3. claims: 1, 2, 6, 7, 12-14, 17-19(all partially)

     idem invention 1, each invention being characterised by the
     2nd and 3rd genes as listed in table 1, respectively.
     ---

4. claims: 3-5, 8, 9, 16(completely); 1, 2, 6, 7, 12-15,
     17-19(partially)

     idem invention 1, wherein the marker gene in LIPC. Invention
     4 further concerns a method of selecting an appropriate
     treatment.
     ---

5-55. claims: 1, 2, 6, 7, 12-14, 17-19(all partially)

     idem invention 1, each invention being characterised by the
     5th to 55th genes as listed in table 1, respectively.
     ---

56. claims: 1, 2, 6, 7, 12-15, 17-19(all partially)

     idem invention 1, wherein the marker gene is ALDH7A1.
     Invention 56 further concerns a method of selecting an
     appropriate treatment.
     ---

57. claims: 10, 11(completely); 1, 2, 6, 7, 12-15, 17-19(partially)

     idem invention 1, wherein the marker gene PDXK. Invention 57
     further concerns a method of selecting an appropriate
     treatment.
     ---

58-85. claims: 1, 2, 6, 7, 12-14, 17-19(all partially)

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

idem invention 1, each invention being characterised by the 58th to 85th genes as listed in table 1, respectively.
---

86. claims: 6, 7, 12-14(all partially)

concerns a method of assessing the prognosis of a cancer in a subject comprising determining the presence, absence, or level of vitamin B6 or a derivative thereof as listed in claim 6.
---

87. claims: 1, 2, 6, 7, 12-15, 17-19(all partially)

idem invention 1, wherein the marker gene is PDXP. Invention 87 further concerns a method of selecting an appropriate treatment.
---

**ANNEX TO THE EUROPEAN SEARCH REPORT
ON EUROPEAN PATENT APPLICATION NO.**

EP 10 30 5937

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2011

| Patent document cited in search report | | | Publication date | Patent family member(s) | | Publication date |
|---|---|---|---|---|---|---|
| US 2009123925 | A1 | | 14-05-2009 | EP 1934370 A2<br>WO 2007034221 A2 | | 25-06-2008<br>29-03-2007 |
| US 2007196840 | A1 | | 23-08-2007 | EP 1818413 A1 | | 15-08-2007 |
| US 2005095607 | A1 | | 05-05-2005 | EP 1651772 A1<br>JP 2007516692 T<br>WO 2005098037 A1 | | 03-05-2006<br>28-06-2007<br>20-10-2005 |
| US 2007269432 | A1 | | 22-11-2007 | AT 508202 T<br>CN 1890381 A<br>CN 1898563 A<br>EP 1668354 A2<br>EP 1668156 A2<br>WO 2005029067 A2<br>WO 2005028676 A2<br>JP 4579246 B2<br>JP 2007506424 T<br>JP 4620670 B2<br>JP 2007506426 T<br>JP 2008092949 A<br>US 2009286856 A1 | | 15-05-2011<br>03-01-2007<br>17-01-2007<br>14-06-2006<br>14-06-2006<br>31-03-2005<br>31-03-2005<br>10-11-2010<br>22-03-2007<br>26-01-2011<br>22-03-2007<br>24-04-2008<br>19-11-2009 |
| US 2009291853 | A1 | | 26-11-2009 | KR 20080065476 A<br>US 2008166729 A1 | | 14-07-2008<br>10-07-2008 |
| US 2009105167 | A1 | | 23-04-2009 | US 2010279957 A1<br>WO 2009052484 A2 | | 04-11-2010<br>23-04-2009 |
| WO 2008043561 | A2 | | 17-04-2008 | AU 2007306542 A1<br>CA 2666185 A1<br>EP 2087110 A2<br>JP 2010505897 T<br>SG 166778 A1 | | 17-04-2008<br>17-04-2008<br>12-08-2009<br>25-02-2010<br>29-12-2010 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FROMMER et al.** *Proc Natl Acad Sci U S A.,* 1992, vol. 89, 1827-31
- **BOYD et al.** *Anal Biochem.,* 2004, vol. 326, 278-80
- Lung Carcinoma. **BEERS, M.H.** The Merck manual of diagnosis and therapy. Merck & Co., Inc, 2008, 2992
- **BERTOLINI, G. ; ROZ, L. ; PEREGO, P. ; TORTORETO, M. ; FONTANELLA, E. ; GATTI, L. ; PRATESI, G. ; FABBRI, A. ; ANDRIANI, F. ; TINELLI, S. et al.** Highly tumorigenic lung cancer CD133+ cells display stem-like features and are spared by cisplatin treatment. *Proc Natl Acad Sci U S A,* 2009, vol. 106, 16281-16286
- **BOSSENMEYER-POURIE, C. ; KANNAN, R. ; RIBIERAS, S. ; WENDLING, C. ; STOLL, I. ; THIM, L. ; TOMASETTO, C. ; RIO, M.C.** The trefoil factor 1 participates in gastrointestinal cell differentiation by delaying G1-S phase transition and reducing apoptosis. *J Cell Biol,* 2002, vol. 157, 761-770
- **BOYD, V.L. ; ZON, G.** Bisulfite conversion of genomic DNA for methylation analysis: protocol simplification with higher recovery applicable to limited samples and increased throughput. *Anal Biochem.,* 2004, vol. 326, 278-280
- **BREEN, D. ; BARLESI, F.** The place of excision repair cross complementation 1 (ERCC1) in surgically treated non-small cell lung cancer. *Eur J Cardiothorac Surg,* 2008, vol. 33, 805-811
- **CASTEDO, M. ; COQUELLE, A. ; VIVET, S. ; VITALE, I. ; KAUFFMANN, A. ; DESSEN, P. ; PEQUIGNOT, M.O. ; ASARES, N. ; VALENT, A. ; MOUHAMAD, S. et al.** Apoptosis regulation in tetraploid cancer cells. *EMBO J,* 2006, vol. 25, 2584-2595
- **COSAERT, J. ; QUOIX, E.** Platinum drugs in the treatment of non-small-cell lung cancer. *Br J Cancer,* 2002, vol. 87, 825-833
- **DAI, C.H. ; LI, J. ; SHI, S.B. ; YU, L.C. ; GE, L.P. ; CHEN, P.** Survivin and Smac gene expressions but not livin are predictors of prognosis in non-small cell lung cancer patients treated with adjuvant chemotherapy following surgery. *Jpn J Clin Oncol,* 2010, vol. 40, 327-335

- **DE LA MOTTE ROUGE, T. ; GALLUZZI, L. ; OLAUSSEN, K.A. ; ZERMATI, Y. ; TASDEMIR, E. ; ROBERT, T. ; RIPOCHE, H. ; LAZAR, V. ; DESSEN, P. ; HARPER, F. et al.** A novel epidermal growth factor receptor inhibitor promotes apoptosis in non-small cell lung cancer cells resistant to erlotinib. *Cancer Res,* 2007, vol. 67, 6253-6262
- **DZAGNIDZE, A. ; KATSARAVA, Z. ; MAKHALOVA, J. ; LIEDERT, B. ; YOON, M.S. ; KAUBE, H. ; LIMMROTH, V. ; THOMALE J.** Repair capacity for platinum-DNA adducts determines the severity of cisplatin-induced peripheral neuropathy. *J Neurosci.,* 2007, vol. 27, 9451-9457
- **FROMMER, M. ; MCDONALD, L.E. ; MILLAR, D.S. ; COLLIS, C.M. ; WATT, F. ; GRIGG, G.W. ; MOLLOY, P.L. ; PAUL, CL.** A genomic sequencing protocol that yields a positive display of 5-methylcytosine residues in individual DNA strands. *Proc Natl Acad Sci U S A.,* 1992, vol. 89, 1827-1831
- **GALLUZZI, L. ; ZAMZAMI, N. ; DE LA MOTTE ROUGE, T. ; LEMAIRE, C. ; BRENNER, C. ; KROEMER, G.** Methods for the assessment of mitochondrial membrane permeabilization in apoptosis. *Apoptosis,* 2007, vol. 12, 803-813
- **GALLUZZI, L. ; VITALE, I. ; KEPP, O. ; SÉROR, C. ; HANGEN, E. ; PERFETTINI, J.L. ; MODJTAHEDI, N. ; KROEMER, G.** Methods to dissect mitochondrial membrane permeabilization in the course of apoptosis. *Methods Enzymol.,* 2008, vol. 442, 355-374
- **GALLUZZI, L. ; AARONSON, S.A. ; ABRAMS, J. ; ALNEMRI, E.S. ; ANDREWS, D.W. ; BAEHRECKE, E.H. ; BAZAN, N.G. ; BLAGOSKLONNY, M.V. ; BLOMGREN, K. ; BORNER, C. et al.** Guidelines for the use and interpretation of assays for monitoring cell death in higher eukaryotes. *Cell Death Differ.,* 2009, vol. 16, 1093-1107
- **HOFFMANN, J. ; VITALE, I. ; BUCHMANN, B. ; GALLUZZI, L. ; SCHWEDE, W. ; SENOVILLA, L. ; SKUBALLA, W. ; VIVET, S. ; LICHTNER, R.B. ; VICENCIO, J.M. et al.** Improved cellular pharmacokinetics and pharmacodynamics underlie the wide anticancer activity of sagopilone. *Cancer Res,* 2008, vol. 68, 5301-5308

- **JEMAL, A. ; THUN, M.J. ; RIES, L.A. ; HOWE, H.L. ; WEIR, H.K. ; CENTER, M.M. ; WARD, E. ; WU, X.C. ; EHEMAN, C. ; ANDERSON, R. et al.** Annual report to the nation on the status of cancer, 1975-2005, featuring trends in lung cancer, tobacco use, and tobacco control. *J Natl Cancer Inst,* 2008, vol. 100, 1672-1694
- **JOHANSSON, M. ; RELTON, C. ; UELAND, P.M. ; VOLLSET, S.E. ; MIDTTUN, O. ; NYGARD, O. ; SLIMANI, N. ; BOFFETTA, P. ; JENAB, M. ; CLAVEL-CHAPELON, F. et al.** Serum B vitamin levels and risk of lung cancer. *JAMA,* 2010, vol. 303, 2377-2385
- **KANCHA, R.K. ; VON BUBNOFF, N. ; PESCHEL, C. ; DUYSTER, J.** Functional analysis of epidermal growth factor receptor (EGFR) mutations and potential implications for EGFR targeted therapy. *Clin Cancer Res,* 2009, vol. 15, 460-467
- **KROEMER, G. ; GALLUZZI, L. ; BRENNER, C.** Mitochondrial membrane permeabilization in cell death. *Physiol Rev,* 2007, vol. 87, 99-163
- **KROEMER, G. ; POUYSSEGUR, J.** Tumor cell metabolism: cancer's Achilles' heel. *Cancer Cell,* 2008, vol. 13, 472-482
- **LEE, H.S. ; MOON, B.J. ; CHOI, S.Y. ; KWON, O.S.** Human pyridoxal kinase: overexpression and properties of the recombinant enzyme. *Mol Cells,* 2000, vol. 10, 452-459
- **LIEDERT, B. ; PLUIM, D. ; SCHELLENS, J. ; THOMALE, J.** Adduct-specific monoclonal antibodies for the measurement of cisplatin-induced DNA lesions in individual cell nuclei. *Nucleic Acids Res.,* 2006, vol. 34, e47
- **LORIOT, Y. ; MORDANT, P. ; DORVAULT, N. ; DE LA MOTTE ROUGE, T. ; BOURHIS, J. ; SORIA, J.C. ; DEUTSCH, E.** BMS-690514, a VEGFR and EGFR tyrosine kinase inhibitor, shows anti-tumoural activity on non-small-cell lung cancer xenografts and induces sequence-dependent synergistic effect with radiation. *Br J Cancer,* 2010, vol. 103, 347-53
- **MADEO, F. ; HERKER, E. ; MALDENER, C. ; WISSING, S. ; LÄCHELT, S. ; HERLAN, M. ; FEHR, M. ; LAUBER, K. ; SIGRIST, S.J. ; WESSELBORG, S. et al.** A caspase-related protease regulates apoptosis in yeast. *Mol Cell,* 2002, vol. 9, 911-917
- **MANDIC, A. ; HANSSON, J. ; LINDER, S. ; SHOSHAN, M.C.** Cisplatin induces endoplasmic reticulum stress and nucleus-independent apoptotic signaling. *J Biol Chem,* 2003, vol. 278, 9100-9106
- **MARTIN, L.P. ; HAMILTON, T.C. ; SCHILDER, R.J.** Platinum resistance: the role of DNA repair pathways. *Clin Cancer Res,* 2008, vol. 14, 1291-1295

- **MIDTTUN, Ø. ; HUSTAD, S. ; UELAND, P.M.** Quantitative profiling of biomarkers related to B-vitamin status, tryptophan metabolism and inflammation in human plasma by liquid chromatography/tandem mass spectrometry. *Rapid Commun Mass Spectrom,* 2009, vol. 23, 1371-1379
- **MILLS, P.B. ; STRUYS, E. ; JAKOBS, C. ; PLECKO, B. ; BAXTER, P. ; BAUMGARTNER, M. ; WILLEMSEN, M.A. ; OMRAN, H. ; TACKE, U. ; UHLENBERG, B. et al.** Mutations in antiquitin in individuals with pyridoxine-dependent seizures. *Nat Med,* 2006, vol. 12, 307-309
- **MONDRAGON, L. ; GALLUZZI, L. ; MOUHAMAD, S. ; ORZAEZ, M. ; VICENCIO, J.M. ; VITALE, I. ; MOURE, A. ; MESSEGUER, A. ; PEREZ-PAYA, E. ; KROEMER, G.** A chemical inhibitor of Apaf-1 exerts mitochondrioprotective functions and interferes with the intra-S-phase DNA damage checkpoint. *Apoptosis,* 2009, vol. 14, 182-190
- **MOUHAMAD, S. ; GALLUZZI, L. ; ZERMATI, Y. ; CASTEDO, M. ; KROEMER, G.** Apaf-1 Deficiency Causes Chromosomal Instability. *Cell Cycle,* 2007, vol. 6, 3103-3107
- **OLAUSSEN, K.A. ; DUNANT, A. ; FOURET, P. ; BRAMBILLA, E. ; ANDRE, F. ; HADDAD, V. ; TARANCHON, E. ; FILIPITS, M. ; PIRKER, R. ; POPPER, H.H. et al.** DNA repair by ERCC1 in non-small-cell lung cancer and cisplatin-based adjuvant chemotherapy. *N Engl J Med,* 2006, vol. 355, 983-991
- **OSOEGAWA, A. ; YOSHINO, I. ; TANAKA, S. ; SUGIO, K. ; KAMEYAMA, T. ; YAMAGUCHI, M. ; MAEHARA, Y.** Regulation of p27 by S-phase kinase-associated protein 2 is associated with aggressiveness in non-small-cell lung cancer. *J Clin Oncol,* 2004, vol. 22, 4165-4173
- **OTA, S. ; ISHII, G. ; GOTO, K. ; KUBOTA, K. ; KIM, Y.H. ; KOJIKA, M. ; MURATA, Y. ; YAMAZAKI, M. ; NISHIWAKI, Y. ; EGUCHI, K. et al.** Immunohistochemical expression of BCRP and ERCC1 in biopsy specimen predicts survival in advanced non-small-cell lung cancer treated with cisplatin-based chemotherapy. *Lung Cancer,* 2009, vol. 64, 98-104
- **RODRIGUEZ-NAVARRO, S. ; LLORENTE, B. ; RODRIGUEZ-MANZANEQUE, M.T. ; RAMNE, A. ; UBER, G. ; MARCHESAN, D. ; DUJON, B. ; HERRERO, E. ; SUNNERHAGEN, P. ; PÉREZ-ORTÍN, J.E.** Functional analysis of yeast gene families involved in metabolism of vitamins B1 and B6. *Yeast,* 2002, vol. 19, 1261-1276
- **SAEED, AI ; SHAROV, V ; WHITE, J ; LI, J ; LIANG, W ; BHAGABATI, N ; BRAISTED, J ; KLAPA, M ; CURRIER, T ; THIAGARAJAN, M et al.** TM4: a free, open-source system for microarray data management and analysis. *Biotechniques,* 2003, vol. 34 (2), 374-8

- **SCHWARZER, G.** Meta: An R Package for Meta-Analysis. *R News,* 2007, vol. 7/3, 40-45
- **SEVE, P. ; DUMONTET, C.** Chemoresistance in non-small cell lung cancer. *Curr Med Chem Anticancer Agents,* 2005, vol. 5, 73-88
- **SODA, M. ; CHOI, Y.L. ; ENOMOTO, M. ; TAKADA, S. ; YAMASHITA, Y. ; ISHIKAWA, S. ; FUJIWARA, S. ; WATANABE, H. ; KURASHINA, K. ; HATANAKA, H. et al.** Identification of the transforming EML4-ALK fusion gene in non-small-cell lung cancer. *Nature,* 2007, vol. 448, 561-566
- **STREIT, M. ; RICCARDI, L. ; VELASCO, P. ; BROWN, L.F. ; HAWIGHORST, T. ; BORNSTEIN, P. ; DETMAR, M.** Thrombospondin-2: a potent endogenous inhibitor of tumor growth and angiogenesis. *Proc Natl Acad Sci U S A.,* 1999, vol. 96, 14888-14893
- **TAJEDDINE, N. ; GALLUZZI, L. ; KEPP, O. ; HANGEN, E. ; MORSELLI, E. ; SENOVILLA, L. ; ARAUJO, N. ; PINNA, G. ; LAROCHETTE, N. ; ZAMZAMI, N. et al.** Hierarchical involvement of Bak, VDAC1 and Bax in cisplatin-induced cell death. *Oncogene,* 2008, vol. 27, 4221-4232
- **VICENCIO, J.M. ; ORTIZ, C. ; CRIOLLO, A. ; JONES, A.W. ; KEPP, O. ; GALLUZZI, L. ; JOZA, N. ; VITALE, I. ; MORSELLI, E. ; TAILLER, M. et al.** The inositol 1,4,5-trisphosphate receptor regulates autophagy through its interaction with Beclin 1. *Cell Death Differ,* 2009, vol. 16, 1006-1017
- **VITALE, I. ; GALLUZZI, L. ; VIVET, S. ; NANTY, L. ; DESSEN, P. ; SENOVILLA, L. ; OLAUSSEN, K.A. ; LAZAR, V. ; PRUDHOMME, M. ; GOLSTEYN, R.M. et al.** Inhibition of Chkl kills tetraploid tumor cells through a p53-dependent pathway. *PLoS One,* 2007, vol. 2, e1337
- **ZERMATI Y. ; MOUHAMAD S. ; STERGIOU L. ; BESSE B. ; GALLUZZI L. ; BOEHRER S. ; PAULEAU A.L. ; ROSSELLI F. ; D'AMELIO M. ; AMENDOLA R et al.** Nonapoptotic role for Apaf-1 in the DNA damage checkpoint. *Mol Cell,* 2007, vol. 28, 624-37
- **ZHANG, L. ; HANIGAN, M.H.** Role of cysteine S-conjugate beta-lyase in the metabolism of cisplatin. *J Pharmacol Exp Ther,* 2003, vol. 306, 988-994
- **ZISCHKA, H. ; LAROCHETTE, N. ; HOFFMANN, F. ; HAMÖLLER, D. ; JÄGEMANN, N. ; LICHTMANNEGGER, J. ; JENNEN, L. ; MÜLLER-HÖCKER, J. ; ROGGEL, F. ; GÖTTLICHER, M. et al.** Electrophoretic analysis of the mitochondrial outer membrane rupture induced by permeability transition. *Anal Chem.,* 2008, vol. 80, 5051-5058